# EUROPEAN PATENT SPECIFICATION

(11) **EP 3 558 436 B1**
(45) Date of publication and mention of the grant of the patent: **21.10.2026**
(21) Application number: 17885285.1
(22) Date of filing: 19.12.2017
(51) Int. Cl.: A61M 25/01, A61B 1/00, A61B 1/005

(54) **STEERABLE MEDICAL DEVICE**
LENKBARE MEDIZINISCHE VORRICHTUNG
DISPOSITIF MÉDICAL ORIENTABLE

(30) Priority: 22.12.2016 US 201662437916 P; 22.12.2016 US 201662437946 P; 06.10.2017 US 201762569055 P
(43) Date of publication of application: 30.10.2019
(73) Proprietor: Boston Scientific Medical Device Limited, Galway (IE)
(72) Inventor: CHEN, Jan-Hung, Halton Hills Ontario L7G 2L1 (CA); LAU, Kandice, Aurora Ontario L4G 6W4 (CA); OLDHAM, Lorraine, Georgetown Ontario L7G 4S8 (CA); THOMPSON SMITH, Melanie, Toronto Ontario M8V 1X1 (CA); KUSINS, Jonathan, London Ontario N5X 1X7 (CA); DAVIES, Gareth, Toronto Ontario M6G 1P6 (CA); CHAN, Audrey, Richmond Hill Ontario L4S 1C1 (CA); ALNABELSEYA, Ahmed, Ottawa Ontario K1T 0B4 (CA); PEQUEGNAT, Andrew, Cambridge Ontario N1P 1G8 (CA); ALLEY, Ferryl, Devonshire DV 07 (BM); BRUGHA, Cathal, Toronto Ontario M6S 2X9 (CA)
(74) Representative: Pfenning, Meinig & Partner mbB
(86) International application number: PCT/IB2017/058137
(87) International publication number: WO 2018/116162

(56) References cited:
- WO-A1-2016/175882
- WO-A1-2016/175882
- US-A- 5 395 327
- US-A- 5 666 970
- US-A1- 2013 131 667
- US-B2- 7 591 799
- US-B2- 9 174 023

## Description

### CROSS-REFERENCES TO RELATED APPLICATIONS

### TECHNICAL FIELD

The disclosure relates to a handle for a steerable medical device. More specifically, this disclosure relates to a handle for a steerable medical device that provides an inefficiency generator that is configured to interact with the means for translating the actuation of the actuator into deflection of the steerable device.

### PRIOR ART

Document US 5 666 970 A describes a locking mechanism for a steerable catheter. The catheter includes a resilient body member having a restoring force that tends to maintain the body member at a predetermined orientation, and a manipulation handle including a control element and a second element movable in relation to one another and adapted for controlling the orientation of the body member. The locking mechanism maintains the selected deflection of the body member against the restoring force but permits a physician to overcome the locking force by moving the control element. The locking mechanism does not interfere with the other control movement of the handle. The locking device has a biasing member and a contact member mounted between the control element and the second element. The biasing member urges the contact member into frictional contact with one of the elements for holding the control element at the selected longitudinal position.

Document US 2013/131667 A1 describes medical devices including catheters for renal nerve ablation and/or modulation as well as methods for making and using such devices are disclosed. An example catheter may, have a proximal region and a distal region. The catheter may be configured to shift between a first straightened configuration and a second deflected configuration. The catheter may also include an ablation member coupled to the distal region and a handle coupled to the proximal region. The handle may include an actuation member for shifting the catheter between the first configuration and the second configuration. A lock may be coupled to the handle that maintains the catheter in either the first configuration or the second configuration.

Document US 5 395 327 A describes a steering mechanism that includes a steering shaft coupled to a controller which includes a handle and apparatus for manipulating the distal end of the steering shaft. The steering shaft includes a flexible coiled spring having a lead spring fixed in position with respect to a distal end thereof in the distal end of the steering shaft. One or more steering wires is affixed at the distal ends thereof to the lead spring. The steering wires extend through the steering shaft to the controller, and the steering apparatus of the controller is used to place tension on one or both of the steering wires. The attachment of the distal ends of the steering wires to the lead spring may be opposite one another or may be offset for providing greater maneuverability. Tension may be placed on the steering wires by wedges mounted transversely to the controller housing, or by rotation of a shaft mounted transversely to the controller housing, the steering wires being attached to the shaft such that rotation in one direction tenses one steering wire, and rotation in the other direction tenses the other steering wire. Two independently rotatable shafts may be used to separately control the two steering wires. The steering shaft is adapted for insertion into a lumen of a catheter for use in guiding the distal end of the catheter to a treatment site within a patient.

Documents WO 2016/175882 A1, US 9 174 023 B2, and US 7 591 799 B2 describes further examples of steerable medical devices.

### SUMMARY

The invention is defined in claim 1. Further aspects and preferred embodiments are defined in the dependent claims. Aspects, embodiments and examples of the present disclosure which do not fall under the scope of the appended claims are merely provided for illustrative purposes.

### BRIEF DESCRIPTION OF THE DRAWINGS

In order that the invention may be readily understood, embodiments of the invention are illustrated by way of examples in the accompanying drawings, in which:
Fig. 1A-1B are an illustration of a steerable catheter control handle in accordance with an embodiment of the present invention;
Figs. 2A-2B are an illustration of a steerable catheter control handle in accordance with an embodiment of the present invention;
Fig. 3 is an illustration of a steerable catheter control handle in accordance with an embodiment of the present invention;
Figs. 4A-4C are an illustration of a steerable catheter control handle in accordance with an embodiment of the present invention;
Figs. 5A-5C are an illustration of a steerable catheter control handle in accordance with an embodiment of the present invention;
Figs. 6A-6C are an illustration of a steerable catheter assembly in accordance with an embodiment of the present invention;
7A is an illustration of a steerable catheter control handle not forming part of the present invention;
7B-7D are an illustration of a steerable catheter control handle in accordance with the present invention;
Fig. 8A-8D are an illustration of a steerable catheter control handle in accordance with an alternate embodiment of the present invention;
Fig. 9A-9C are an illustration of a steerable catheter control handle in accordance with an alternate embodiment of the present invention;
Fig. 10A-10B are an illustration of a steerable catheter control handle in accordance with an alternate embodiment of the present invention;
Fig. 11A-11D are an illustration of an inefficiency generator for a steerable catheter control handle in accordance with an alternate embodiment of the present invention;
Fig. 12A-12F are an illustration of an inefficiency generator for a steerable catheter control handle in accordance with an alternate embodiment of the present invention;
Fig. 13A-13D are an illustration of an inefficiency generator for a steerable catheter control handle in accordance with an alternate embodiment of the present invention;
Fig. 14A-14F are an illustration of an inefficiency generator for a steerable catheter control handle in accordance with an alternate embodiment of the present invention;
Fig. 15A-15D are an illustration of an inefficiency generator for a steerable catheter control handle in accordance with an alternate embodiment of the present invention;
Fig. 16A-16D are an illustration of an inefficiency generator for a steerable catheter control handle in accordance with an alternate embodiment of the present invention;
Fig. 17A-17C are an illustration of an inefficiency generator for a steerable catheter control handle in accordance with an alternate embodiment of the present invention;
Fig. 18A-18C are an illustration of an inefficiency generator for a steerable catheter control handle in accordance with an alternate embodiment of the present invention;
Fig. 19A-19C are an illustration of an inefficiency generator for a steerable catheter control handle in accordance with an alternate embodiment of the present invention;
Fig. 20A-20C are an illustration of an inefficiency generator for a steerable catheter control handle in accordance with an alternate embodiment of the present invention;
21A-21D are an illustration of an inefficiency generator for a steerable catheter control handle in accordance with an alternate embodiment of the present invention; and
Figs. 22A-22B are an illustration of a steerable catheter control handle in accordance with an alternate embodiment of the present invention.
Figs. 23A-23C are an illustration of a threaded slider mechanism, in accordance with alternative embodiments of the present invention;
Figs. 24A-24F are an illustration of an alternate embodiment of a threaded slider mechanism with an increased pitch and a corresponding threaded inner knob, in accordance with an embodiment of the present invention;
Figs. 25A-25F are an illustration of an alternate embodiment of a threaded slider mechanism with an increased pitch and a corresponding threaded inner knob, in accordance with an embodiment of the present invention;
Figs 26A-26F are an illustration of an alternate embodiment of a threaded slider mechanism with a reversed thread and a corresponding threaded inner knob, in accordance with an embodiment of the present invention;
Fig. 27A is a side view of a slide assembly for use within a steerable control handle, illustrating a first indicator portion that is functional to provide neutral zone feedback, in accordance with an embodiment of the present invention;
Fig. 27B is an illustration of a bottom view of a slide assembly for use in a steerable control handle, illustrating the first indicator portion that is functional to provide feedback, in accordance with an embodiment of the present invention;
Fig. 27C is an enlarged view of a slide assembly for use in a steerable control handle, illustrating the first indicator portion for providing feedback, in accordance with an embodiment of the present invention;
Fig. 27D is an illustration of a side view of an inner housing of a steerable control handle, showing a second indicator portion in accordance with an embodiment of the present invention;
Fig. 27E is an illustration of a top view of an inner housing of a steerable control handle, the inner housing being configured to provide feedback, in accordance with an embodiment of the present invention;
Figs. 28A-28B are an illustration of a feedback mechanism for steerable control handle, in accordance with an alternate embodiment of the present invention;
Figs. 28C-28E are an illustration of a feedback mechanism for steerable control handle, in accordance with an alternate embodiment of the present invention;
Figs. 29A-29E are an illustration of a feedback mechanism for steerable control handle, in accordance with an alternate embodiment of the present invention;
Figs. 30A-30B are an illustration of a feedback mechanism for steerable control handle, in accordance with an alternate embodiment of the present invention;
Figs. 31A-31C are an illustration of a feedback mechanism for steerable control handle, in accordance with an alternate embodiment of the present invention;
Figs. 32A-32C are an illustration of a feedback mechanism for steerable control handle, in accordance with an alternate embodiment of the present invention; and
Figs. 33A-33C are an illustration of a feedback mechanism for steerable control handle, in accordance with an alternate embodiment of the present invention.

### DETAILED DESCRIPTION

Some medical procedures, which as such do not form part of the invention as claimed, may use a steerable catheter in order to reach a desired location within a patient's body. The steerable catheter may be deflectable upon actuation of a steerable catheter control handle. Some such steerable catheter control handles comprise actuation mechanisms that do not provide adequate feedback in order to allow the user to ascertain the degree of deflection of the steerable sheath. Additionally, some actuation mechanisms are not able to retain a curvature or position of the sheath once the desired degree of deflection or curvature has been achieved. Furthermore, some prior art actuation mechanisms that do not provide adequate feedback may lead to in breakage or failure of pull-wires as user may not be able to ascertain that excessive force is being applied on the pull-wires.

For example, a steerable catheter control handle may comprise a mechanical actuation mechanism, for example a rotatable knob whose rotation is converted into linear translation of one or more components within the handle assembly using a threadable engagement. The movement of the one or more components results in actuation of one or more pull wires to cause deflection of a distal tip of a catheter. Typically, such a system of the prior art utilizes a square thread for providing a high level of mechanical efficiency in translating rotational motion to linear translation with little loss of energy.

However, in some such systems that have a high level of mechanical efficiency, reverse translation of motion from linear to rotational may also be very efficient when the system is under tension such that when the catheter is deflected, the tension in the pull wires and the catheter may result in reverse linear translation of the one or more components that results in a reverse rotation of the control knob. In other words, due to the high efficiency of the system, when components of the handle assembly are under load by pull wire tension, the components can be effectively back-driven to unwind the knob, which may cause the handle to fail to maintain the sheath curvature. This results in a loss of positioning once the user releases a grip on the knob. As such, systems that utilize efficient components in the rotation to linear translation are typically not self-locking upon actuation and the desired position of the catheter is not maintained after desired deflection.

Additionally, another problem that is encountered with efficient components used in the control handle is that the level of feedback the handle provides may not be sufficient to provide the user with feedback about what is happening at the distal tip of the catheter. In such a system, the user can rotate the knob relatively easily even when the pull wire tension is high due to the efficiencies in the system. This results in little tactile feedback that is available to the user and the user may not be able to easily determine to what extent the catheter has been deflected. In some such embodiments, a force/deflection graph of the rotational force it takes to cause a deflection of the catheter has a gradual slope and the user does not receive the a desired level of tactile feedback, as the difference between the tactile feedback obtained upon minimum and maximum deflection of the catheter is insufficient for the user to readily distinguish. Additionally, the system may not provide sufficient feedback to allow the user to ascertain how much the catheter is being deflected and as such may not allow the user to ascertain if there is excessive force being applied on the pull wires.

In other words, some steerable sheath handle mechanism of the prior art cannot reliably maintain maximum sheath curvature when a dilator and a needle are inserted. Some prior art systems do not provide tactile feedback. Some such prior art systems that are based on a rotation actuation, may have a very high mechanical efficiency translating the knob into linear movement of one or more components, which tension the sheath pull wires and curves the sheath. When the efficiency of this motion translation is high, the user can turn the knob easily to move the one or more components even when the pull wire tension is high, which results in no or minimal tactile feedback. The high efficiency also means that, when the one or more components are under load by pull wire tension, the one or more components can be effectively back-driven to unwind the knob, which causes the handle to fail to maintain the sheath curvature.

As will be presently described, the present inventors have discovered and invented embodiments of unique systems for converting or translating an actuation of the actuator into deflection of the catheter. For example, the present inventors have invented embodiments of a unique system for converting or translating the actuation of an actuator into movement of the pull wires, such as a means for deflecting the one or more pull wires that is coupled to the actuator. In some such example, the system of the present invention may be a single step rotation to linear system. In other examples, the system of the present invention may be a two-step rotation to linear system.

The present inventors have discovered and invented embodiments of unique systems for a rotation to linear system that enables the user to ascertain the degree of deflection of the steerable sheath and/or to maintain a curvature or position of the sheath once the desired degree of deflection or curvature has been achieved. For example, some embodiments provide an enhanced difference in tactile feel between minimum deflection of the catheter and maximum deflection of the catheter. Some embodiments additionally provide an optimized handle design that provides ease of handling for the physician and ease of use. Some embodiments may provide sufficient feedback to the user and may help minimize failure of the control or pull wires (and may help minimize failure resulting from the pull or control wires from breaking from the pull ring), as the physician may be more careful as the catheter is deflected more.

In accordance with some specific embodiments of the present invention, a unique steerable catheter handle assembly is described, that provides a means for reducing efficiency or, in other words, a means for introducing inefficiency, as a component of a rotatable to linear translation mechanism.

The present invention provides a steerable catheter control handle wherein a means for introducing inefficiency into the rotation to linear translation system is provided, that provides feedback regarding the deflection of the catheter at the distal tip. In some such examples, the mechanism may be built into the steerable handle. Some embodiments of the present invention may provide weighted steering which may inform the user of the relative degree of sheath curvature, but also the neutral zone location [which may be ascertained by when the knob is the easiest to turn]. In some such embodiments, the means for reducing efficiency or introducing inefficiency into the mechanical translation system may provide tactile feedback that is taken directly from a pathway of the rotation to linear system itself in order to get a true picture of what is happening at the distal tip of the catheter. In some such instances, the mechanism interacts directly with a pathway of the rotation to linear system that is coupled to the pull wires [coupled to the catheter] in order to provide the user with actual feedback for providing an indication of the distal tip deflection.

The present invention provides a system that comprises a means for reducing efficiency or a means of introducing inefficiency that has been built into the translation mechanism to enable a position of the catheter to be substantially maintained after it has been deflected. The system for introducing inefficiency may for example introduce sufficient friction into the system to maintain the position of the sheath or catheter after it has been deflected. The system enables self-locking after a catheter has been deflected.

In accordance with the present invention, a means for reducing efficiency or a means for introducing inefficiency is provided that allows the position of the catheter to be maintained after deflection and may provide feedback using the same system. Thus, a system or mechanism for introducing inefficiency is provided that is built into the rotation to linear translation system and reduces the mechanical efficiency of translation. The system for reducing efficiency or inefficiency introducing system allows for an opportunity to get feedback and enables curve retention using the same mechanism.

The system for introducing inefficiency is an inefficiency generator that provides increasing resistance to actuation, resulting in increasing force or increased force to actuate the actuator upon increasing deflection of the catheter, which provides one or more advantages of provide feedback of curve deflection and/or curve retention.

The steerable catheter control handle utilizes a threadable engagement for converting the rotational movement of the knob into linear movement of a slide assembly for actuating one or more pull wires to deflect a portion of a catheter. In accordance with the invention, the threadable engagement between the knob and slide assembly is provided through an angled thread that functions as a means for reducing efficiency of the system or an inefficiency introducing system that provides sufficient tactile feedback from the deflection of the catheter and additionally allows the deflection of the catheter to be maintained. As the sheath is actuated it gets harder and harder to actuate which gives the user a feel for what is happening at the distal tip. In other words, the inefficiency introducing system provides increasing resistance to actuation, resulting in increasing force to actuate the actuator, upon increasing deflection of the catheter. The threadable engagement comprising an angled thread provides actual feedback regarding the deflection of the catheter, for example of the distal tip of the catheter. The threadable arrangement introduces inefficiency into the system that prevents reverse translation of the slide assembly. The system for reducing mechanical efficiency provides a self-locking mechanism so that once the catheter has been deflected to the desired level of deflection it is difficult to move the position of the catheter in the absence of a force from the user or rotation of the knob by the user.

As such, the present inventors have discovered that providing an inefficiency generator in the rotation to linear translation system itself, the system is simpler to manufacture, more robust and utilizes less components and as such is less likely to break and can additionally help provide the user with true feedback of the deflection of the catheter from the system itself. However, by changing the thread angle it increases the slope of the force/deflection graph and increases the force feel between the minimum deflection and the maximum deflection of the catheter. This way the user is able get better tactile feedback to better ascertain the level of deflection at the distal end.

In some embodiments the inefficiency generator may be provided that provides a feedback mechanism that provides simulated feedback to provide the user with a picture of what is happening at the distal tip of the catheter. In some such examples, the feedback mechanism may be built into the steerable handle. In some such instances, the feedback mechanism may interact indirectly with a pathway of the rotation to linear system in order to provide the user with simulated feedback for providing an indication of the distal tip deflection.

In some embodiments of the present invention, the introduction of an inefficiency generator enables a physician to curve a steerable sheath or catheter with a dilator and a needle inserted, where the inefficiency generator enables the steerable sheath handle to maintain the sheath curvature under higher pull wire tension.

Additionally, some embodiments of the present invention employing an inefficiency generator provide sufficient tactile feedback allowing the physician to rely on tactile feedback from the knob turning, to know the relative sheath curvature and the sheath neutral zone. Specifically, this tactile feedback means that the knob becomes harder to turn as the sheath curvature increases. This "weighted steering" not only informs the user the relative sheath curvature, but also the neutral zone location (i.e. when the knob is the easiest to turn).

In one broad aspect, embodiments of the present invention comprise, inter alia, a steerable catheter control handle for deflecting a catheter. The control handle comprises an actuator, one or more pull wires, and a means for deflecting the pull wires by translating the actuation of the actuator into movement of the pull wires. A distal end of each of the one or more pull wires is coupled to the catheter and a proximal end of each of the pull wires is coupled to the means for deflecting the pull wires. The control handle further comprises an inefficiency generator for interacting with the means for deflecting the pull wires, wherein the inefficiency generator substantially increases a force required to actuate the actuator to deflect the catheter.

In another broad aspect, embodiments of the present invention comprise, inter alia, a steerable catheter control handle for deflecting a catheter. The control handle comprises an actuator, and a means for deflecting the catheter upon actuation of the actuator, the means for deflecting the catheter being coupled to the actuator. The steerable catheter control handle additionally comprises an inefficiency generator configured to interact with the means for deflecting the catheter, wherein the inefficiency generator provides increasing resistance to actuation, requiring an increasing force required to actuate the actuator upon increasing deflection of the catheter.

As a feature of this broad aspect, a force required to actuate varies substantially based on an amount of deflection of the catheter.

As another feature of this broad aspect, a force required to actuate varies substantially based on an amount of actuation of the actuator.

As still another feature of this broad aspect, the inefficiency generator enables weighted steering by providing feedback to the user regarding a relative degree of deflection of the catheter.

The inefficiency generator in accordance with the invention enables curve retention allowing the deflection of the catheter to be substantially maintained at a desired curvature after it has been deflected, and wherein the inefficiency generator enables self-locking such that a force required to actuate is greater than the force exerted on the actuator by the one or more pull wires from the deflection of the catheter, wherein the inefficiency generator allows the deflection of the catheter to be maintained in the absence of actuation of the actuator.

The inefficiency generator in accordance with the invention enables curve retention allowing the deflection of the catheter to be substantially maintained at a desired curvature after it has been deflected. As an example of this feature, the inefficiency generator enables self-locking such that a force required to actuate is greater than the force exerted on the actuator by the catheter from the deflection of the catheter, wherein the inefficiency generator allows the deflection of the catheter to be maintained in the absence of actuation of the actuator. The catheter comprises pull wires where the actuator is operable to deflect the pull-wires, the inefficiency generator enables self-locking such that a force required to actuate is greater than the force exerted on the actuator by the pull-wires from the deflection of the catheter.

As another feature of this aspect, the means for deflecting the catheter comprises a rotation to linear translation system. The catheter comprises pull wires where the actuator is operable to deflect the pull-wires, the means for deflecting the catheter comprises the means for deflecting the pull wires comprises a rotation to linear translation system.

The actuator comprises a rotatable knob and the means for deflecting the catheter comprises a rotation to linear translation system comprising a slide assembly that is coupled to the rotatable knob via a threadable engagement. The catheter comprises pull wires where the actuator is operable to deflect the pull-wires, the means for deflecting the pull wires comprises a rotation to linear translation system comprising a slide assembly that is coupled to the rotatable knob via a threadable engagement.

In accordance with the invention, the inefficiency generator comprises one or more angled threads that define the threadable engagement. In a particular embodiment of this, the threadable engagement is defined by external angled threads on a shaft of the slide assembly, and corresponding internal angled threads on the knob that are engageable therewith.

In one example of this embodiment, the angled threads have an angle of greater than about zero degrees and less than about 180 degrees.

In one such example, the angled threads have an angle of greater than about 160 degrees and less than about 180 degrees. In a specific example of this, the angled threads have an angle of between about 160 degrees and 163 degrees.

In some embodiments, the inefficiency generator provides actual feedback of deflection of the catheter. In other embodiments, the inefficiency generator provides simulated feedback of deflection of the catheter.

In some embodiments of the present invention, the slide assembly is moveable within an inner housing of the steerable catheter control handle.

In one example of this, the inefficiency generator comprises a gradient friction device, positioned within the handle housing, wherein the gradient friction device interacts with the slide assembly upon movement of the slide assembly, wherein the gradient friction device provides increasing friction upon increasing linear translation of the slide assembly within the inner housing upon deflection of the catheter.

In a specific example of this, gradient friction device comprises opposing triangular friction pads positioned along an inner surface of the handle housing. In one instance of this example, the triangular frictional pads are positioned along one or more walls of the inner housing.

In another example of this, the inefficiency generator is defined by the inner housing, the inner housing comprising opposing walls wherein the opposing walls of the inner housing taper towards one another, from a middle of the inner housing towards proximal and distal ends of the inner housing, wherein the slide assembly experiences increasing friction between the slide assembly and the opposing walls of the inner housing, upon increasing linear translation of the slide assembly within the inner housing upon deflection of the catheter.

In a specific example of this, the slide assembly is engageable with the walls of the inner housing via a biasing mechanism. In a specific instance of this example, the biasing mechanism comprises a spring biased mechanism.

In one embodiment, the rotation to linear translation system comprises a single step rotation to linear translation system. In another embodiment, the rotation to linear translation system comprises a two-step rotation to linear translation system.

As a feature of this broad aspect, the steerable catheter control handle comprises a bi-directional control handle, wherein the means for deflecting the catheter is coupled to the catheter via one or more pull wires, where the means for deflecting the catheter comprises means for deflecting the one or more pull wires, the one or more pull wires comprising two pull wires, wherein the actuation of the actuator in first and second directions causes the means for deflecting the pull wires, to enable deflection of the catheter via actuation of each of the respective pull wires to impart desired curvature to the catheter in respective first and second deflection directions, and wherein the inefficiency generator results in increased force required to curve the catheter in each of the first and second deflection directions upon increasing deflection of the catheter.

As another feature of this broad aspect, the steerable catheter control handle comprises a unidirectional control handle, wherein the means for deflecting the catheter is coupled to the catheter via one or more pull wires, where the means for deflecting the catheter comprises means for deflecting the one or more pull wires, the one or more pull wires comprising a single pull wire, wherein actuation of the actuator causes the means for deflecting the one or more pull wires, to enable deflection of the catheter via actuation of the pull wire to impart desired curvature to the catheter in a single deflection direction, and wherein the inefficiency generator results in increased force required to curve the catheter in the single deflection direction upon increasing deflection of the catheter.

As another feature of the broad aspect, wherein the means for deflecting the catheter is coupled to the catheter via at least one of the one or more pull wires, where at least one of the one or more pull wires is coupled to a distal end of the catheter to enable deflection of the distal end of the catheter.

In a further broad aspect, embodiments of the present invention comprise, inter alia, a control handle for a steerable catheter comprising a rotation to linear translation system for deflecting a catheter comprising a rotatable knob, a slide assembly, the rotatable knob being coupled to the slide assembly via a threadable engagement or arrangement, and at least one control wire, a distal end of the control wire being operable to be coupled to the catheter, and a proximal end or portion of the at least one control or pull wire being operable to be coupled to or coupled to the slide assembly. The control handle further comprising a means to introduce inefficiency into the rotation to linear translation system, wherein the means to introduce inefficiency is inherent or integral to a component of/in the rotation to linear translation system, wherein a rotation of the knob causes a linear movement of the slide assembly, to enable deflection of the catheter via actuation of the at least one control wire to impart a desired curvature to the catheter, and wherein the means to introduce inefficiency results in increased force required to actuate the at least one control wire to curve the sheath or catheter upon increasing deflection of the catheter (for example while providing an optimized handle design that provides ease of handling for the physician and ease of use.)

As a feature of this broad aspect, the means to introduce inefficiency substantially increases an amount of force required to rotate the knob and translate the slide assembly where this force varies substantially based on the amount of rotation and/or translation to provide enhanced tactile feedback (to the user) regarding the deflection of the catheter upon increasing deflection of the catheter.

The means to introduce inefficiency enables curve retention allowing the deflection of the catheter to be substantially maintained at a desired curvature after it has been deflected.

The means to introduce inefficiency enables self-locking such that a force required to rotate the knob to move the slide assembly is greater than the force exerted on the slide assembly from the catheter after deflection of the catheter, preventing a curvature of the catheter to be altered in the absence of input from a user without the actuation of the knob.

In accordance with the invention, the means to introduce inefficiency comprises one or more angled threads that define the threadable engagement or arrangement. As an example of this feature, the threadable arrangement or engagement is defined by external angled threads on a shaft of the slide assembly, and corresponding internal angled threads on the knob that are engageable therewith.

In one such embodiment, the angled threads have an angle of greater than about zero degrees and less than about 180 degrees. In one such example, the angled threads have an angle of greater than about 160 degrees and less than about 180 degrees. In a specific example, the angled threads have an angle of between about 160 degrees and 163 degrees.

In one such embodiment of the steerable catheter control handle, the threadable engagement is defined by external threads on a shaft of the slide assembly, and corresponding internal threads on the knob that are engageable therewith.

In some embodiments, the means to introduce inefficiency (which comprises an inefficiency generator) provides actual feedback of deflection of the catheter. In other embodiments, the means to introduce inefficiency (which comprises an inefficiency generator) provides simulated feedback of deflection of the catheter.

In one embodiment, the slide assembly is moveable within a housing of the handle (such an inner housing of the handle).

In one such example, the means to introduce inefficiency comprises a gradient friction device positioned within the handle housing, wherein the gradient friction interacts with the slide assembly upon movement of the slide assembly, wherein the gradient friction device provides increasing friction upon increasing linear translation of the slide assembly within the housing (such as the inner housing) upon deflection of the catheter. In a specific example of this, the gradient friction device comprises one more triangular friction pads (such as opposing triangular friction pads) positioned along an inner surface of the handle housing. I n a specific instance of this example, the one or more triangular frictional pads (such as the opposing triangular friction pads) are positioned along one or more walls of the inner housing.

In another such example, the means for generating inefficiency (which comprises an inefficiency generator) is defined by the housing (such as the inner housing), the housing comprising opposing walls, wherein the opposing walls of the housing taper towards one another, from a middle of the housing towards the proximal and distal ends of the housing, wherein the slide assembly experiences increasing friction between the slide assembly and the opposing walls of the housing, upon increasing linear translation of the slide assembly within the housing upon deflection of the catheter. In one specific example, the slide assembly is engageable with the walls of the housing via a biasing mechanism. In some examples, the biasing mechanism forms a part of the slide assembly. In a specific instance of this example, the biasing mechanism comprises a spring biased mechanism.

As a feature of this broad aspect, wherein the means for deflecting the catheter comprises a rotation to linear translation system, the rotation to linear translation system comprising a single step rotation to linear translation system.

As another feature of this broad aspect, wherein the means for deflecting the catheter comprises a rotation to linear translation system, the rotation to linear translation system comprising a two-step rotation to linear translation system.

As another feature of this broad aspect, the steerable catheter control handle comprises a bidirectional control handle, wherein the means for deflecting the catheter is coupled to the catheter via at least one control wire, where the means for deflecting the catheter comprises a means for deflecting the at least one control wire, wherein the at least one control wire comprises two control wires, wherein a rotation of the actuator (such as the knob) in first and second rotational directions (for example that causes linear movement of a slide assembly in respective first and second linear directions) enables deflection of the catheter via actuation of respective one of the two control wires to impart desired curvature to the catheter in respective first and second deflection directions, and wherein the means to introduce inefficiency results in increased force required to curve the catheter in each of the first and second deflection directions upon increasing deflection of the catheter.

As another feature of this broad aspect, the steerable catheter control handle comprises a unidirectional control handle, wherein the means for deflecting the catheter is coupled to the catheter via at least one control wire, where the means for deflecting the catheter comprises a means for deflecting the at least one control wire, the at least one control wire comprises a control wire, wherein the rotation of the actuator [comprising a knob causes a linear movement of the slide assembly via rotation of the actuator (the knob)], enables deflection of the catheter via actuation of the control wire to impart desired curvature to the catheter in a single deflection direction; and wherein the means to introduce inefficiency results in increased force required to curve the sheath or catheter in the single deflection direction upon increasing deflection of the catheter.

As another feature of this broad aspect, wherein the means for deflecting the catheter is coupled to the catheter via at least one control wire, where the means for deflecting the catheter comprises a means for deflecting the at least one control wire, wherein the at least one control wire is coupled to a distal end of the catheter to enable deflection of the distal end of the catheter.

In a further broad aspect, embodiments of the present invention comprise, inter alia, a steerable catheter control handle for a catheter, comprising, a rotatable knob, and a slide assembly, the rotatable knob being coupled to the slide assembly via a threadable engagement, the threadable engagement comprising an angled thread. The catheter comprises at least one control wire, a proximal end or portion of the at least one control wire being coupled to the slide assembly or being couplable thereto, wherein a rotation of the knob causes a linear movement of the slide assembly (via rotation of the knob), to enable deflection of the catheter via actuation of the at least one control wire to impart a desired curvature to the catheter, and wherein the angled thread provides enhanced tactile feedback to the user regarding the deflection of the catheter (for example at a distal end thereof) upon increasing deflection of the catheter, and enables curve retention allowing the deflection of the catheter to be maintained at the desired curvature.

As a feature of this broad aspect, the angled thread provides enhanced tactile feedback to the user such that there is a substantial difference in a level of tactile feedback provided upon a minimum deflection of the catheter and a maximum deflection of the catheter.

In another broad aspect (as noted previously) embodiments of the present invention comprise a steerable catheter control handle for deflecting a catheter. The control handle comprises, inter alia, an actuator, and a means for deflecting the catheter upon actuation of the actuator, the means for deflecting being coupled to the actuator. The steerable catheter control handle additionally comprises an inefficiency generator configured to interact with the means for deflecting the catheter, wherein the inefficiency generator provides increasing resistance to actuation, requiring increasing force required to actuate the actuator upon increasing deflection of the catheter.

As a feature of this broad aspect, one or more pull wires each have a distal end that is coupled to the catheter; and the means for deflecting the catheter comprising a means to deflect the one or more pull wires, a proximal portion of each of the one or more pull wires is coupled to the means for deflecting the one or more pull wires. As an example of this feature, the means for deflecting the one or more pull wires comprises a rotation to linear translation system.

As another feature of this broad aspect, the means for deflecting the catheter comprises a translation mechanism for translating the actuation of the actuator into deflection of the catheter.

As another feature of this broad aspect, the inefficiency generator interacts indirectly with the means for deflecting the catheter.

As still another feature of this broad aspect, the inefficiency generator interacts directly with the means for deflecting the catheter.

As still another feature of this broad aspect, the inefficiency generator is defined by the means for deflecting the catheter. As an example of this feature, the inefficiency generator comprises a tactile feedback mechanism that provides tactile feedback regarding a degree of deflection of the catheter upon actuation of the actuator. As an example of this feature, the tactile feedback mechanism comprises a neutral zone indicator.

As still another feature of this broad aspect, the inefficiency generator is integral with the means for deflecting the catheter.

As another feature of this broad aspect, the inefficiency generator provide discrete feedback regarding a degree of deflection of the catheter upon actuation of the actuator.

As another feature of this broad aspect, the inefficiency generator provides a range of feedback regarding a degree of deflection of the catheter upon actuation of the actuator, upon increasing deflection of the catheter.

In one broad aspect, embodiments of the present invention comprise, inter alia, a steerable catheter control handle for deflecting a catheter, the control handle comprising: an actuator; one or more pull wires each having a distal end being coupled to the catheter; a means for translating the actuation of the actuator into deflection of the one or more pull-wires for deflecting the catheter by translating the actuation of the actuator into movement of the pull wires, the means for translating the actuation being coupled to the actuator; a proximal portion of each of the one or more pull wires being coupled to the means for translating the actuation (which in one example comprises a translation mechanism or actuation mechanism); and an inefficiency generator configured to interact with the means for translating the actuation of the actuator for actuating or deflecting the one or more pull wires to deflect the same; wherein the inefficiency generator (substantially) increases a force required to actuate the actuator to deflect the catheter.

In another broad aspect, embodiments of the present invention comprise, inter alia, a steerable catheter control handle for deflecting a catheter, the control handle comprising an actuator and one or more pull wires each having a distal end being coupled to or for coupling to the catheter; a translation mechanism (or in other words an actuation mechanism) for translating the actuation of the actuator into defection of the catheter by deflecting the one or more pull wires to deflect the catheter, the translation mechanism being coupled to the actuator; a proximal portion of each of the one or more pull wires being coupled to the translation mechanism for deflecting the one or more pull wires; and an inefficiency generator configured to interact with the means for deflecting the one or more pull wires; wherein the inefficiency generator substantially increases a force required to actuate the actuator to deflect the catheter upon increasing deflection of the catheter.

With specific reference now to the drawings in detail, it is stressed that the particulars shown are by way of example and for purposes of illustrative discussion of certain embodiments of the present invention only. Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

In accordance with the present invention, a steerable catheter control handle is provided for deflecting a catheter, the control handle comprises, inter alia, an actuator and a means for deflecting the catheter. In some embodiments, the control handle is operable to deflect the catheter through actuation or deflection of one or more pull or control wires that are coupled to the means for deflecting the catheter at one end and the sheath or catheter at another end. The control handle is provided with a means for deflecting the one more pull wires by translating the actuation of the actuator into movement of the pull wires. In other words the control handle provides a means for translating the actuation of the actuator into movement of the pull wires. In some such examples, a distal end of each of the one or more pull wires is coupled to the sheath or catheter and a proximal end of each of the pull wires is coupled to the means for deflecting the pull wires [means for translating the actuation of the actuator into movement of the pull wires].

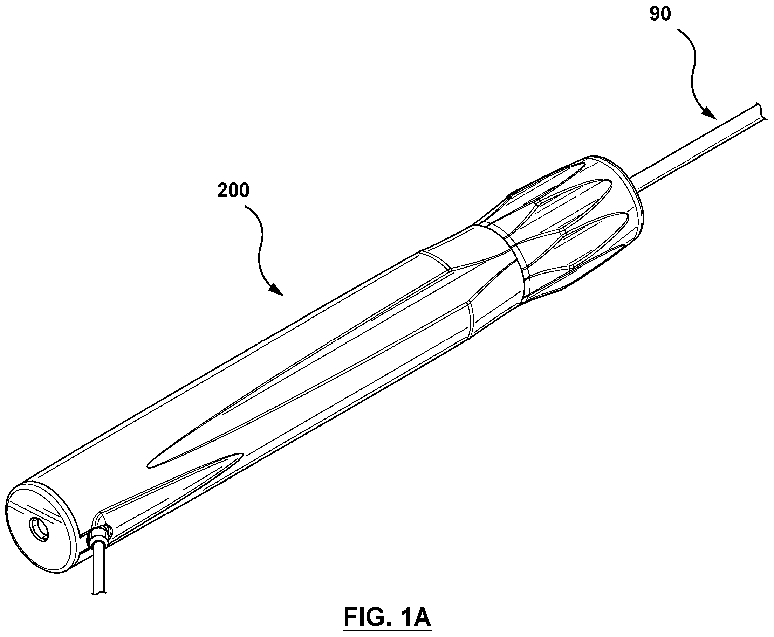

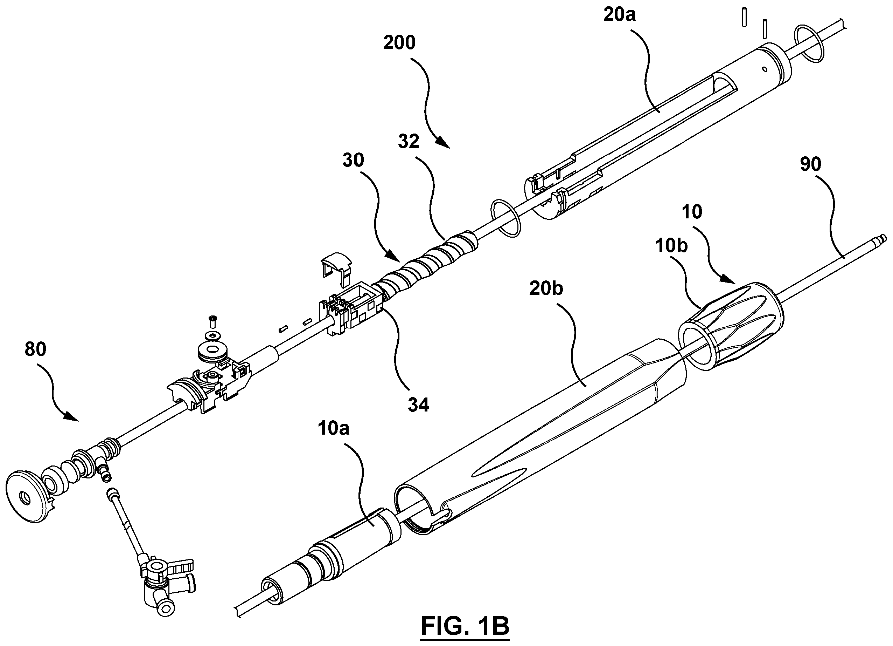

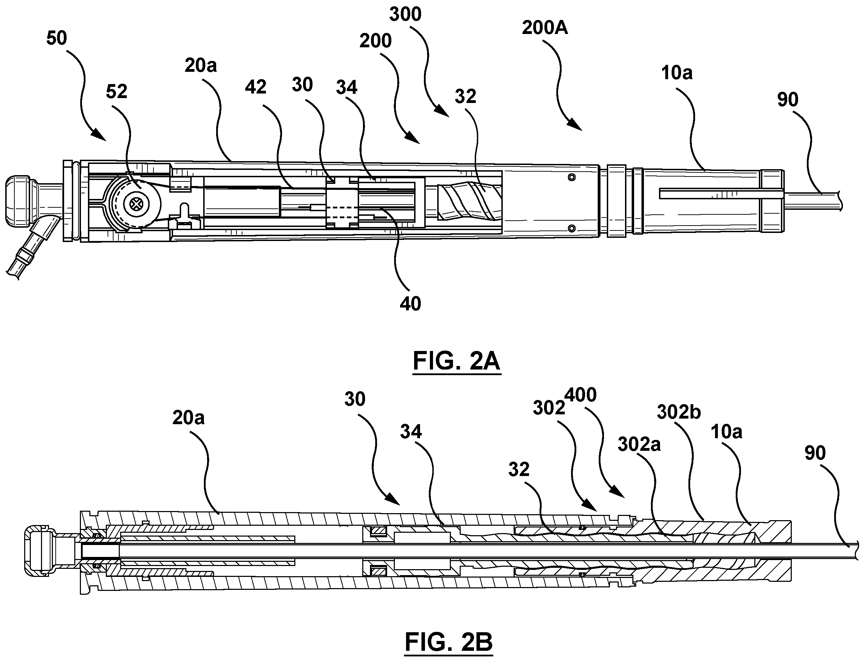

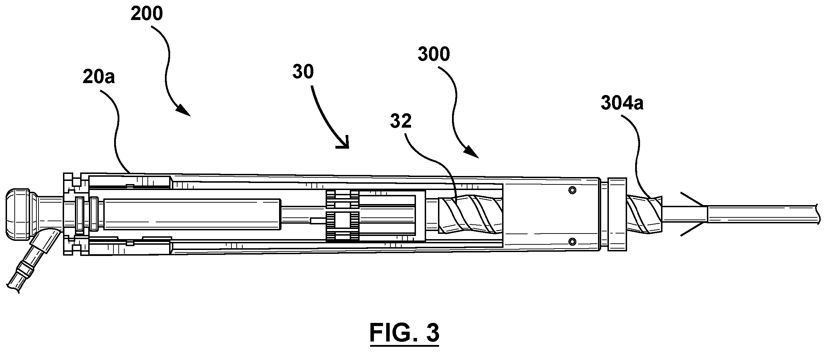

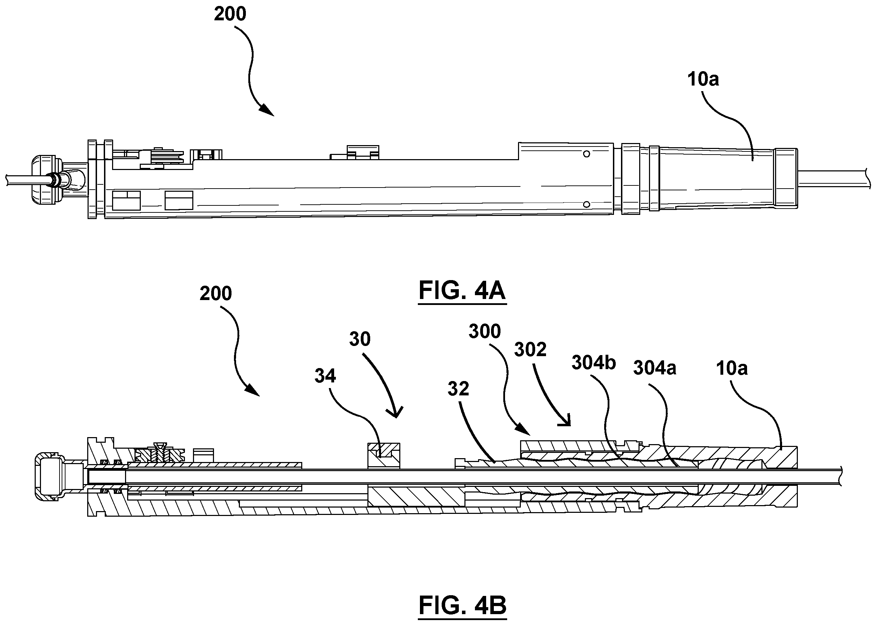

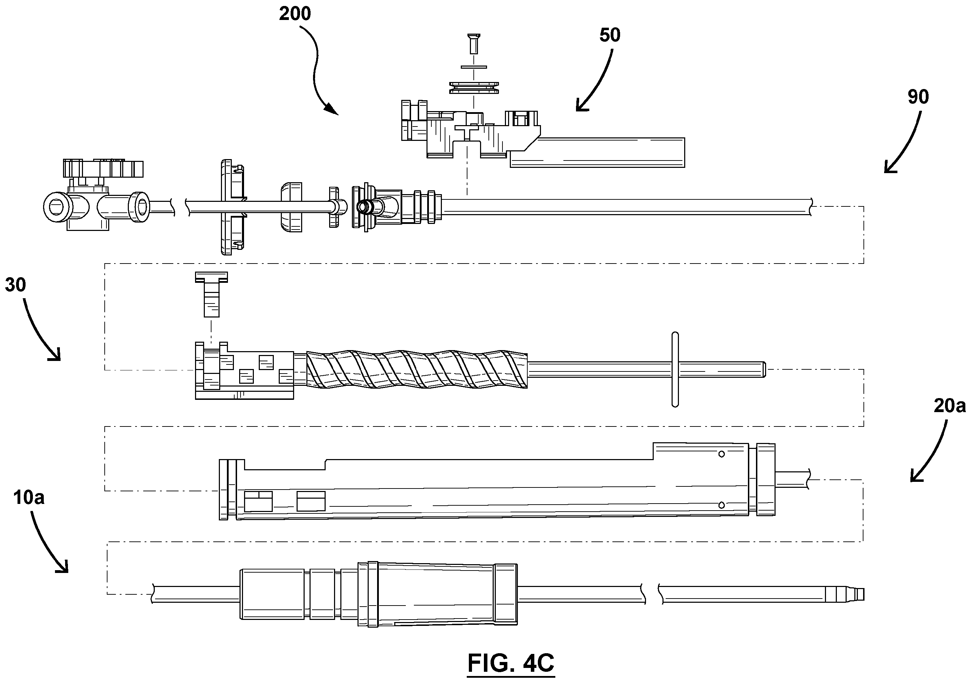

In one embodiment of the present invention, as illustrated in Fig. 1A and IB, and Fig. 4, a steerable catheter control handle 200 is disclosed for deflecting a medical device such as a sheath or catheter 90. The control handle 200 is coupled to the sheath or catheter 90 via proximal hub portion 80 of the steerable sheath or catheter 90. The control handle 200, as additionally shown in Figs 2A and 2B and Fig. 3, comprises a rotation to linear translation system 300 that comprises a rotatable knob 10 whose rotational movement is converted into linear movement of the slide assembly 30 via a threadable engagement 302. In other words, the knob 10 is coupled to the slide assembly via the threadable arrangement or engagement 302 such that upon rotation of the knob 10, the slide assembly 30 is moveable linearly within a portion of the housing 20, specifically the inner housing 20a, and the linear movement of the slide assembly 30 is converted into deflection of a catheter or sheath 90 via one or more control or pull wires 40, 42. In this particular example, as additionally shown in Fig. 2A and Fig. 2B, the threadable engagement 302 is defined by external threads 302b formed on a shaft or bolt 32 of the slide assembly 30 and corresponding internal threads 302a on the knob 10 that are engageable therewith. In one embodiment, the knob 10 comprises an external knob 10b (Fig. 1) that is coupled to an inner knob 10a, where the inner threads 302a are defined by the inner knob 10a.

The inefficiency generator is additionally provided for interacting with the means for deflecting the catheter (which deflects the catheter by actuating the pull wires). The inefficiency generator functions to introduce inefficiency into conversion of the actuation into the deflection of the catheter. The inefficiency generator substantially increases a force required to actuate the actuator upon increasing deflection of the catheter. In other words, the inefficiency generator provides increasing force feel or resistance to actuation to deflect the catheter upon increasing deflection of the catheter.

The inefficiency generator is integral to or inherent in means for translating the actuation into deflection of the catheter, such as in the means for deflecting the pull wires. In some such examples, the inefficiency generator interacts directly with the means for translating the actuation into the deflection of the catheter. In other words, the inefficiency generator interacts directly with the means for deflecting the catheter. In other embodiments the inefficiency generator interacts indirectly with the means for translating the actuation into the deflection of the catheter. In other words, the inefficiency generator interacts indirectly with the means for deflecting the catheter. The inefficiency generator functions to introduce or generate energy loss into the system that is provided for converting the actuation of an actuator of the steerable control handle into deflection of the catheter, such as by actuation of one or more pull wires.

The inefficiency generator 400 substantially increases a force required to actuate the actuator to deflect the sheath or catheter 90 with increasing deflection of the catheter. In other words, the inefficiency generator interacts the means for deflecting the catheter to provide increasing resistance to actuation of the actuator upon increasing deflection of the catheter. The inefficiency generator results in increased force required to curve the sheath while providing an optimized handle design that provides ease of handling for the physician and ease of use. In some embodiments of the present invention, the force required to actuate varies substantially based on an amount of deflection of the sheath or catheter 90 and/or in other embodiments the force required to actuate varies substantially based on an amount of actuation of the actuator.

The inefficiency generator functions to provide relative feedback regarding the deflection of the catheter at for example the distal tip of the catheter and/or retain the curvature. The inefficiency generator enhances energy loss in the system to reduce the mechanical efficiency in actuation to deflection of the catheter 90, to provide one or more advantages such as enhancing tactile feedback received by the user through the actuator regarding the distal tip deflection by making it harder to actuate and/or deflect, and/or additionally allows a curvature or deflection of the sheath or catheter 90 to be substantially maintained at the desired curvature or deflection after the catheter 90 has been deflected to the desired curvature by. The inefficiency generator introduces friction into the system to reduce the mechanical efficiency of converting the actuation of the actuator into deflection of the sheath or catheter 90. Some embodiments of the present invention that provide the advantage of providing enhanced tactile feedback to the user, may additionally help minimize failure of the control or pull wires (for example resulting from the pull or control wires from breaking from the pull ring), as the physician may be able to more readily ascertain how much the catheter is being deflected, and as such may allow the physician to be more careful under increasing deflection of the catheter.

The inefficiency generator 400 enables self-locking such that a force required to actuate is greater than the force exerted on the actuator from the deflection of the catheter (for example by the pull wires once the catheter has been deflected) wherein the inefficiency generator allows the deflection of the catheter to be maintained in the absence of actuation of the actuator.

In a steerable catheter control handle 200 according to the invention, the means for deflecting the pull wires (or the means for translating the actuation into deflection of the catheter) comprises a rotation to linear translation system.

In some embodiments of the present invention, the inefficiency generator of the present invention may additionally provide neutral zone indication. The inefficiency generator may provide enhanced tactile feedback to the user, and as such may provide a significant differential in feel or tactile feedback when the sheath or catheter is in the neutral zone. In comparison to when the sheath or catheter is in its deflected position. More specifically, the inefficiency generator allows the user to turn the knob more easily when the sheath or catheter is in the neutral position. As such the handle actuator such as a knob may feel relatively more loose when the sheath or catheter in its neutral position than when the sheath or catheter has been substantially deflected.

In some such embodiments, the inefficiency generator provides for enhanced feedback such as tactile feedback, in both embodiments when the sheath or catheter may be usably by itself and in embodiments where the sheath or catheter may be usable in combination with other devices [for example, one or more devices that may be inserted through the sheath or catheter (internal components) or for example, one or more devices that the sheath or catheter itself may be inserted through (external components).

In some such embodiments, where the sheath or catheter is usable with internal or external components, the tactile feedback that is provided is correlated based on how rigid or stiff the internal or external components are. For example, when there is internal or external resistance (for example resistance to deflection when one or more internal or external components are used with the sheath or catheter), the torque feel may be different. For example, the inefficiency generator may provide a significant difference in the amount of tactile feedback that is received by the user depending on how hard it may be to turn the actuator. In some such examples, the inefficiency generator may provide sufficient tactile feedback, in each of the scenarios (i) for example when an empty sheath or catheter is deflected, (ii) for example when a sheath or catheter is used with [such as within] an ablation catheter, (iii) for example when sheath or catheter is used with a dilator and a transseptal needle positioned within it [such as an RF transseptal needle or a mechanical transseptal needle]. In some such embodiments, the force to actuate the actuator (or the force to turn a knob of a rotation to linear system, may be greater for scenario (iii) than scenario (i).

As such, in some embodiments of the present invention, the inefficiency generator that is provided may provide differences in feedback which may be dependent on one or more devices located within the sheath or catheter, and one or more devices that the sheath or catheter may be positioned in.

In some such embodiments of the present invention, providing an inefficiency generator within an actuation mechanism for deflecting the pull wires within a rotation to linear system, the amount of actuation of the actuator that is required to deflect the sheath or catheter (such as the degree of turns needed to deflect the sheath or catheter in rotational system) may be the same as a system without an inefficiency generator, but the feel is different. The actuation mechanism with an inefficiency generator may provide greater feedback than an actuation mechanism lacking the same. In some such embodiments, the feedback may help minimize failure at the point of contact of the wire with the sheath [for example by the wire breaking from the at the pull ring where the pull wire is attached to the sheath via a pull ring]. As such in some embodiments of the present invention, the inefficiency generator may provide an additional advantage of heightened feedback to reduce risk of breakage of the pull wires and/or the risk of failure at the point of contact or connection to the sheath or catheter.

The novel control handle 200 of the present invention provides a means to introduce inefficiency or an inefficiency generator 400 into the rotation to linear translation system 300, wherein the means to introduce inefficiency or inefficiency generator 400 interacts with the rotation to linear translation system 300. In some embodiments, the inefficiency generator is integral to or inherent in the rotation to linear system. In other words, the inefficiency generator interacts directly with the rotation to linear system. In other embodiments the inefficiency generator interacts indirectly with the rotation to linear system.

The inefficiency generator functions to reduce the mechanical efficiency of the rotation to linear translation system in translating the rotation of an actuator into linear movement of the pull or control wires. The reduction in mechanical efficiency makes it harder to deflect or curve the sheath requiring increased amount of force compared to a more efficient system in order to curve the sheath or catheter 90. In some embodiments this may provide one or more advantages of providing feedback to the user regarding a relative degree of deflection of the catheter. The inefficiency generator enables weighted steering and curve retention allowing the deflection of the catheter to be substantially maintained at a desired curvature after it has been deflected.

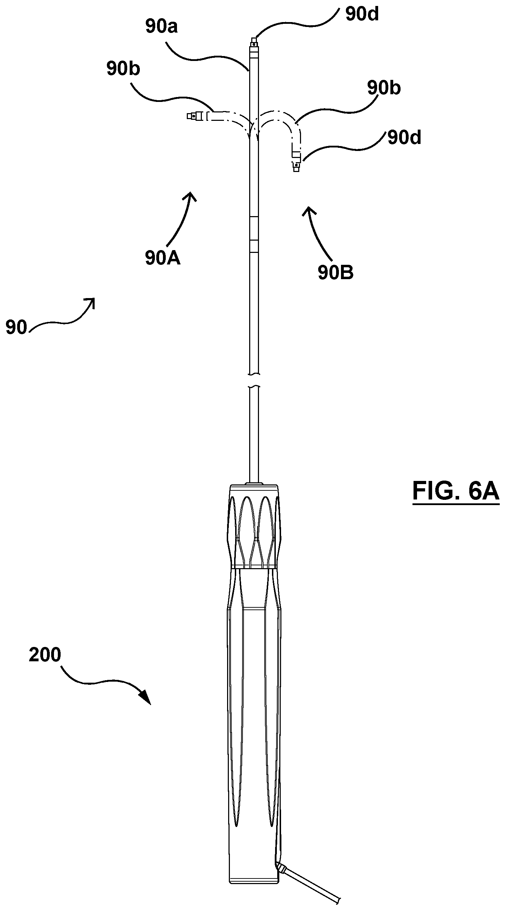

With reference now to Figs. 1A-6C, a control handle 200 for a steerable catheter 90 is provided comprising a rotation to linear translation system comprising a rotatable knob 10 is provided as noted previously, that is actuable about a longitudinal axis of the control handle 200. The actuator comprises a rotatable knob 10 and the means for deflecting the pull wires (or in other words the means for translating the actuation into deflection of the pull wires) comprises a rotation to linear translation system comprising a slide assembly 30 that is coupled to the rotatable knob 10 via a threadable engagement. More specifically, the rotatable knob 10 is coupled to the slide assembly 30 via a threadable engagement, where a distal end of at least one control or pull wire 40, 42 is coupled the catheter, and a proximal end of the at least one control or pull wire 40, 42 is coupled to the slide assembly 30. The rotation of the knob 10 causes a linear movement of the slide assembly 30 via rotation of the knob 10 to enable deflection of the sheath or catheter 90 via actuation of at least one the one or more control or pull wires 40, 42 to impart desired curvature to the sheath or catheter, for example along a distal end thereof.

The control handle 200 includes a means for introducing inefficiency or inefficiency generator 400 into the rotation to linear translation system, where the means to introduce inefficiency or inefficiency generator 400 is integral to or inherent in the rotation to linear translation system. In accordance with one or more embodiments of the present invention, the means of introducing inefficiency or the inefficiency generator 400 results in a reduction in the mechanical efficiency in converting the rotational movement of the rotatable knob 10 into linear movement of the slide assembly 30.

The inefficiency generator 400 results in increased force required to curve the sheath or catheter 90 while providing an optimized handle design that provides ease of handling for the physician and ease of use. In some such examples, the inefficiency generator 400 requires more force or increased amount of force relative to a more efficient system in order to curve the sheath or catheter 90, which makes it harder to turn the knob 10 to deflect the sheath or catheter 90. In other words, the inefficiency generator 400 substantially increases the amount of force required to deflect the sheath or catheter. The inefficiency generator enables the force required to deflect to vary substantially based on the amount of deflection of the sheath or catheter 90 and the amount of rotation of the knob. This provides the advantages of providing relative feedback regarding degree of deflection of the distal tip of the catheter and/or substantially retaining the distal tip curvature.

The means to introduce inefficiency, i.e. the inefficiency generator, substantially increases or adds to or enhances the amount of force required to rotate the actuator including knob 10 and translation of the slide assembly 30, where this force varies substantially based on the amount of rotation of the actuator and translation of the slide assembly 30 to provide enhanced tactile feedback to the user regarding the behavior deflection of the catheter.

The inefficiency generator is embedded within the rotation to linear mechanism 300 of the control handle 200 which provides a control handle 200 that requires an increased amount of force to curve the sheath while providing an optimized sleek handle design that provides ease of handling for the physician and ease of use.

Thus, the control handle 200 of the present invention, provides a handle with limited exterior dimensions that are optimized for ease of use and handling. In some such embodiments, the means for introducing inefficiency or inefficiency generator 400 is provided in the rotation to linear mechanism 300 of the control handle 200.

### Angled thread

In accordance with the present invention, the steerable catheter control handle 200 comprises, a means for introducing inefficiency or an inefficiency generator 400 that comprises one or more angled threads 304 that form or define the threadable engagement 302. The angled threads 304 result in increased force required to curve the sheath or catheter 90 while providing an optimized handle design that provides ease of handling for the physician and ease of use. The use of angled threads 304 to decrease the mechanical efficiency of a rotation to linear system allows the efficiency reduction to be incorporated into the mechanism of operation of the control handle 200 itself. This provides an optimized handle which may reduce the number of components within the handle, and as such may provide a more robust design, and may reduce complexity and cost of manufacturing.

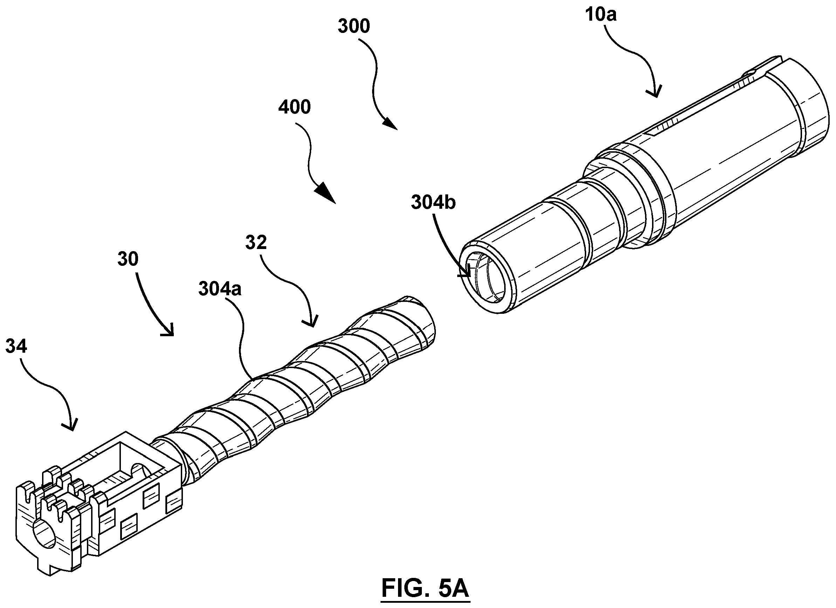

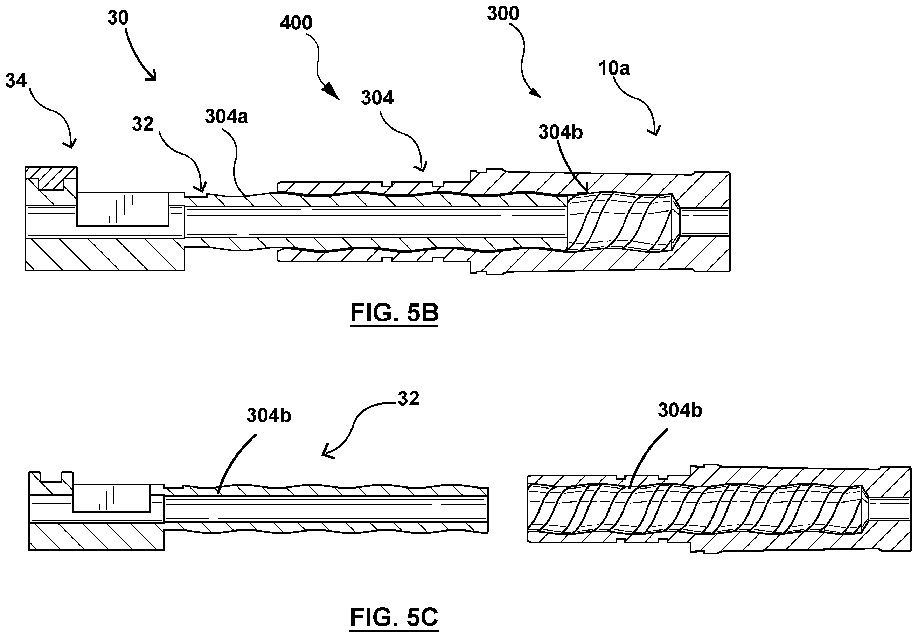

In the particular example shown in Fig. 1 and Figs. 2A-2B, the steerable catheter control handle 200 comprises a means for deflecting the pull wires that comprises a rotatable to linear translation system, the system includes a rotatable actuator such as the control knob 10 that is coupled to the slide assembly 30 via a threadable arrangement 302 or in other words a threadable engagement 302. In this particular example, as shown in Fig. 3 and 4B and 4C (and additionally shown in Fig. 5A and Figs. 5B, 5C), the threadable engagement 302 is defined by angled external threads 304, (angled external threads 304a formed on a shaft or bolt 32 of the slide assembly 30 and corresponding angled internal threads 304b on the knob 10 that are engageable therewith). In one embodiment, the angled internal threads 304b are defined by the inner knob 10a.

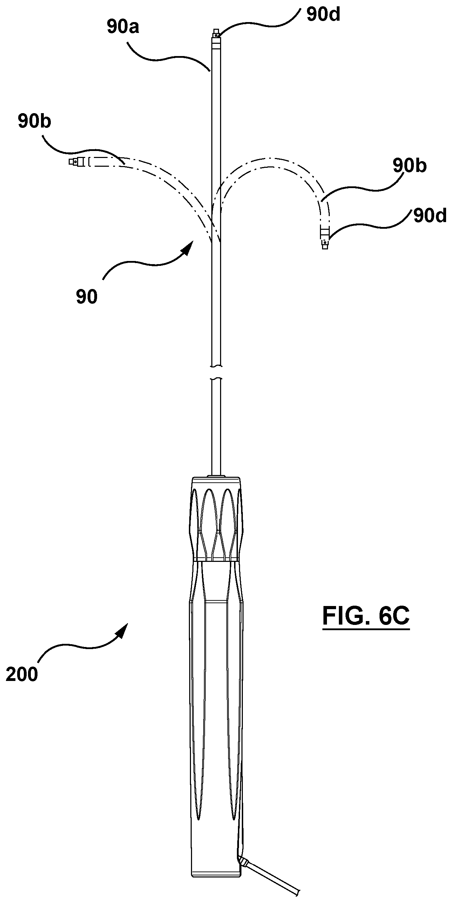

In some embodiments, as shown in Figs. 1A-6C, the angled threads 304 have an angle Z have an angle that is greater than about zero degrees and less than about 180 degrees. In some such embodiments, the angled threads 304 have an angle Z of greater than about 160 degrees and less than about 180 degrees. In an example of this, the angled threads 304 have an angle Z of between about 160 degrees and 163 degrees. In the particular embodiment shown in Figs. 1A-6C, the angled threads 304 have an angle Z of about 163 degrees. In one such example, as shown in Fig. 6C, angled external threads 304a are shown on the bolt or shaft 32 of have an angle Z of about 163 degrees.

In the particular example as shown herein above, specifically with respect to Fig. 2A, where the angled thread 304 defines the inefficiency generator 400, the inefficiency generator 400 provides increased force or substantially increases the force required to rotate the knob 10 to curve or deflect the sheath or catheter 90. In accordance with the present invention, a means is provided to introduce inefficiency or an inefficiency generator 400 is provided or built into the rotation to linear translation system, where the means to introduce inefficiency or inefficiency generator 400 is integral to or inherent in the rotation to linear translation system.

In the example in accordance with the invention as shown, where a rotation to linear system is provided as shown in Figs. 1A-6C, specifically with reference to Fig. 2A, the rotation of the knob 10 causes a linear movement of the slide assembly 30 via rotation of the knob 10, to enable deflection of the catheter 90 via actuation of the at least one control wire to impart desired curvature to the catheter 90. The angled or tapered thread 304, for example as shown in Figs. 5A, 5B, and 5C introduce inefficiency into the system such that the mechanical efficiency in translating the knob rotation into the linear actuation of the slide assembly 30 is reduced. This results in the reduction in the reverse mechanical efficiency of the system as well. Thus the angled or tapered thread 304 reduces the efficiency of the system in translating the linear movement or actuation of the slide assembly 30 under tension from the pull or control wires, into rotation of the knob 10.

The inefficiency that is introduced through the tapered or angled thread 304 leading to increased resistance or more force or increased amount of force to deflect the catheter 90 in the forward actuation that is in the actuation of the control knob 10 to cause linear movement of the slide assembly 30. The force required to deflect varies substantially based on the amount of deflection of the catheter. As shown in Figs. 6A-6C, a range of deflection of the catheter 90 is shown in examples of the steerable catheter control handle 200. The inefficiency generator is operable to provide relative feedback to the user regarding the deflection of the catheter 90 at the distal tip. The steerable catheter control handle of claim 23, wherein angled or tapered thread 304 provides enhanced tactile feedback to the user such that there is a substantial difference in a level of tactile feedback provided upon a minimum deflection [for example as shown by 90a] of the catheter and a maximum deflection [for example as shown by 90b] of the catheter.

The angled threads provide a substantial difference in the amount of force required to deflect the catheter 90 to cause minimum deflection 90a of the sheath or catheter 90 and the force required to cause maximum deflection 90b of the sheath or catheter 90. In some such examples, the tapered or angled threads 304 inform the user of the relative deflection or curvature of the sheath or catheter 90 as well the neutral zone location. The user may be able to ascertain the neutral zone location as the location where the knob 10 may be the easiest to turn, where the slide assembly 30 may be positioned within the handle in its substantially unactuated position where it has not yet substantially been moved by the rotation of the knob 10, where the catheter 90 deflection is a minimum deflection 90a of the catheter 90. The neutral zone position 30N of the slide assembly 30 may be observed in Figs. 2A-2B, as well as Figs. 4A, 4B and 4C. In one example, the position of the slide assembly 30 at maximum actuation and thus maximum deflection of the catheter 90 [as shown by 90B], is shown in Figs. 2C and 2D.

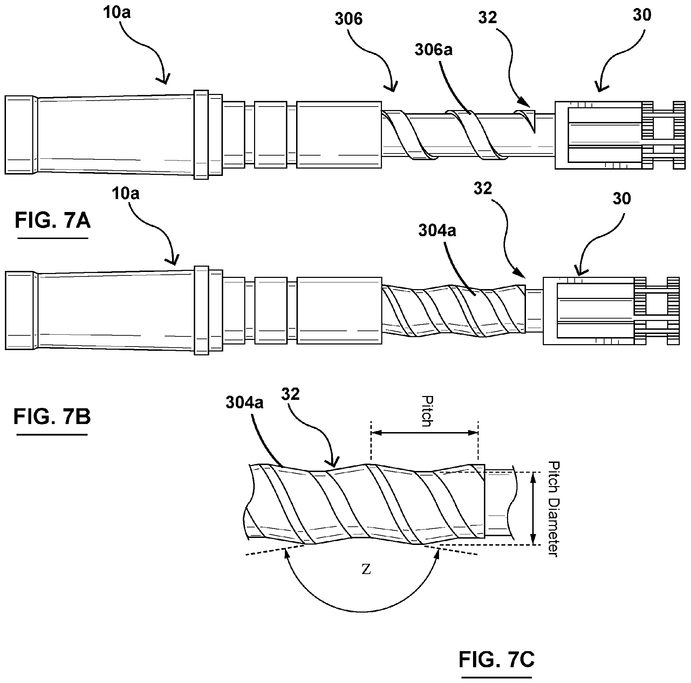

The inefficiency generator or inefficiency introducer 400 formed by the angled or tapered threads 304 reduces the mechanical efficiency in translating the knob 10 rotation into linear movement of the slide assembly 30. As such, the tapered or angled threads 304, additionally shown in Figs. 7B and 7C, substantially decrease the efficiency of the translation system [making it more inefficient for the rotational movement of the knob 10 to be converted into linear movement of the slide assembly 30] and thus increase the amount of force required to rotate the knob 10, for example compared to a system that is more mechanically efficient in translating the rotational motion into linear translation of the slide assembly 30. In an illustrative example not forming part of the invention as claimed, a rotation to linear translation system comprising a knob 10 that is threadably engaged to the slide assembly 30 via square threads 306x that have angle of about zero degrees, as shown in Fig. 7A, is more efficient in translating rotational movement of the knob 10 into linear movement of the slide assembly 30. The square threads 306x comprise square threads 306a on the shaft or bolt 32 of the slide assembly 30 and corresponding internal threads 306b on the inner knob 10a.

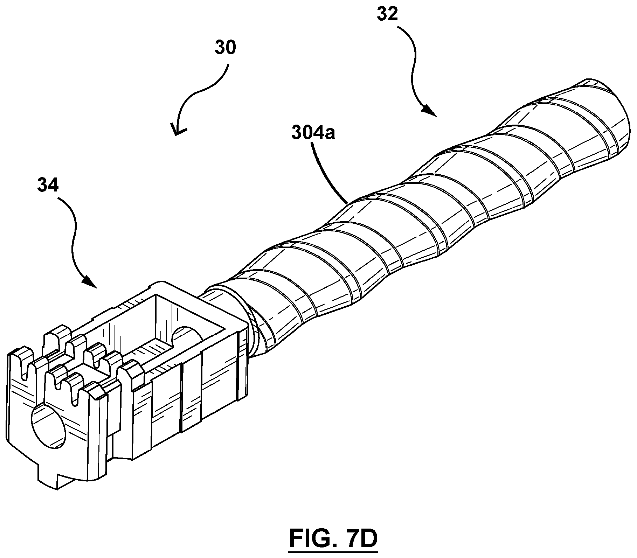

Unlike a system that uses square threads as shown in Fig. 7A, the use of the angled or tapered thread 304 in accordance with the present invention, as shown in Figs. 7B-7C and Fig. 7D, provide one or more additional advantages. In some such examples, the angled thread 304 allows the force required to rotate the knob 10 to be varied substantially based on the amount of rotation of the knob 10 and thus the translation of the slide assembly 30 to provide enhanced tactile feedback to the user regarding the behavior or deflection of the catheter, for example at the distal tip of the catheter. As the knob 10 is rotated more, it gets harder to actuate or rotate the knob 10. This enables feedback to be provided to the user. Additionally as noted above, introducing inefficiency into the system additionally results in the reduction in the reverse mechanical efficiency of the system as well. Thus, the angled or tapered thread 304 (including tapered thread 304a on the slide assembly 30, specifically on the bolt or shaft 32 of the slide assembly 30 and the tapered inner or internal thread 304b] reduces the efficiency of the system in translating the linear movement or actuation of the slide assembly 30 under tension from the pull or control wires, into rotation of the knob 10.

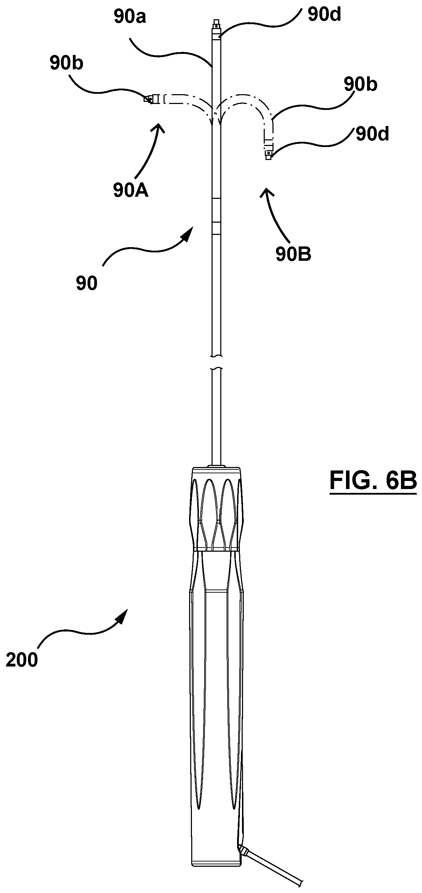

This facilitates curve retention allowing the deflection or curvature of the sheath or catheter 90 to be substantially maintained at the desired curvature after it has been deflected, for example any desired curvature between minimum deflection of the catheter 90a and maximum deflections of the catheter 90b as shown in Figs. 6A, 6B and 6C. The means to introduce inefficiency such as an inefficiency generator] 400 defined by the angled or tapered threads 304 enables self-locking such that a force required to rotate the knob 10 to move the slide assembly 30 is greater than the force exerted on the slide assembly 30 from the deflection of the catheter 90, preventing the deflection or curvature of the catheter 90 to be altered in the absence of input from the user. As such, the curve or position of the sheath or catheter 90 upon deflection of sheath or catheter 90 is maintained in the absence of or without the rotation actuation of the knob 10. The inefficiency generator 400, defined by angled threads 304, provides actual feedback of deflection of the sheath or catheter 90.

As such, in embodiments of the present invention, a steerable catheter control handle 200 for a catheter is provided, that comprises a rotatable knob 10 and a slide assembly 30, where the rotatable knob 10 is coupled to the slide assembly 30 via a threadable engagement 302, the threadable engagement 302 comprising an angled thread 304. The catheter comprises at least one control or pull wire 40, 42 where a distal end of the at least one control or pull wire 40, 42 is coupled to the sheath or catheter 90, and a proximal end of the at least one control wire or pull wire 40, 42 is coupled to the slide assembly 30. The rotation of the knob 10 causes a linear movement of the slide assembly 30 via rotation of the knob, to enable deflection of the sheath or catheter 90 via actuation of the at least one control wire 40, 42 to impart desired curvature to the catheter. The angled thread 304 provides enhanced tactile feedback to the user regarding the behavior or deflection of the sheath or catheter 90 at the distal end, and enables curve retention allowing the deflection or curvature of the sheath or catheter 90 to be maintained at the desired curvature. The angled thread 304 provides enhanced tactile feedback to the user such that there is a substantial difference in a level of tactile feedback provided upon a minimum deflection of the catheter and a maximum deflection of the sheath or catheter 90.

In some such embodiments of the present invention, neutral zone feedback is provided now the knob 10 may be easier to actuate or turn and thus may feel looser at the beginning before you start to turn. In some such embodiments of the present invention, the angled thread 304 provides true feedback of what is going on at the distal tip of the sheath or catheter 90 or the behavior of the sheath or catheter 90 under deflection. The angled thread 304 gives tactile feedback that provides a true picture of what is happening at the distal tip. In some such examples, the user may not get a constant feel. As the knob 10 is actuated and the sheath is tensioned, as a result of the angled thread 304, it gets harder and harder to actuate or turn the knob 10 and that is the feedback the user is getting. In other words, the system provides weighted steering giving feedback of what is happening at the distal tip (i.e. behavior of the sheath or catheter 90 upon deflection) such an indication of the degree or amount of deflection of the sheath or catheter 90 based on the actuation of the knob 10.

Since the inefficiency generator 400 comprises angled threads 304, with the knob 10 (such as the inner knob) and slide assembly 30 thread interaction, there is no or minimal feedback when the sheath or catheter 90 is not present or in other words not coupled to the steerable catheter control handle 200. It is the interaction of the whole assembly or system (that comprises the steerable catheter control handle 200 that is coupled to a sheath or catheter 90) where the sheath or catheter 90 (which in some examples includes pull or control wires that being tensioned as the knob 10 is being turned, which provides the user with feedback and gives the user a feel for what is happening in terms of deflection or behavior of the sheath or catheter 90. Anything internal to the translation mechanism or directly coupled thereto (in terms of the system including the actuator and the sheath and the translation system for converting the actuation of the actuation of the actuator into deflection of the sheath) may provide feedback if it is tapped into at any point along or within the system. As such, feedback may be obtained from any part of the system (for example through an inefficiency generator 400 that is part of the system or directly coupled to the system) where the system is formed by an actuator, a translation system and a sheath or catheter coupled thereto, where the translation mechanism is for converting the actuation of the actuator into deflection of the sheath or catheter 90. In some such examples, the inefficiency generator 400 provides true feedback of the deflection of the sheath or catheter.

In accordance with the present invention, the system comprising the inefficiency generator 400 provides feedback to the user is feedback regarding the amount of deflection of the sheath or catheter 90 resulting from the actuation or rotation of the knob 10. The system comprising the inefficiency generator 400 as defined by angled thread 304 also provides feedback regarding what is happening at the distal tip based on internal input resulting from actuation of the knob 10. As such, the feedback obtained may be from the level or deflection of the sheath or catheter 90. In some such examples, the steerable handle system comprising the steerable control handle 200 that is coupled to the sheath or catheter 90, also provides feedback regarding what is happening at the distal tip based on external input, for example resulting from the force against the distal tip (for example contact force) resulting from actuation of the knob 10. As such, the feedback obtained may be a result of the behavior of the distal tip (for example in a particular anatomy), for example the distal tip of the sheath or catheter 90 is against something hard or an obstruction that would be conveyed back to the user. As such, the feedback obtained may be from a force exerted by the sheath or catheter 90 at the distal tip or an external force exerted by something against the distal tip (where for example, the catheter or sheath 90 may not be able to deflect). In some such embodiments the feedback obtained is true feedback that is reflective of actual behavior of the sheath or catheter.

As noted previously, the angled thread 304 also enables self-locking. In some such examples, the efficiency for the linear to rotational mechanism (i.e. movement of the knob 10 due to movement of the slide assembly 30 due to tension in the pull wires) is at a point that the force in the system (even at maximum actuation) is not able to rotate the knob 10. In other words, the system where the inefficiency generator 400 is defined by angled threads 304 reduced the risk of reverse movement - that of the slide assembly 30 under tension to rotate the knob 10 resulting in movement of the position of the sheath or catheter 90), allowing the position of the sheath or catheter 90 to be maintained.

Furthermore, an inefficiency generator as defined by an angled thread 304 may allow for repeatability and may allow the position of the sheath or catheter 90 to be maintained under repeated actuation for example for the same movement. In some such examples, a steerable catheter control system is provided that is more reliable and/or more predictable.

In some embodiments of the present invention, inefficiency for rotation to linear translation (rotation of the knob 10 to translation of the slide assembly 30), as well as for the reverse movement - i.e. linear translation to rotational movement of the knob 10, may not be equal.

In one example where the inefficiency generator 400 defines an angled thread 304, the inefficiencies of the 'rotational to linear' movement and 'linear to rotational' movement are not equal. In one such example, where efficiencies that are not equal, the reverse movement - i.e. the 'linear to rotational' movement may be substantially or completely inefficient, which may allow for self-locking, as the force in the system from the deflected sheath will not be able to over-come the energy barrier built into the system from the inefficiency generator 400. As such, the inefficiency generator 400 adds inefficiency into the 'linear to rotational' movement. The inefficiency generator also adds inefficiency into the 'rotation to linear' movement. As the load increases [for example with the deflection of the sheath or catheter 90] effort increases and more resistant is felt by the user to actuate the knob 10, which provides feedback. In some such examples, the inefficiency in the system provides inefficiency in the 'rotation to linear' movement to provide adequate feedback while still allowing the user to actuate the knob and thus the sheath without difficulty, while providing inefficiency in the 'linear to rotational' movement or reverse movement to enable self-locking. In some such examples, the inefficiency is altered in the same way in that the inefficiency generator 400 for example comprising angled threads 304 introduced inefficiency into both the 'rotation to linear' movement and 'linear to rotational' movement. However, in some such examples, the magnitude of the two inefficiencies (or inefficiencies in the two directions) may vary. In some such examples, the feedback in the 'rotation to linear' movement upon actuation of the knob may be based on the difference in effort required to actuate the knob based on the level of deflection of the sheath or catheter 90.

In embodiments of the present invention where, in accordance with the invention, an inefficiency generator is provided in an assembly or system comprising a steerable catheter control handle for a steerable sheath, the system allows the user to actuate the system [which in one example may be a single step rotation to linear translation system] with a reasonable amount of force while introducing inefficiency that still allows the user to operate the knob 10 but adds sufficient inefficiency that reverse translation of motion from linear to rotational under tension is not possible allowing self-locking upon actuation, while at the same time still allowing the system (including the handle 200 and sheath 90) to provide a reliable level of feedback so the user is getting a true picture of what is happening at the distal tip.

In accordance with the invention, as noted above, the inefficiency generator 400 as defined by angled threads 304 provides a self-locking mechanism that is built into the system 200 - in other words it has inherently been built into the rotation to linear translation system by providing angled threads 304. Additionally, in some such examples an input that is taken from any part of the system can be used to get a picture of what is happening at the distal tip. As the sheath 90 is actuated it gets harder and harder to actuate which gives the user a feel for what is happening at the distal tip and additionally if the sheath 90 is against something the system will provide feedback to the user. In such examples, by providing a system that provides inefficiency built into the translation mechanism to enable self-locking also allows for an opportunity to get feedback.

As such, embodiments of the present invention provide tactile feedback by increasing the force required to rotate the knob as sheath curvature increases. The inefficiency generator, is defined by the thread angle and may additionally be defined by material on a portion of the slide assembly and on the inner knob. The components of the system are the slider, inner knob, sheath, and sheath pull wire. In some such examples, the inefficiency generator is defined by the internal and external thread geometry of the inner knob and slider assembly, respectively. The thread enables the translations of the knob rotation (controlled by the user) to the slider assembly linear displacement, which increases the pull-wire tension and in tum deflects the sheath or catheter. In other words, the pull-wire tension is the lowest when the sheath or catheter is in the neutral zone and increases as the sheath curvature increases. The inefficiency generator as defined by the thread geometry and material, increases how much feedback is felt as the user rotates the knob, in the presence of increasing pull-wire tension. The inefficiency generator allows the user to feel the change in pull-wire tension as a cue for the sheath neutral zone and/or the amount of deflection of the sheath or catheter.

In such examples according to the invention, a steerable catheter control handle is provided as an assembly that is operable to deflect a sheath. In some examples, the steerable catheter assembly may be provided for different curve sizes. In some embodiments, the steerable catheter assembly may be provided in three separate curve sizes, and in all three curve sizes the following parameters are the same for the thread that is provided in the threadable arrangement defined by the knob and slider assembly:

| Variable | Min | Nom | Max |
|---|---|---|---|
| Pitch (mm) | 12.65 | 12.7 | 12.75 |
| Mean diameter (mm) | 8.66 | 8.71 | 8.76 |
| Thread angle (°) | 161 | 162 | 163 |
| Material | Delrin 100af (Delrin with PTFE fibres) | | |

In some such embodiments, in the steerable catheter control handles for example for small and medium curves have an additional surface coating on the bolt or shaft component of the slider assembly to modify the friction or friction co-efficient.

| (Small and medium curves) | |
|---|---|
| Surface Coating | Nyefilm (PTFE Dry-Film Lubricant) |

In embodiments of the present invention, the inefficiency generator is defined by the threaded inner knob and the threaded slider assembly. In some examples, the inefficiency generator is defined by the thread geometry and may additionally defined by the material. In some such examples, the inefficiency generator may provide one or more advantages of curve retention and/or tactile feedback. In some such examples, the inefficiency generator provides curve retention through the thread geometry.

In examples in accordance with the present invention, the inefficiency generator is defined by the thread angle, i.e. an angled thread as described in the present disclosure. In a specific example, the thread angle is increased, so that it is greater than about zero degrees and less than about 180 degrees.

In some examples, the inefficiency generator is additionally defined by the thread pitch and/or thread pitch diameter. In some such examples, the thread pitch diameter is increased and/or thread pitch is decreased.

In some examples, the inefficiency generator is additionally defined by the thread material. In some such examples, a high friction material is used.

The above inefficiency generators may provide one or more advantages of the inefficiency generators as provided in the present disclosure including preventing curve relaxation.

In accordance with the present invention, an inefficiency generator is provided that increases curve retention capacity and provides tactile feedback, where in some embodiments, the inefficiency generator is defined by both the material and the thread angle of the slider assembly and inner knob. In some embodiments of the present invention, the inefficiency generator is defined by both an increased thread angle and increased material friction coefficient. In some such examples, the material for screw or bold of the slide assembly (defining the threads) comprises Delrin. In other examples, the material of the screw or bolt of the slider assembly comprises PTFE layer on top of the Delrin. In some such examples, the screw is defined by Delrin combined with PTFE, with additional PTFE lubricant on top. In other words, the Delrin is provided with PTFE on surface to provide a desired friction co-efficient, to enable curve retention and/or tactile feedback. In some such examples, lubricant is provided a cavity of an O-ring such as Nyogel 774. In some such embodiments, through thread angle such as an angled thread and friction co-efficient, the inefficiency generator provides the one or more advantages as outlined in the present disclosure. In embodiments of the present invention, the inefficiency generator is provided by angle thread or within the actuation mechanism (such a rotation to linear translation system) enables inefficiency to be introduced into the rotation to linear system while maintaining the exterior dimensions of the handle. This may allow for a rotation to linear translation system that allows for maintaining a sleek exterior profile by providing an ergonomic handle design that allows for ease of use and handling while introducing inefficiency into the system to provide one or more advantages provided in the present disclosure which may include curve retention, tactile feedback and/or minimizing pull wire breakage/failure. In alternate embodiments of the present invention, the inefficiency generator 400 may be provided that provides feedback [such as simulated feedback] of deflection of the catheter 90. In one such example, the slide assembly 30 is moveable within an inner housing 20a of the steerable catheter control handle 200.

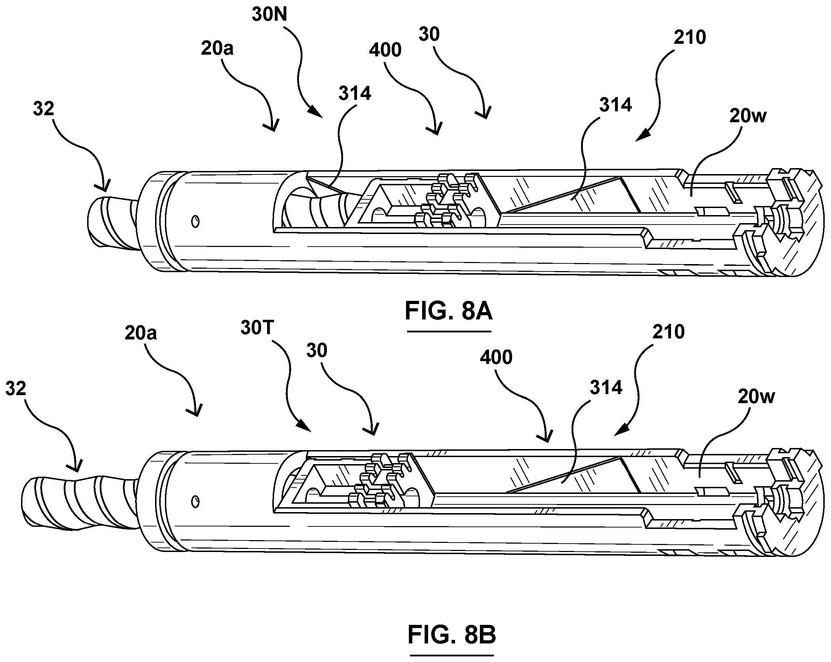

In one such example, a steerable catheter control handle 210 is provided which is similar to embodiments described herein above with respect to Figs. 1A-7D, in terms of operation of the steerable control handle 210 wherein the inefficiency generator 400 comprises a gradient friction device 314, positioned within the handle housing 20 (such as inner housing 20a), with specific reference to Figs. 8A-8D. The gradient friction device 314 interacts with the slide assembly 30 upon movement of the slide assembly 30, wherein the gradient friction device 314 provides increasing friction upon increasing linear translation of the slide assembly 30 within the inner housing 20a upon deflection of the catheter 90. In some such example, which do not form part of the present invention, the slide assembly 30 may comprise a bolt or shaft 32 having square threads 306x having an angle of about zero degrees, as shown in Fig. 7A. Alternatively, in examples according to the invention, the threads are tapered or angled threads 304, as shown in any of the embodiments disclosed herein and in Figs. 7B, 7C and 7D, for instance.

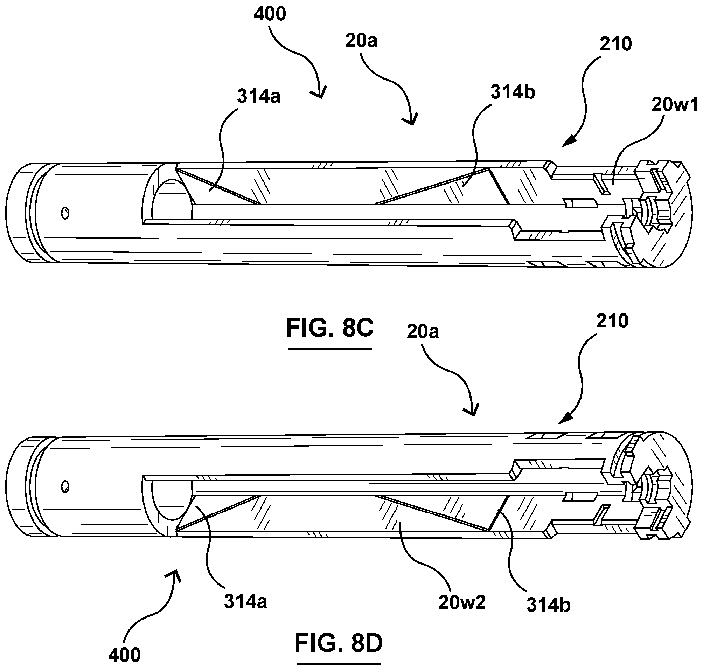

In the specific example, shown in Figs. 8A, 8B, 8C and 8D, the gradient friction device 314 comprises opposing triangular friction pads 314a, 314b positioned along an inner surface of the handle housing, for example along an inner wall 20w, as shown in Figs. 8A and 8B. In some such embodiments, the opposing triangular friction pads 314a, 314b may be positioned along one or more walls (such as opposing walls) 20wl, 20w2 of the inner housing 20a, as shown in Figs. 8C and 8D. In one such example, the opposing triangular friction pads 314a, 314b are positioned along both walls 20wl and 20w2 of the inner housing 20a, as shown.

In operation, when the slide assembly 30 is in the neutral position 30N as shown in Fig. 8A, the slide assembly 30 has minimal contact with the friction device 314 as shown, which is when the sheath or catheter 90 is in its substantially un-deflected position 90a as shown in Figs. 6A-6C. When the knob 10 is actuated to cause linear movement of the slide assembly 30, the slide assembly 30 moves from its neutral position 30N to one of its translated positions 30T, where for example, the slide assembly 30 has traversed the length of the handle housing 20 (such as inner housing 20a), as shown in Fig. 8B. The slide assembly 30 has maximal contact with the friction device 314 as shown, which is when the sheath or catheter 90 is in one of its deflected positions 90b, where for example, the sheath or catheter 90 may be curved to its maximum curvature, as shown in Figs. 6A-6C. The gradient friction device 314, as such provides a maximum amount of friction when the sheath or catheter 90 is in its most deflected position 90b in comparison to when the sheath or dilator is in its substantially un-deflected position 90a.

As such, the inefficiency generator 400, defined by the friction device 314 reduces the efficiency of the rotation to linear translation of the control handle assembly 210 such that it provides the user with simulated feedback regarding the deflection of the sheath or catheter at the distal tip. The inefficiency generator 400, defined by the gradient friction device 314, as such interacts indirectly with the rotation to linear translation system and provides enhanced friction with increasing rotation of the knob 10, to provide the user with the a feel for the amount of deflection or relative deflection of the sheath or catheter 90 [or in other words tactile feedback regarding the deflection of the sheath or catheter 90]. The inefficiency generator 400 comprising the gradient friction device 314 additionally enables curve retention by providing sufficient friction to enable the steerable catheter control handle 210 to maintain the position of the sheath or catheter 90 after it has been deflected. In the particular embodiment shown, the amount of friction increases as the knob 10 is rotated the slide assembly 30 is translated, so that the amount of friction within the rotation to linear system varies with the amount of deflection, allowing the position of the sheath or catheter 90 to be maintained.

In some embodiments of the present invention, the inefficiency generator 400 provides simulated feedback. In one such example, the inefficiency generator 400 comprising the friction device 314 provides simulated feedback. In one such example, the handle 200 may still be functional to provide feedback when the handle 200 is not coupled to the sheath 90. As such, it provides the user with feedback regarding the behavior of the sheath 90 when the sheath 90 is coupled thereto, where the feedback is reflective of the behavior of the sheath at the tip but may not be representative of actual behavior of the sheath 90 or in other words true feedback.

In some such embodiments of the present invention, the components that interact are the slider assembly and the inner housing of the housing that the slide assembly is moveable in. The inefficiency generator for example comprising the gradient friction device, increases interference between the slider and the inner handle based on the position of the slider assembly. Where there is increased interference, there is more friction between the slider assembly and the inner housing, which requires the user the rotate the knob with greater force, providing greater tactile feedback. The interference is less when the slider is in the neutral position [when the knob is not turned and the slide assembly is its nominal or neutral position when the sheath has not been deflected] then when it is in other position. In other words the user would need to rotate the knob with less force in the neutral zone. This change in knob-rotating-force (or the force required to turn the knob) can be used as a tactile cue for neutral zone.

In accordance with the present invention, the inefficiency generator 400 is provided that provides feedback of deflection of the catheter 90. In one such example, the slide assembly 30 is moveable within an inner housing 20a of the steerable catheter control handle 200.

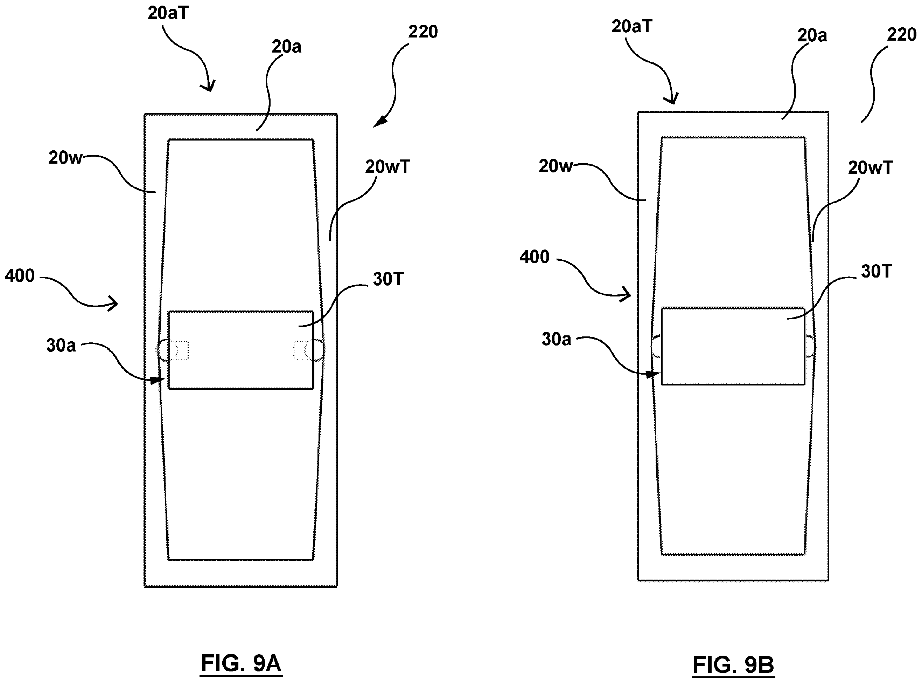

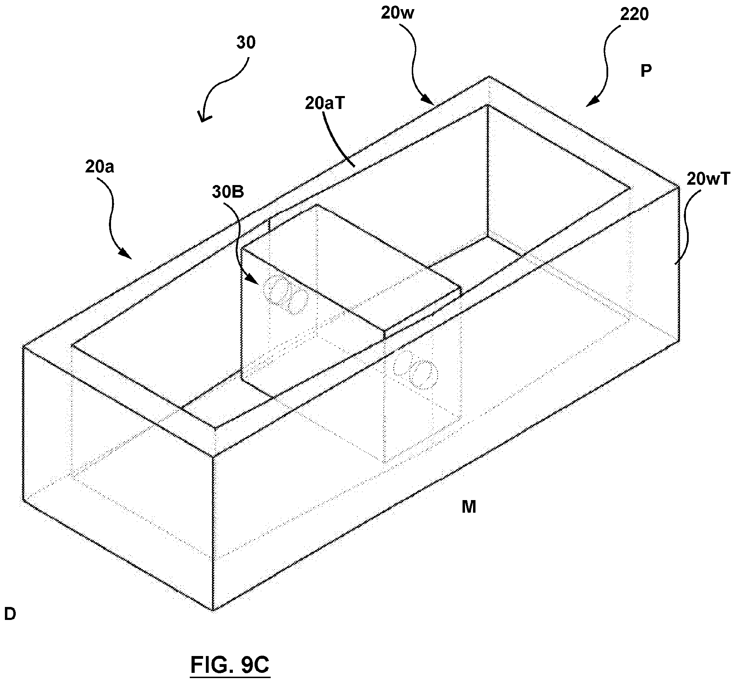

In one embodiment of the present invention as shown in Figs. 9A, 9B and 9C, a steerable catheter control handle 220 is provided that comprises an inefficiency generator 400 that is defined by the inner housing 20a. In, one such example, the inner housing 20a is defined by a tapered inner housing 20aT, wherein the opposing walls 20wl, 20w2 of the inner housing 20a taper towards one another towards the ends of the housing [from a middle [m] of the inner housing 20a towards the proximal and distal ends [P, D] of the inner housing 20a]. The slide assembly 30 experiences increasing friction between the slide assembly 30 and the opposing walls [20wT] of the inner housing 20a, upon increasing linear translation of the slide assembly 30 within the inner housing upon deflection of the sheath or catheter 90.

In some such examples, the steerable catheter control handle 220, the slide assembly 30 is engageable with the walls 20wT of the inner housing via a biasing mechanism 30B. In a specific example, as shown in Figs. 9B and 9C, the biasing mechanism 30B comprises a spring biased mechanism.

As such, the inefficiency generator 400, comprising the tapered inner housing 20aT, reduces the efficiency of the rotation to linear translation of the control handle assembly 220 such that it provides the user with feedback regarding the deflection of the sheath or catheter 90 at the distal tip. The inefficiency generator 400, comprising the tapered inner housing 20aT, as such interacts indirectly with the rotation to linear translation system and provides enhanced friction with increasing rotation of the knob 10, to provide the user with a feel for the amount of deflection or relative deflection of the sheath or catheter 90 [or in other words tactile feedback regarding the deflection of the sheath or catheter 90]. The inefficiency generator 400 comprising the tapered inner housing 20aT additionally enables curve retention by providing sufficient friction to enable the steerable catheter control handle 220 to maintain the position of the sheath or catheter 90 after it has been deflected. In the particular embodiment shown, the amount of frictional engagement between the slide assembly 30 and the walls 20wT of the tapered inner housing 20aT increases as the knob 10 is rotated the slide assembly 30 is translated, so that the amount of friction within the rotation to linear system varies with the amount of deflection, allowing the position of the sheath or catheter 90 to be maintained. In some such embodiments, the tapered inner housing 20aT provides the user with actual feedback regarding the deflection of the catheter at the distal tip.

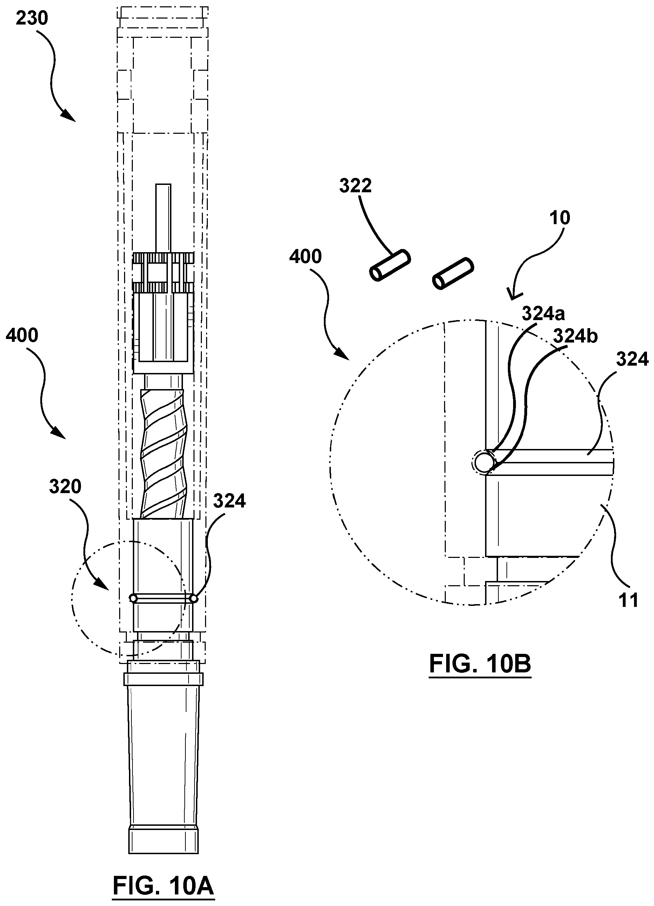

In alternate embodiments of the present invention, a steerable catheter control handle 230 is provided that may comprise one or more means for generating inefficiency into an actuation system for deflecting the sheath or catheter 90. In one such example as shown in Figs. 10A,10B, a coupling means for coupling the rotatable knob 10 to the inner housing 20a may comprise an inefficiency generator or a means for generating or introducing inefficiency 400 comprising a tapered pin and groove joint 320 that comprises pins 322 that may be passed through a tapered groove 324 formed within a wall or surface 11 of the knob 10 to retain the coupling between the knob 10 and a portion of the handle housing 20 (such as inner housing 20a). In the specific example shown, a tapered groove 324 is provided with tapered or angled walls 324a, 324b that may enhance the amount of friction between the knob and a portion of the handle housing which may increase the normal force present between the pins 322 and the angled groove 326. This may help keep the position or curvature of the catheter after it has been deflected. This mechanism may enhance friction by interacting indirectly with the rotation to linear translation system and the mechanism and may indirectly impact the operation of rotation to linear translation system by making it more inefficient in deflecting the sheath or catheter 90. As such, the embodiment as shown in Figs. 10A-10B introduces inefficiency into the rotation to linear translation system for deflecting the catheter and may provide one or more advantages of a means for introducing inefficiency as provided in other embodiments described herein in the present disclosure. The tapered pin and groove joint 320 may help provide feedback to the user regarding the deflection of the catheter, and may provide the user with some tactile feedback. Additionally, the inefficiency generator 400 as provided by the tapered pin and groove joint 320 may additionally introduce friction between the knob 10 and inner housing 20a to help maintain the position of a sheath or catheter 90 after it has been deflected.

In some such embodiments of the present invention, the inefficiency generator is defined by a modified inner knob-pin contract angle. In some such examples, the inner knob-pins contact angle is modified such that the inner knob groove (that receives the pins) has angled walls as shown. In some such examples, the steerable catheter handle comprises an inner knob, pins, and a slider assembly, and an inner handle housing. In one such example, there are two through holes in the inner handle (or inner housing). The through holes are positioned so that when the pins are inserted, the pins sit in the inner knob groove and lock the inner knob in position, as shown. As shown, in one example a tapered groove is provided that increases the contact angle (i.e. a "V" groove may provide increased contact angle than a square groove). As the contact angle increases the normal force on the pins also increases, which increases the friction at the pin joint.

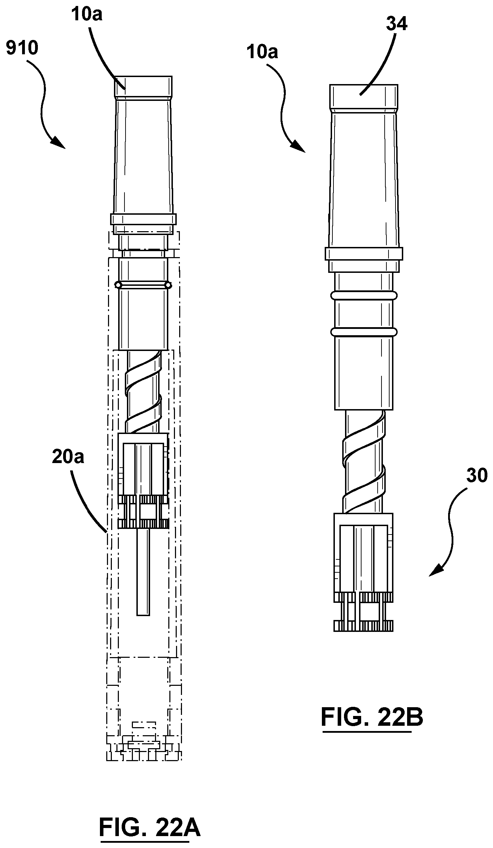

In some additional embodiments of the present invention as shown in Figs. 22A and 22B, the inefficiency generator is defined by an O-ring 900 which may be usable with one or more inefficiency generators of the present invention. In some such embodiments, a steerable catheter control handle 910 is provided that comprises the components comprising: an O-ring 900, inner knob 10a, inner handle (or inner housing) 20a, and slider assembly 30. In some such examples, an O-ring 900 is provided at the interface between the inner knob 10a and the inner handle (or inner housing) 20a. The squeezing of the O-ring 900 between the inner knob 10a and the inner handle (or inner housing) 20a creates friction which may allow for curve retention. In one such example, the O-ring 900 is positioned on the inner knob 10a. The inner knob 10a is then inserted into the inner handle (inner housing) 20a. As a result, the O-ring 900 is squeezed/compressed between the inner knob 10a and inner housing 20a. The compression of the O-ring 900 creates a constant normal force to the inner surface of the inner handle (or inner housing) 20a, which creates a constant friction force against the rotation of the knob 10. The magnitude of the friction force is defined by the size of the O-ring 900 (diameter and thickness), material of the O-ring 900, and material of the inner handle (or inner housing) 20a.

Embodiments of the steerable catheter control handle of the present invention comprises at least one or more means for generating inefficiency into system for translating the actuation of an actuator into deflection of the sheath or catheter 90, as described in the present disclosure, and may provide one or more advantages thereof of the means for generating inefficiency as described in the present disclosure.

In embodiments in accordance with the invention, a steerable catheter control handle [200, 210, 220] and the operation thereof is explained, with particular reference to Fig. 2A, the steerable sheath or catheter 90 comprises at least one control or pull wire 40, 42, where a distal end of the control wire 40, 42 is coupled the sheath or catheter 90, and a proximal end of the at least one control or pull wire 40, 42 is coupled to the slide assembly 30. In one particular example, the control handle 200 comprises a bi-directional control handle 200A, where the least one control wire 40, 42 comprises a least two control wires 40, 42 a distal end of each of the at least two control wires being coupled the sheath or catheter 90, and a proximal end of each of the at least two control wires being coupled to the slide assembly 30. In this particular example, one of the control wires such as control wire 40, also referred to as the non-pulley control wire 40 is routed proximally from the sheath or catheter 90, such as a distal end of the sheath or catheter 90, for example, to which the non-pulley control wire 40 is coupled at its distal end. The proximal end of the non-pulley control wire 40 is then routed through the handle (such as handle housing 20) and through the slide assembly 30 and is then coupled directly to the slide assembly 30. For example, the proximal end of the non-pulley control wire is crimped at the proximal face of a portion of slide assembly 30, such as a portion of the carriage 34, such as a wall of the carriage 34.

In this particular example, additionally shown in Figs. 1A-6C, the other control wire such as control wire 42, also referred to as the pulley control wire 42 is also routed proximally from the sheath or catheter 90, such as a distal end of the sheath or catheter 90, for example, to which the pulley control wire 42 is coupled at its distal end. The proximal end of the pulley control wire 42 is then also routed through the handle housing 20 and through the slide assembly 30 and is then coupled indirectly to the slide assembly 30. For example, the proximal end of the pulley control wire 42 extends through the slide assembly 30 and is then routed around a direction reversing element 50' such as pulley 52 and through a portion of the slide assembly 30 (such as a portion of the carriage 34, such as a wall of the carriage 34) and is crimped at the distal face thereof.

In operation the rotation of the knob 10 in a first rotational direction causes a linear movement of the slide assembly 30 in a first linear direction, to enable deflection of the sheath or catheter 90 via actuation of one of the at least two control wires 40, 42 [for example, such as a non-pulley wire 40] to impart desired curvature to the sheath or catheter 90 in a first direction (For example, 90A as shown in Figs. 6A-6C). The rotation of the knob 10 in a second rotational direction causes a linear movement of the slide assembly 30 in a second linear direction, to enable deflection of the sheath or catheter 90 via actuation of the other of the at least two control wires 40, 42 (such as the pulley control wire 42) to impart desired curvature to the sheath or catheter 90 in a second direction (for example 90B, as shown in Figs. 6A-6C).

The inefficiency generator 400 provides feedback to the user regarding the behavior or deflection of the sheath or catheter 90 at the distal end for example, and enables curve retention allowing the deflection or curvature of the catheter to be maintained at the desired curvature in both of its deflection directions. In some such embodiments of the present invention, the inefficiency generator 400 results in increased force required to curve the sheath or catheter 90 in each of the first and second deflection directions.

In a particular example of a bi-directional steerable catheter control handle 200 as shown in Fig. 2A, wherein the inefficiency generator 400 is provided as an angled thread 304, the angled thread 304 provides enhanced tactile feedback to the user that provides true feedback regarding the behavior or deflection of the sheath or catheter 90, for example at the distal end thereof, in each of its deflection directions, and enables curve retention allowing the deflection or curvature of the catheter to be maintained at the desired curvature in each of the deflection directions of the sheath or catheter 90.

As such, in some embodiments of the present invention the steerable catheter control handle 200 comprises a bi-directional control handle 200, 210, 220, where the one or more control or pull wires [40, 42] comprise two control or pull wires 40, 42, wherein the actuation of the actuator in first and second directions 90A and 90B, causes the means for deflecting the control or pull wires 40, 42, such as the rotation to linear system [for example defined by a knob 10 and slide assembly 30] to enable deflection of the sheath or catheter 90 via actuation of each of the respective pull wires to impart desired curvature to the sheath or catheter 90 in respective first and second deflection directions [90A, 90B], where the inefficiency generator 400 results in increased force required to curve the sheath or catheter 90 in each of the first and second deflection directions [90A, 90B] .

In some embodiments a steerable catheter control handle [200, 210, 220] is provided and the operation thereof is explained with reference to Fig. 2A. In some such examples, the steerable catheter control handle [200, 210, 220] comprises a uni-directional control handle where the steerable sheath or catheter 90 comprises one of the control or pull wires 40, 42, where a distal end of the control wire 40 or 42 is coupled the sheath or catheter 90, and a proximal end of the control or pull wire 40 or 42 is coupled to the slide assembly 30. In one particular example, the control handle 200 comprises a uni-directional control handle, where the distal end of the control wire 40, 42 is coupled to the sheath or catheter 90, and a proximal end of the control wire 40 or 42 is coupled to the slide assembly 30.

In one particular example, additionally shown in Figs. 1A-6C, only one control wire is provided where the control wire comprises control wire 40, also referred to as the non-pulley control wire 40 that is routed proximally from the sheath or catheter 90, such as a distal end of the sheath or catheter 90, for example, to which the non-pulley control wire 40 is coupled at its distal end. The proximal end of the non-pulley control wire 40 is then routed through the handle housing 20 (such as inner housing 20a) and through the slide assembly 30 and is then coupled directly to the slide assembly 30. For example, the proximal end of the non-pulley control wire is crimped at the proximal face of a portion of slide assembly 30, such as a portion of the carriage 34, such as a wall of the carriage 34.

In another particular example, additionally shown in Figs. 1A-6C, only one control wire is provided, where the control wire comprises control wire 42, also referred to as the pulley control wire 42 that is routed proximally from the sheath or catheter 90, such as a distal end of the sheath or catheter 90, for example, to which the pulley control wire 42 is coupled at its distal end. The proximal end of the pulley control wire 42 is then also routed through the handle 20 and through the slide assembly 30 and is then coupled indirectly to the slide assembly 30. For example, the proximal end of the pulley control wire 42 extends through the slide assembly 30 and is then routed around a direction reversing element 50' such as pulley 52 and through a portion of the slide assembly 30 (such as a portion of the carriage 34, such as a wall of the carriage 34) and is crimped at the distal face thereof.

In operation the rotation of the knob 10 in a single rotational direction causes a linear movement of the slide assembly 30 in a single linear direction (90A or 90B, as shown in Figs. 6A-6C), to enable deflection of the sheath or catheter 90 via actuation of the single control or pull wire [either the non-pulley control wire 40, or the pulley control wire 42], to impart desired curvature to the sheath or catheter 90 in the single deflection direction.

In some such embodiments, the inefficiency generator 400 provides feedback to the user regarding the behavior or deflection of the sheath or catheter 90 at the distal end for example, and enables curve retention allowing the deflection or curvature of the sheath or catheter 90 to be maintained at the desired curvature in the single deflection direction [90A or 90B]. In a particular example of a uni-directional, steerable catheter control handle 200, with one of the control wires 40, 42 shown in Fig. 2A, the inefficiency generator 400 is provided as an angled thread 304, the angled thread 304 provides enhanced tactile feedback to the user that provides true feedback regarding the behavior or deflection of the sheath or catheter 90 in the single deflection direction at the distal end, for example, and enables curve retention allowing the deflection or curvature of the catheter to be maintained at the desired curvature in the single deflection direction of the sheath or catheter 90. In some such embodiments, the means to introduce inefficiency or [inefficiency generator 400] results in increased force required to curve the sheath in the single deflection direction. In some such embodiments of the present invention, the inefficiency generator 400 provides continuous feedback along the entire range of deflection or curvature of the sheath or catheter.

In some such embodiments, the steerable catheter control handle 200 comprises a unidirectional control handle, wherein the one or more pull wires comprises a single control or pull wire [40, 42], wherein actuation of the actuator (such as knob 10) causes means for deflecting the pull wires (such as the rotation to linear system defined by the knob 10 and the slide assembly 30) to enable deflection of the sheath or catheter 90 via actuation of the pull wire to impart desired curvature to the catheter in a single deflection direction [90A or 90B], wherein inefficiency generator 400 [such as angled threads results in increased force required to curve the sheath or catheter 90 in the single deflection direction [90A or 90B].

In some embodiment of the present invention, as shown in Figs. 1A-9C, the rotation to linear translation system comprises a single step rotation to linear translation system. In some such embodiments a rotatable knob 10 is provided that converts the rotational actuation of the knob 10 into linear movement of the slide assembly 30. In some such embodiments, a single slide assembly 30 as shown is provided where the rotation of the knob 10 is converted into linear movement of a single slide assembly 30.

In other embodiments of the present invention, the rotation to linear translation system comprises a two-step rotation to linear translation system. In some such embodiments a rotatable knob 10 is provided that converts the rotational actuation of the knob 10 into linear movement of two or more slide assemblies such as slide assembly via a threadable arrangement. In some such embodiments, two slides are provided as shown, comprising a first slide with a left thread and second slide with a right thread, where the rotation of the knob 10 is converted into linear movement of both of the first and second slides in opposing linear directions. In some such embodiments, a means to introduce inefficiency or an inefficiency generator 400 is provided, of the type provided in any of the embodiments described herein above. In one specific example, one or more of the first and second slides are provided with left and right threads that are angled left and right threads that introduce inefficiency in the rotation to linear translation in the manner previously described herein above for a single step rotation to linear translation system and provides the one or more advantages thereof.

In some embodiments of the steerable catheter control handle such as [200, 210, 220] of the present invention, for example with reference to Figs. 2A and 6A, the one or more the control wires [non-pulley control wire 40 and/or the pulley control wire 42] is coupled to a distal end [90d] of the sheath or catheter 90 to enable deflection of the distal end 90d of the catheter.

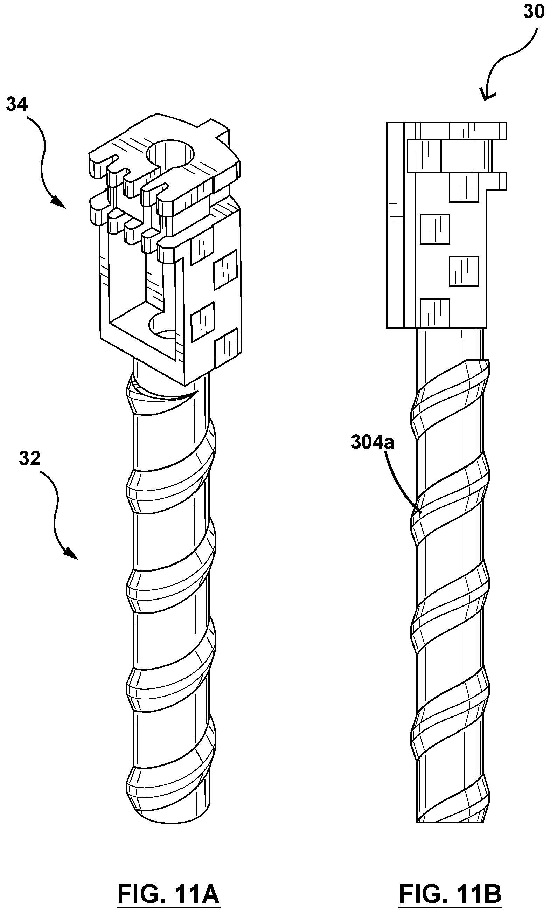

In alternate embodiments of the present invention, as shown in Figs. 11A-19D, an inefficiency generator 400 is provided that comprises a threadable engagement 302 comprising angled threads 304 comprising an external angled thread 304a on the shaft or bolt 32 of the slide assembly 30 that is engageable with a corresponding internal angled thread 304b along the inner surface of the knob 10. These embodiments of an inefficiency generator 400 provide one or more advantages the means for generating inefficiency 400 as described in the present disclosure.

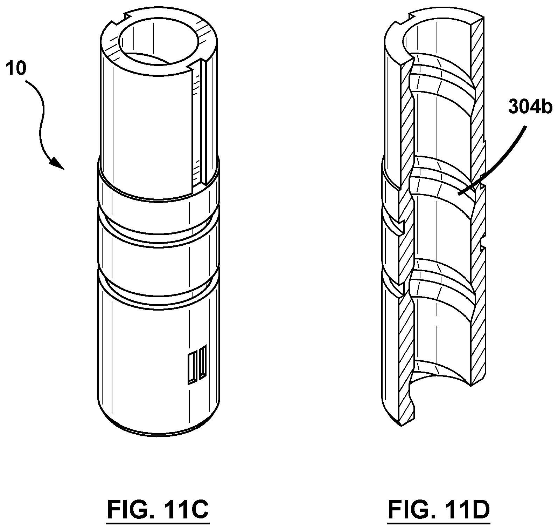

In one example, the inner knob 10a and the slide assembly 30 have a tapered angled thread 304 (comprising an external angled thread 304a and an internal angled thread 304b) respectively, that has an angle of about 140 degrees, as shown in Figs. 11A, 11B, 11C and 11D.

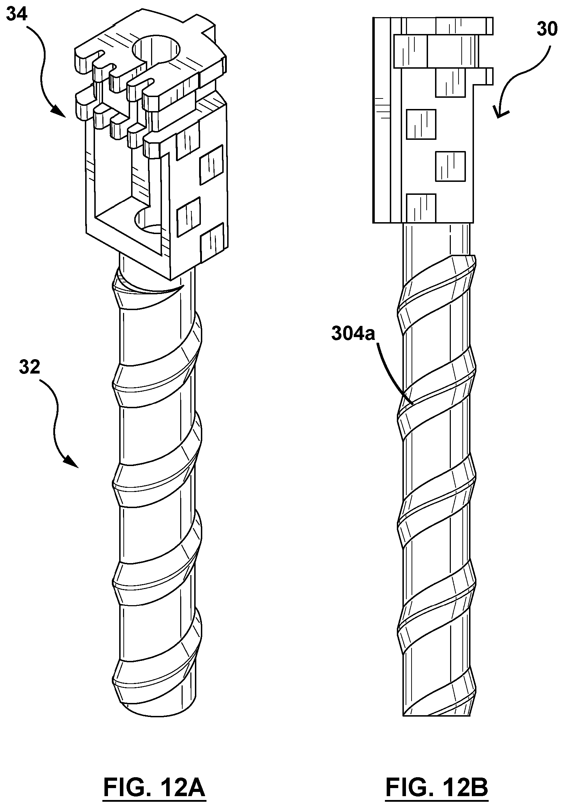

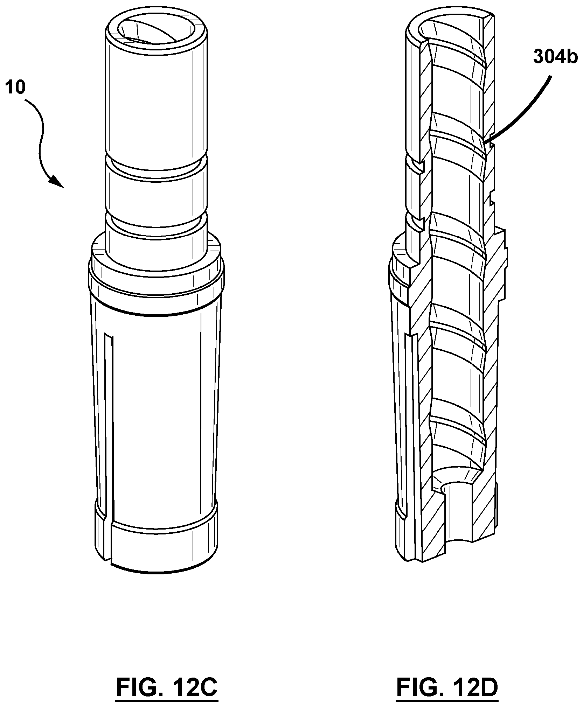

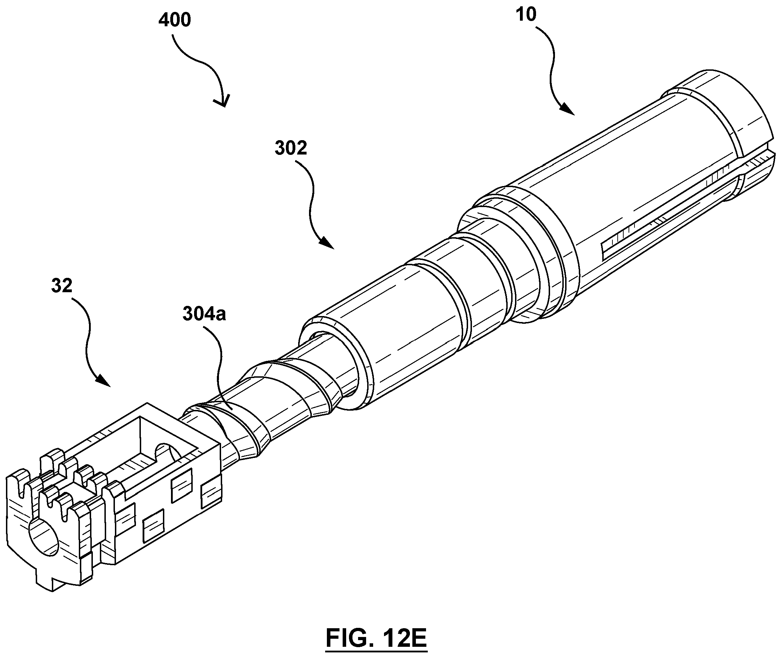

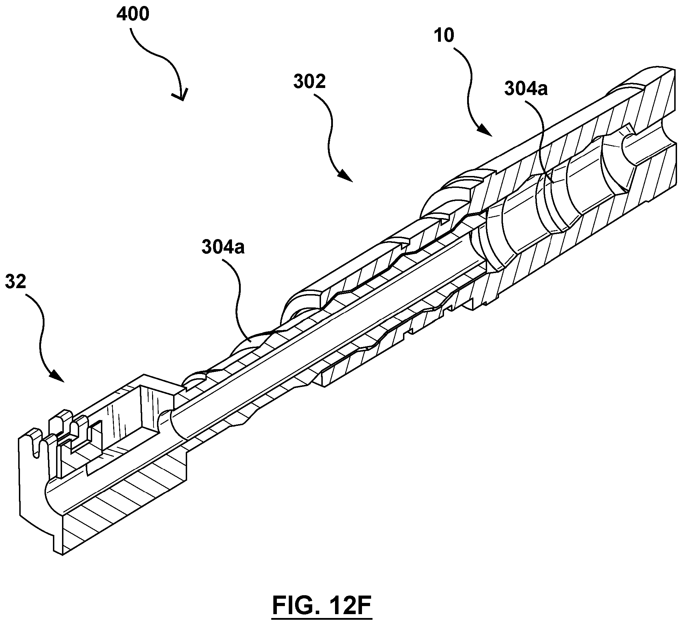

In one example, the inner knob 10a and the slide assembly 30 have a tapered angled thread 304 (comprising an external angled thread 304a and an internal angled thread 304b) respectively, that has an angle of about 148 degrees, as shown in Figs. 12A, 12B, 12C and 12D. In one such example, the inner knob 10a is shown in engagement with the bolt 32 of the slide assembly 30, defining the threadable engagement 302 and the inefficiency generator 400, as shown in Figs. 12E and 12F.

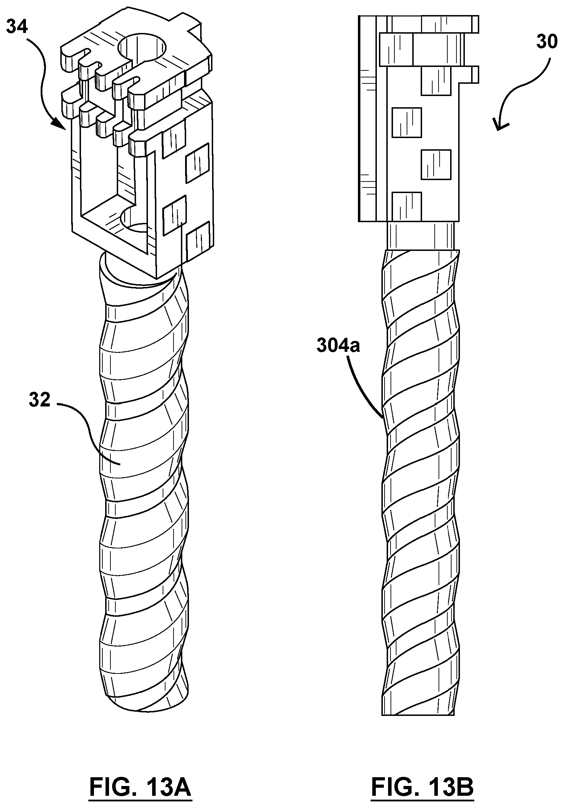

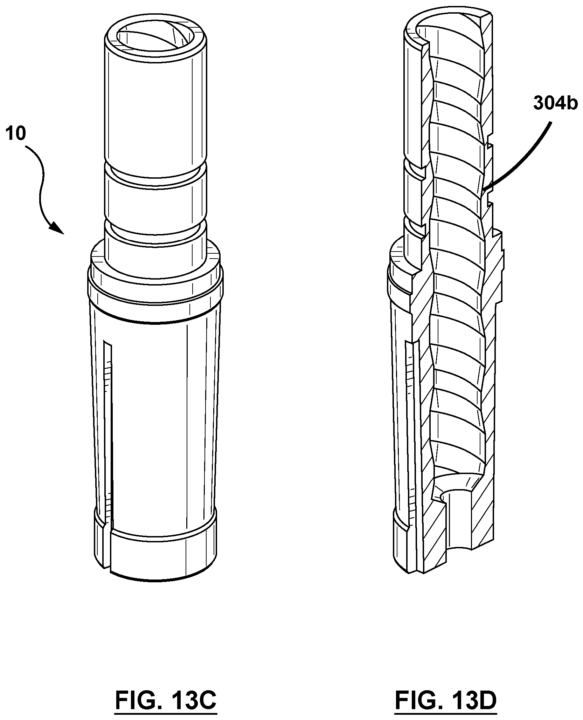

In one example, the inner knob 10a and the slide assembly 30 have a tapered angled thread 304 (comprising an external angled thread 304a and an internal angled thread 304b) respectively, that has an angle of about 156 degrees, as shown in Figs. 13A, 13B, 13C and 13D.

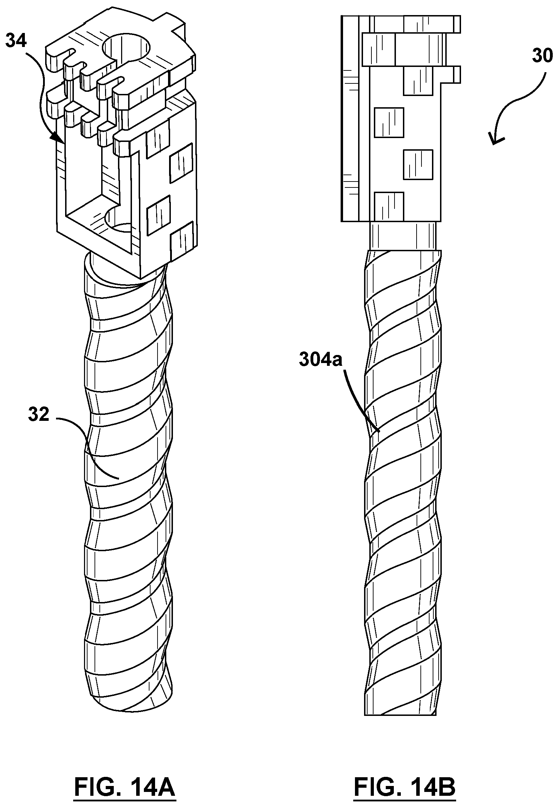

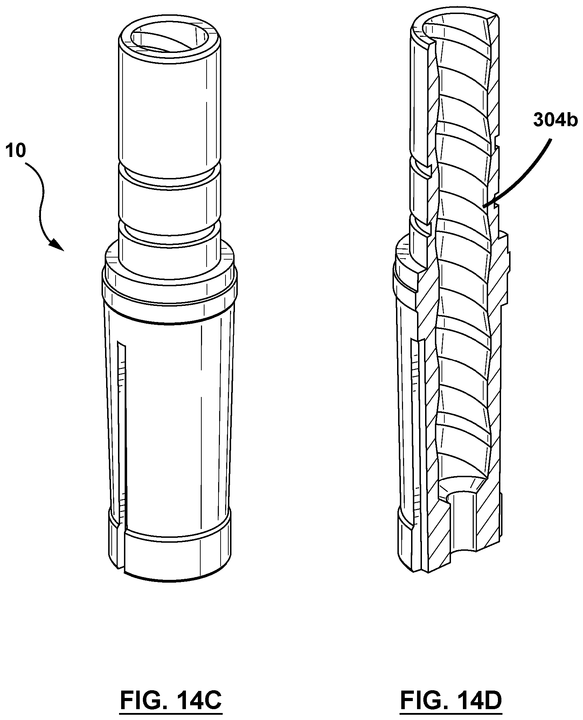

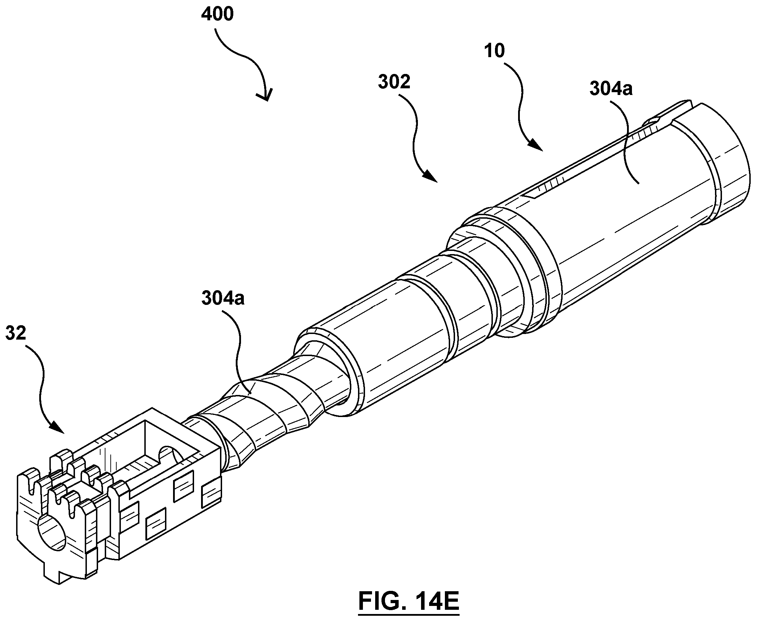

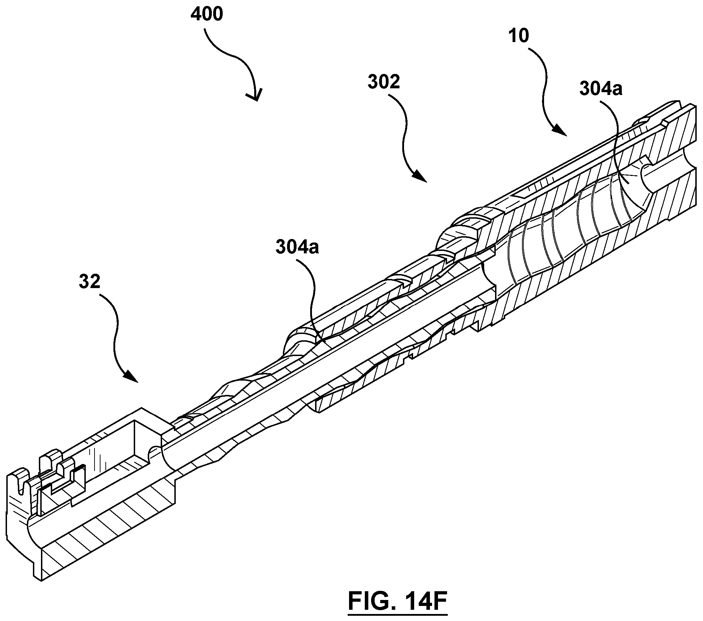

In one example, the inner knob 10a and the slide assembly 30 have a tapered angled thread 304 (comprising an external angled thread 304a and an internal angled thread 304b) respectively, that has an angle of about 160 degrees, as shown in Figs. 14A, 14B, 14C and 14D. In one such example, the inner knob 10a is shown in engagement with the bolt 32 of the slide assembly 30, defining the threadable engagement 302 and the inefficiency generator 400, as shown in Figs. 14E and 14F.

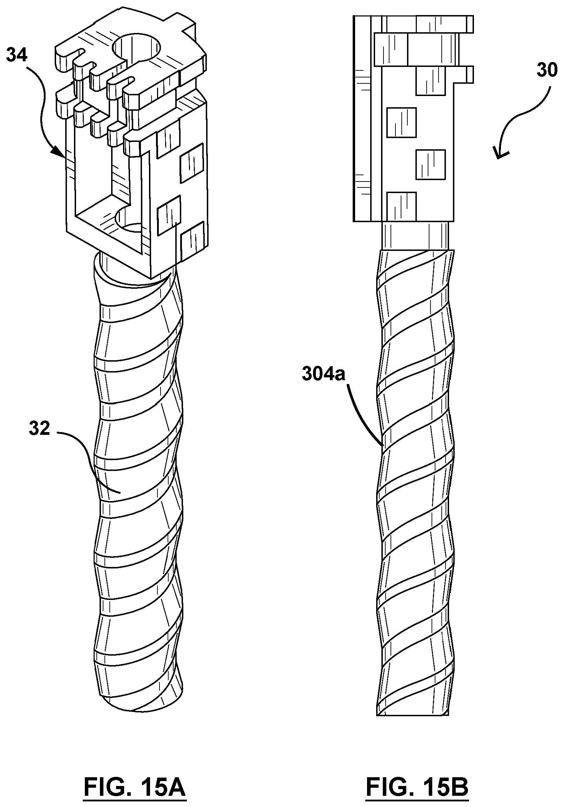

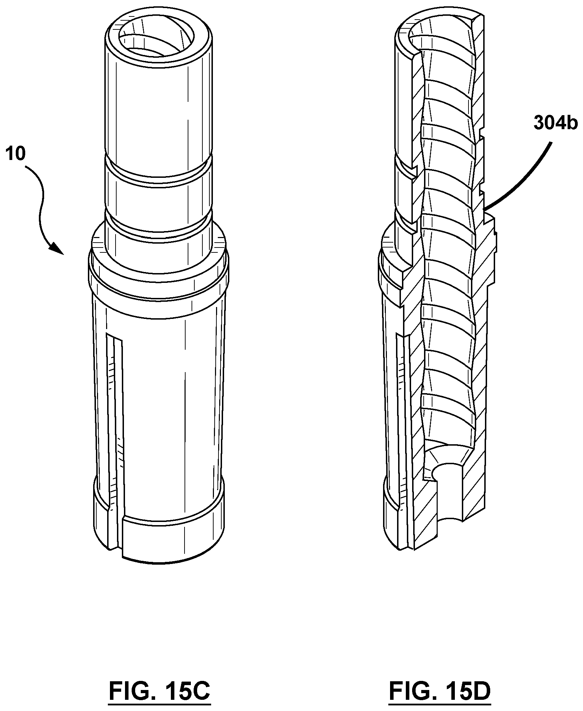

In one example, the inner knob 10a and the slide assembly 30 have a tapered angled thread 304 (comprising an external angled thread 304a and an internal angled thread 304b) respectively, that has an angle of about 162 degrees, as shown in Figs. 15A, 15B, 15C and 15D.

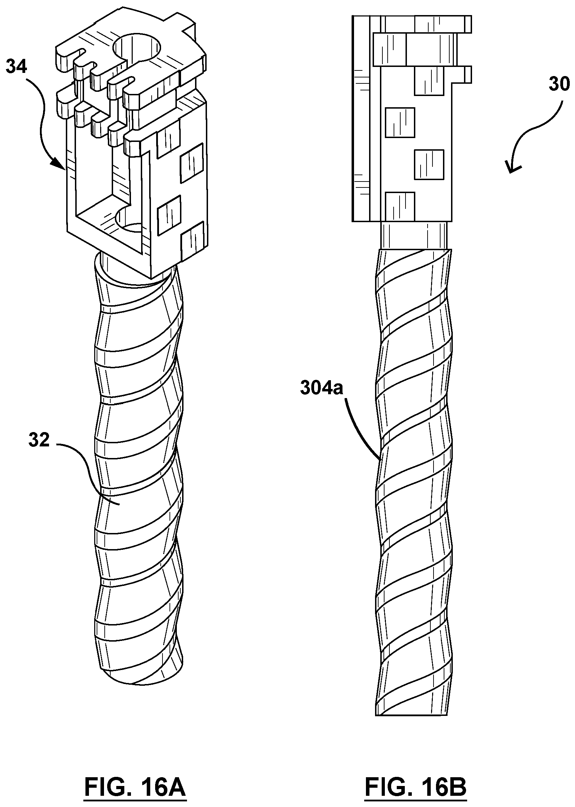

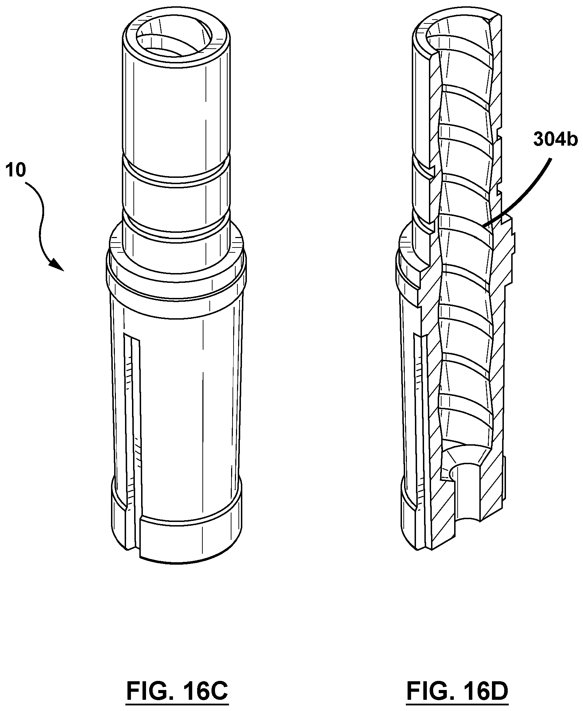

In one example, the inner knob 10a and the slide assembly 30 have a tapered angled thread 304 (comprising an external angled thread 304a and an internal angled thread 304b) respectively, that has an angle of about 166 degrees, as shown in Figs. 16A, 16B,16C and 16D.

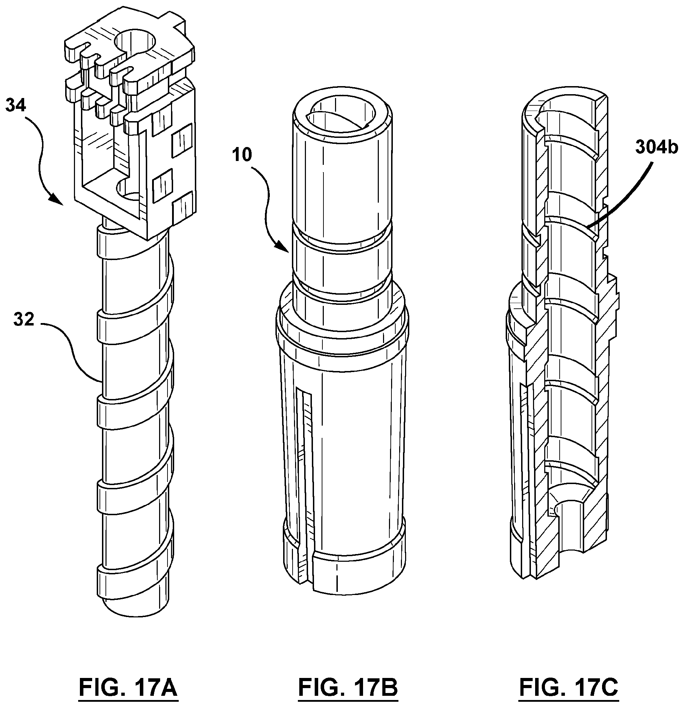

In one example, the inner knob 10a and the slide assembly 30 have a tapered angled thread 304 (comprising an external angled thread 304a and an internal angled thread 304b) respectively, that has an angle of about 30 degrees, as shown in Figs. 17A, 17B, and 17C.

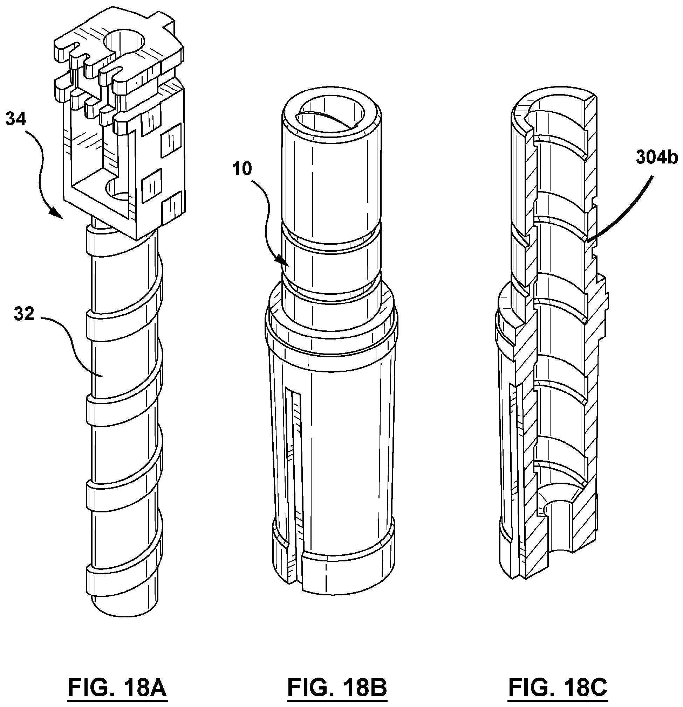

In one example, the inner knob 10a and the slide assembly 30 have a tapered angled thread 304 (comprising an external angled thread 304a and an internal angled thread 304b) respectively, that has an angle of about 60 degrees, as shown in Figs. 18A, 18B and 18C.

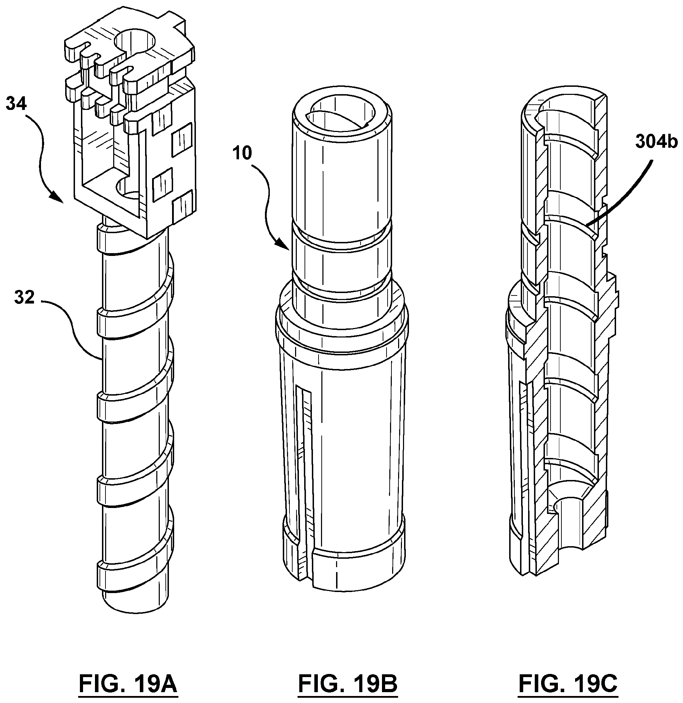

In one example, the inner knob 10a and the slide assembly 30 have a tapered angled thread 304 (comprising an external angled thread 304a and an internal angled thread 304b) respectively, that has an angle of about 70 degrees, as shown in Figs. 19A, 19B and 19C.

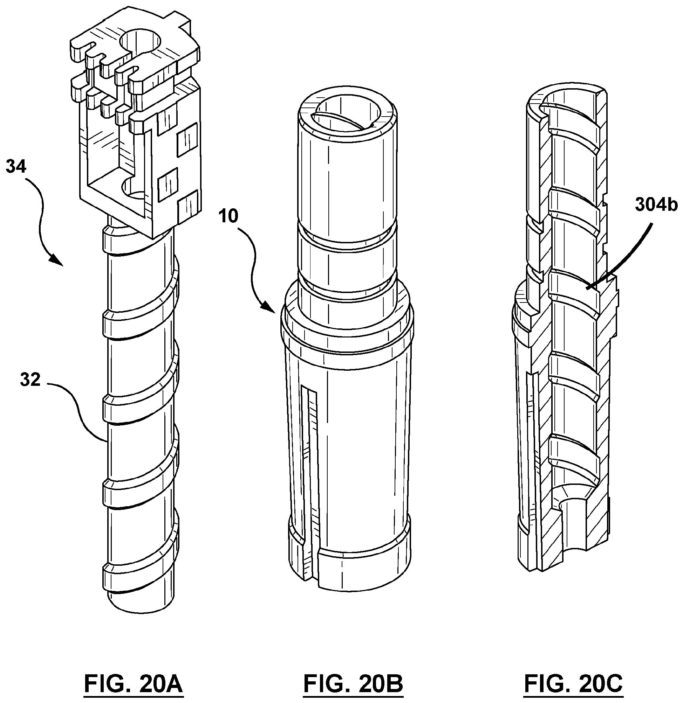

In one example, the inner knob 10a and the slide assembly 30 have a tapered angled thread 304 (comprising an external angled thread 304a and an internal angled thread 304b) respectively, as shown in Figs. 20A, 20B and 20C.

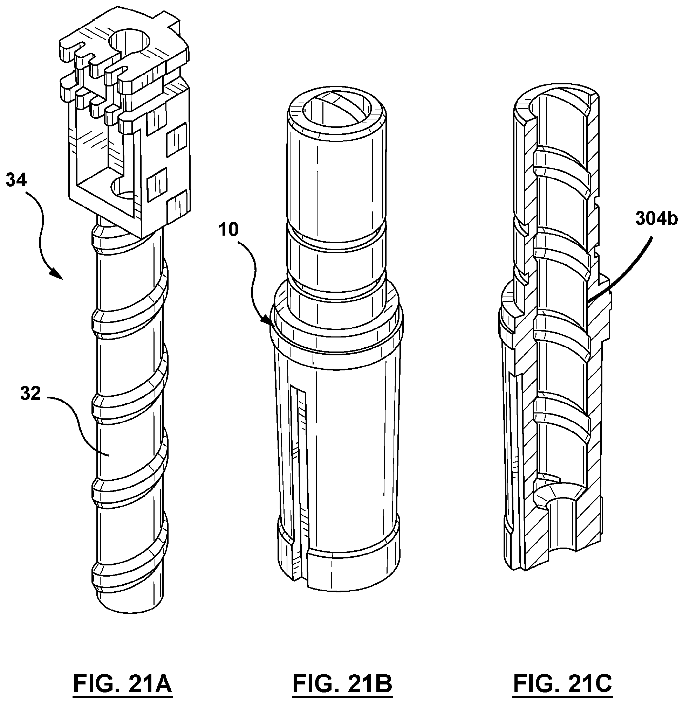

In one example, the inner knob 10a and the slide assembly 30 have a tapered angled thread 304 (comprising an external angled thread 304a and an internal angled thread 304b) respectively, that has an angle of about 120 degrees, as shown in Figs. 21A, 21B and 21C.

In some examples which do not form part of the present invention, a thread angle of zero degrees is provided for the threadable arrangement defined by the inner knob and the slider assembly, and where some other means of introducing inefficiency is provided in the rotation to linear translation system of a steerable catheter control handle.

In some examples which do not form part of the present invention, a rotation to linear translation system is provided such as a single step rotation to linear system, that utilizes a square thread (having an angle of about zero degrees), and a compensation mechanism for retaining curvature of the catheter such an inefficiency generator may be provided in the form of a larger handle, as providing a rotatable knob with a larger diameter may enhance the ability of the steerable control mechanism to maintain the position of the catheter after it has been deflected. This may additionally enhance the amount of tactile feedback that is provided.

In embodiments in accordance with the invention, a rotation to linear system is provided with a threadable arrangement, where the thread has an angle that is greater than zero degree and less than about 180 degrees.

In some such examples in accordance with the invention, an angled thread is defined as a thread that has an angle that is greater than about zero degrees and less than about 180 degrees.

In alternate embodiments of the present invention, a steerable catheter control handle is desired that has a sleek profile to enhance usability and make it easier for the user to handle. As such a steerable catheter control handle with an optimized design for a minimized profile is provided where an alternate compensation mechanism such as an inefficiency generator is provided without increasing the outer diameter of the handle. In one such example, the inefficiency generator is built into the rotation to linear system, the inefficiency generator being defined by angled threads in accordance with the invention.

In some embodiments of the present invention, a steerable catheter control handle is provided that comprise a compensation system defining an inefficiency generator that may provide curve retention and tactile feedback. In some such examples, the tactile feedback provides a differential in feel between maximum deflection of the catheter and the minimum deflection of the catheter. In some examples, the inefficiency generator may enhance friction/force to deflect at both maximum and minimum deflection of the catheter by differential amount. As such, some embodiments of the systems of the present invention may make it easy for the user to ascertain how much the catheter has been deflected and as such provide an improved mechanism that provides an enhanced difference in tactile feel between minimum deflection of the catheter and maximum deflection of the catheter.

In some embodiments of the present invention, a two-step rotation to linear system may be provided that comprises an inefficiency generator, where a rotational knob moves two slides simultaneously in opposite linear directions. This may inherently introduce inefficiency into the two step rotation to linear translation system. As the knob is rotated to move one slide to pull one of the wires, the other slide moves in the opposite direction to push the other wire, which results in deflection of the catheter in a first direction, and there is movement of the second slide that pushes the other wire. Some such systems of the present invention may be provided with an inefficiency generator of the type described herein above. In some such example the inefficiency generator is defined by angled threads. Additionally, the inefficiency generator may provide sufficient loss of energy for retaining deflection and may additionally provide sufficient tactile feedback, for example through use of angled threads.

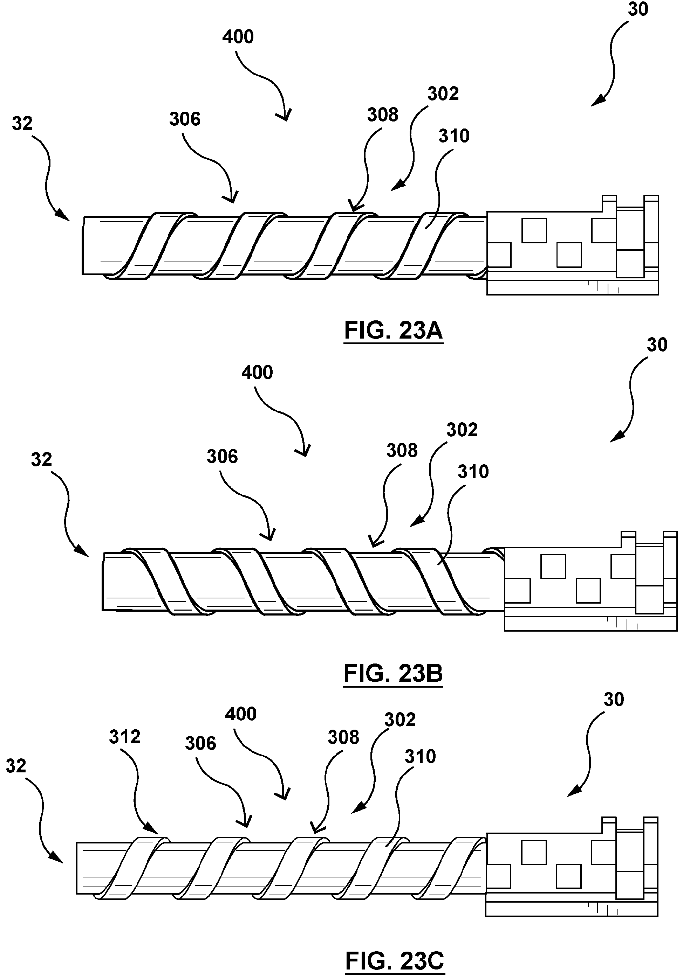

With reference now to Figs. 23A and 23B, in some embodiments of the present invention, an inefficiency generator is incorporated into one or more medical devices used to steer, guide or otherwise manipulate other devices. In particular, an inefficiency generator is provided that is included in steerable devices such as steerable sheaths or catheters, as described previously herein above.

As outlined previously herein above, medical devices used for guidance, such as steerable sheaths, are usable to assist in navigating and positioning devices within a patient's body. In such devices, a user may manipulate a controlling component, such as a handle of a steerable sheath, in order to articulate a portion of the device. On occasion, the user may manipulate the controlling component to cause the articulating portion of the device to achieve a particular curve or orientation. In some such examples, the user may desire the curve or orientation to be maintained by the device. Additionally, the user may desire feedback to determine the actuation of the articulating portion of the device.

As outlined previously herein above, if under certain circumstances, the medical device is unable to maintain that curve or orientation of the articulating portion, it may lead to undesirable movement of the articulating portion. Several further embodiments, as conceived and reduced to practice by the as conceived of, and reduced to practice several embodiments of a are disclosed herein, of a feature or component for use in such medical devices to assist in maintaining a desired position of the articulating component or portion (such as a sheath or catheter 90) by retaining a position or state of the controlling component (i.e. an actuator including a control knob 10 of an actuation mechanism for deflecting the sheath or catheter 90). The invention additionally provides the user with feedback regarding the degree of curvature or articulation of the articulating portion or component (such as a sheath or catheter 90).

In accordance with the invention, the feature or component comprises a means for reducing inefficiency or a means of introducing inefficiency that has been built into a system comprising a sheath or catheter and a translation mechanism for deflection the same.

The feature or component for introducing inefficiency enables a position of the catheter to be substantially maintained after it has been deflected. In some such examples, the system for introducing inefficiency may for example introduce sufficient friction into the system to maintain the position of the sheath or catheter after it has been deflected. As in previous embodiments, embodiments of the present invention provide a system that enables self-locking after a catheter has been deflected. The feature or component for introducing inefficiency additionally provides feedback, for example, of the behavior of the sheath or catheter, such as a degree of curvature or deflection of the sheath or catheter, such as at a distal tip thereof.

Embodiments of the present invention, with reference to Figs. 23A and 23B, comprise an inefficiency generator 400 that is defined by the threadable engagement 302 between the slide assembly 30 and the knob 10 that forms the rotation to linear translation system 300. The means for introducing inefficiency is built into the system comprising a sheath or catheter 90 and the translation mechanism (as defined by the rotation to linear translation mechanism 300) for deflection the same. In some examples, the threadable engagement 302 functions to increase static friction between rotatable and longitudinally translatable components of a steerable sheath handle 200.

Figs. 23A and 23B illustrate a threaded slider mechanism or slide assembly 30 for use in an embodiment of the present invention comprising a steerable catheter control handle (such as control handle 200) for a sheath or catheter 90, as shown previously, comprising a rotation to linear translation system 300. Similar to embodiments described hereinabove, the rotation to linear translation system 300 comprises a rotatable knob 10 and a threaded slider mechanism or slide assembly 30, where the rotational movement of the knob 10 is converted into linear movement of the slide assembly 30 via a threadable engagement 302. Similar to embodiments discussed hereinabove, the threadable engagement 302 is defined by external threads 302b formed on a shaft or bolt 32 of the slide assembly 30 and corresponding internal threads 302a on the knob 10 that are engageable therewith.

Similar to examples noted previously, the control handle 200 includes a means for introducing inefficiency or inefficiency generator 400 into the rotation to linear translation system 300, where the means to introduce inefficiency or inefficiency generator 400 is integral to or inherent in the rotation to linear translation system 300.

With reference again to Figs. 23A and 23B, the rotatable knob 10 is coupled to the slide assembly 30 via a threadable engagement 302, where the threadable engagement 302 comprises a threaded slider mechanism or slide assembly 30 having a modified thread 306 that is engageable with a knob 10 having corresponding threads, defining the inefficiency generator 400. In some such examples, the modified thread 306 comprises a right handed thread as noted previously, as shown in Fig. 23A. In other examples, as noted above, the modified thread 306 comprises a reverse thread or reverse-threaded portion 308 on the slide assembly 30, as shown in Fig. 23C. More specifically, in the illustrated embodiment, the reverse-threaded portion 308 includes a left handed thread 310.

In one such example, embodiments of the present invention provide an inefficiency generator 400 that comprises a threadable engagement 302 that comprises a threaded slider mechanism or slide assembly 30, for example such as a reverse threaded, slider mechanism or slide assembly 30 comprising reverse threads 308 for increasing static friction between rotatable and longitudinally translatable components of a steerable sheath 90. In some such examples, the reverse-threaded slider mechanism or slide assembly 30 comprises a left-handed thread 310.

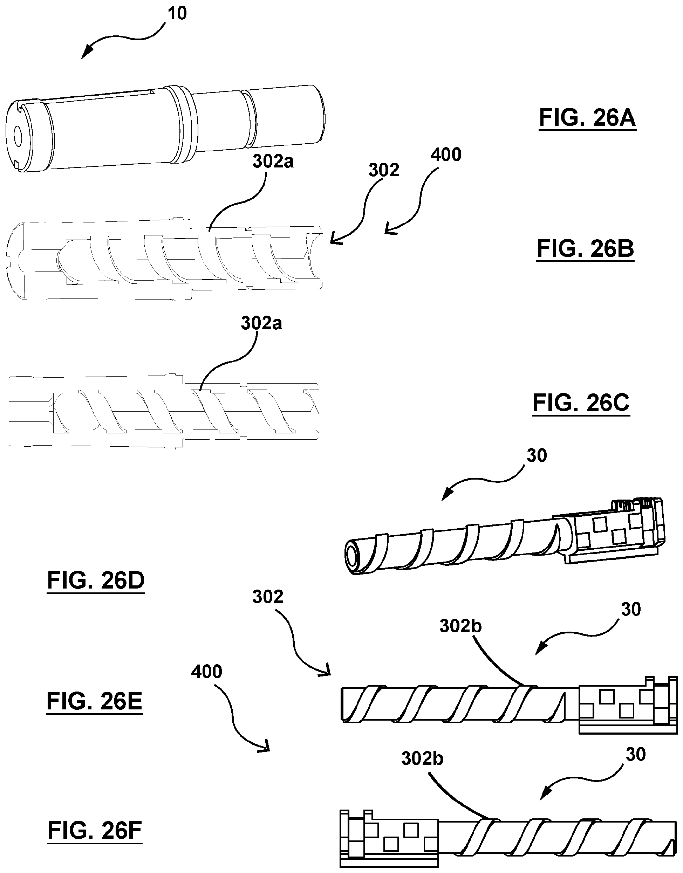

In some such examples, with reference to Fig. 23A, an inefficiency generator 400 is defined by a slider mechanism or slide assembly 30 having a reverse-threaded portion or bolt 32 that includes a left handed thread 310. The inefficiency generator 400 as defined may be provided in a steerable device, such as a steerable handle control system 200 that for example, has a greater range of motion for counter clock-wise rotation of a knob 10 of the steerable device than a clock-wise rotation of the knob 10. In some such devices, the threaded slider mechanism or slide assembly 30 (coupled to the knob 10) may be positioned within the handle 100, in a fully extended or translated position upon a substantially unactuated position of the knob 10 or upon substantially minimum rotation of the knob 10, defining a neutral position of the slide assembly 30, where the catheter or sheath 90 is substantially in its un-deflected position. The slide assembly 30 thus positioned thereby results in minimum threaded engagement between the threaded slider mechanism and inner threads of the knob 10 upon minimum deflection or a minimally deflected position of the sheath or catheter 90. As the knob 10 of the steerable catheter control handle is then actuated upon counter-clockwise rotation of the knob 10, employing a left-handed thread in the present arrangement of a steerable catheter control handle 200 helps to ensure translation of the threaded slider mechanism or slide assembly 30 towards the knob 10, thereby resulting in maximum threaded engagement between the threaded slider mechanism or side assembly 30 and the inner threads of the knob 10. In other words, counter-clockwise actuation of the knob 10 results in deflection of the sheath or catheter 90 as the threadable engagement 302 is being tightened, thereby resulting in maximum engagement between the threaded slider mechanism or slide assembly 30 and knob 10 (or in other words, specifically the external threads 302b on the bolt or shaft 32 of the slide assembly 30 and the inner or internal threads 302a of the knob 10, as also shown later with respect to Figs. 26A-26F) upon maximal deflection of the sheath or catheter 90.

In alternative examples, with reference to Fig. 23B, the inefficiency generator 400 comprising a right handed thread 309 may be provided in a steerable handle control system 200 that for example has a greater range of motion for clock-wise rotation of a knob 10 of the steerable device or steerable handle 200 than a counter clock-wise rotation of the knob 10. In other words, in some examples, the slide assembly 30 may be positioned within the handle 200 at a fully extended or translated position in a substantially unactuated position of the knob 10 or upon substantially minimum rotation of the knob 10, defining a neutral position of the slide assembly 30 where the catheter or sheath 90 is substantially in its un-deflected position. As the knob 10 of the steerable catheter control handle is then actuated upon clockwise rotation of the knob 10, this results in deflection of the sheath or catheter 90 for example as the threadable engagement 302 is being tightened, thereby resulting in maximum threaded engagement between the threaded slider mechanism (or in other words the slide assembly 30, specifically the external threads 302b on the bolt or shaft 32 of the slide assembly 30) and the inner or internal threads 302a of the knob 10. In other words, there is resulting maximum engagement between the slide assembly 30 and knob 10 upon clockwise rotation of the knob 10 at maximal deflection of the sheath or catheter 90.

Thus, in some such embodiments of the present invention, as shown in Figs, 23A and 23B, the inefficiency generator 400 enables increasing engagement between an actuator and the means for actuating the sheath or catheter 90 upon increasing actuation and thus increasing deflection of the sheath or catheter 90. As such the inefficiency generator 400 may help reduce slip. As above, in some such examples, the threadable engagement 302 comprises a reverse-threaded slider mechanism or slide assembly 30 that for example comprises a left-handed thread 310. In other examples, the threadable engagement 302 comprises a right-handed thread 309.

In some such examples, also shown in Figs 26A-26F, where the inefficiency generator 400 is defined by the threadable engagement or arrangement 302, the neutral zone of the slide assembly 30 is provided such that a maximum range of motion of the sheath or catheter 90 is defined in the direction where upon rotation of the knob, the inefficiency generator 400 provides a maximum threaded engagement between the threaded slider mechanism (or in other words the slide assembly 30, specifically the external threads 302b on the bolt or shaft 32 of the slide assembly 30) and the inner or internal threads 302a of the knob 10, for example as the threadable engagement 302 is being tightened. In such examples, additionally, a minimal range of motion of the sheath or catheter 90 is defined in the opposing direction where, upon rotation of the knob in the opposing direction, the inefficiency generator 400 provides a minimal threaded engagement between the threaded slider mechanism (or in other words the slide assembly 30, specifically the external threads 302b on the bolt or shaft 32 of the slide assembly 30) for example as the threadable engagement 302 is being loosened.

Alternatively, some embodiments of the present invention comprise a threaded slider mechanism or slide assembly 30 having a reduced thread pitch for increasing static friction between rotatable (such as a knob 10) and longitudinally translatable components (such as a threaded slider mechanism or slide assembly 30) of a steerable sheath control handle 200 .

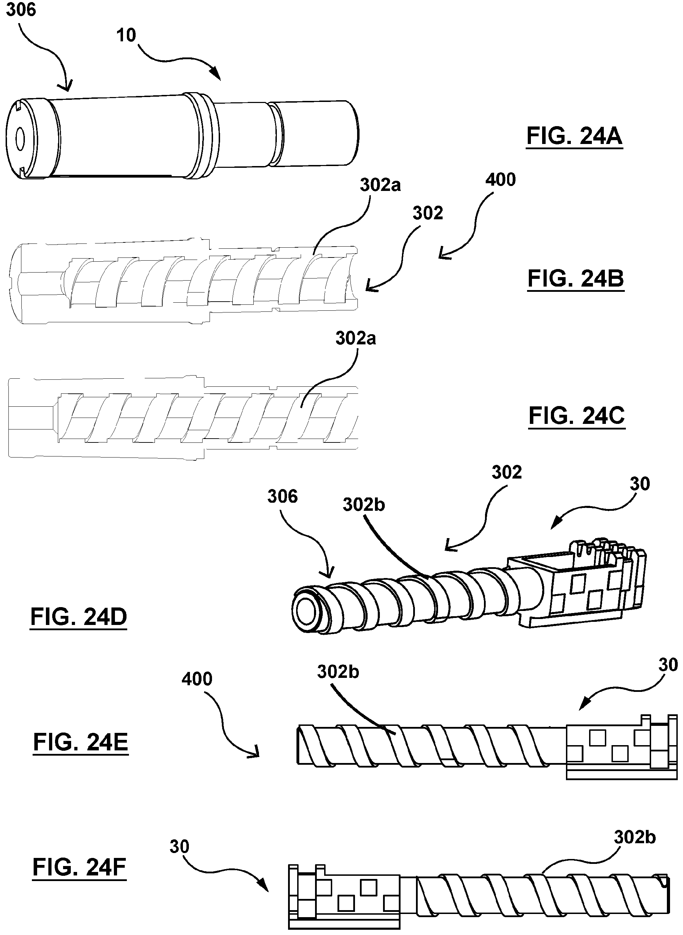

With reference now to Fig. 23C and Figs. 24A, 24B, 24C, 24D and 24E, 24F, a threaded slider mechanism 30 (or in other words the slide assembly 30) having a threaded portion with modified threads 306 having a reduced spacing between threads leading to an increased pitch 312. In such embodiments, the inefficiency generator 400 is defined by or integral to the rotation to linear translation system 300 comprising a slide assembly 30 and knob 10, where a threadable engagement 302 is provided between the slide assembly 30 (such as a bolt or shaft 32 of the slide assembly 30 having external threads 302b) having a reduced pitch and corresponding inner or internal threads 302a of the knob 10 having a reduced pitch 312. Reducing the spacing between threads leading to an increased pitch of the threadable engagement or threads 302 (such as for example, from 6,35 mm (1/4") to 12,7mm (1/2")) aids in further increasing static friction (or in other words frictional engagement) between the slider mechanism 30 or in other words slide assembly 30 (specifically the external threads 302b on the bolt or shaft 32 of the slide assembly 30 and the inner or internal threads 302a of a rotatable component (e.g. a knob 10)) of a steerable sheath assembly (thereby enabling the maintenance of a desired position, curve, or orientation of the articulating portion of the steerable sheath or catheter 90.

Both of the aforementioned embodiments (comprising modified threads 306 comprising reverse thread 308 such as a left thread or left-handed thread 310, right thread or right handed thread 309 and/or increased thread pitch 312) may be utilized, alone or in combination to provide an inefficiency generator 400, that in some examples functions to increase friction between rotatable (i.e. a knob 10) and translatable (e.g. a slider mechanism or slide assembly 30) components of a steerable sheath handle 200. In such examples, a rotatable component (e.g. a knob 10) of the steerable sheath assembly comprises threads 302b that are operable with the threads 302a of the threaded slider mechanism or slide assembly 30, whereby rotation of the knob 10 results in linear translation of the threaded slider mechanism or slide assembly 30.

In some such examples, the number of threads on the threaded slider mechanism or slide assembly 30 may vary based on the particular application, e.g. based on the degree of desired deflection of the articulating portion of the steerable sheath or catheter 90.

In alternative embodiments, an inefficiency generator 400 may be provided by modifying other components of a controlling component (e.g. a steerable control handle) of a medical device such as a steerable sheath or catheter 90 (such as components that are integral to or inherent to the rotation to linear translation system 300 or are integral or inherent in the system comprising a means for deflecting a sheath or catheter and the sheath or catheter 90). In some such examples in according to the invention, the inefficiency generator 400 functions to improve the device's ability to maintain a particular position, curve, or orientation of the articulating portion of the device. For example, overall stroke (extension or translation distance) of the slider mechanism or slide assembly 30 may be reduced, thereby restricting the range of motion of the device to ensure sufficient threaded engagement between the rotatable and translatable components. Alternatively, or in addition, an O-ring or similar component may be included or increased in size to increase friction. Furthermore, lubricant and/or damping grease may be employed or modified to optimize friction. Yet furthermore, in embodiments of steerable sheaths assembly using a control handle such as control handle 200 utilizing direction-reversing elements such as a pulley to guide one of the pull- wires of the device, the wiring or configuration of the pull-wires or control wires on the two sides of the sheath may be reversed so that the greater range of motion is damped by use of the pulley. In a further alternative embodiment, the sheath tubing may be modified to provide reduced force for deflection.

Further details regarding an exemplary medical device with which embodiments of the present invention may be utilized are provided in U.S. provisional patent application serial number 61/661,664, filed on 19- June-2012, and in PCT application serial number PCT/IB2013/055013, filed in English on 18-June-2013 designating the United States of America.

With reference again to Figs. 24A-24F, an alternate embodiment of a threaded slider mechanism or slide assembly 30 is provided that is usable in a steerable handle 200, as described previously herein above. The slide assembly 30 is provided with external threads 302a with an increased pitch 312 and a corresponding threaded knob 10 (such an inner knob) is provided that has internal threads 302b with an increased pitch, the combination defining an inefficiency generator 400, in accordance with an embodiment of the present invention. The increased pitch 312 provides reduced spacing between threads 302, allowing the inefficiency generator 400, to improve the device's ability (in other words the ability of the steerable handle 200) to maintain a particular position, curve, or orientation of the articulating portion of the device such a sheath or catheter 90. In the illustrated embodiment shown in Figs. 24A-24F, the inefficiency generator 400 comprises a right handed thread 309 with an increased pitch 312 equal to about 6,35mm (1/4") pitch is used.

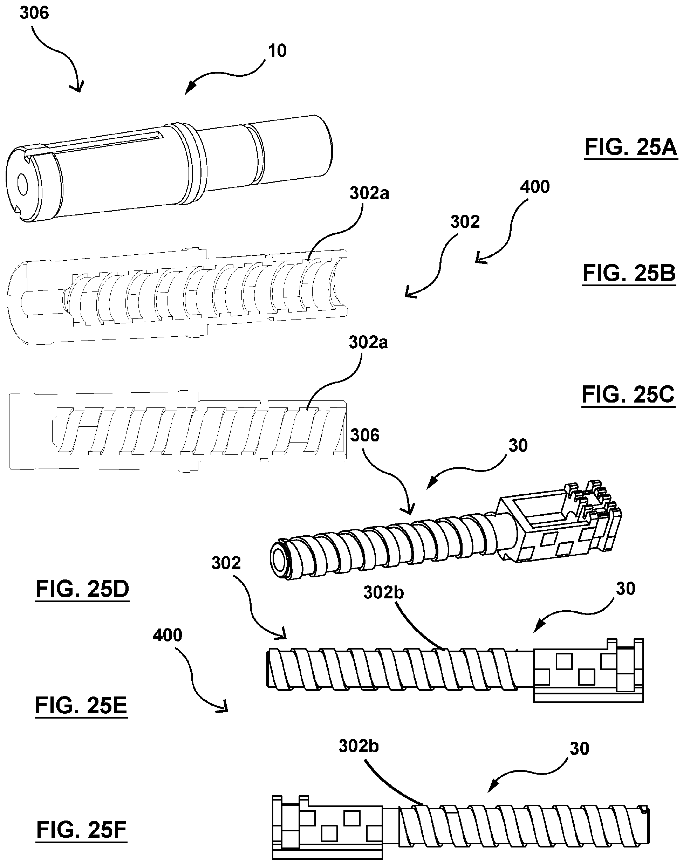

With reference now to Figs. 25A, 25B, 25C, 25D, 25E, and 25F, an alternate embodiment of an inefficiency generator 400 is shown, comprising a threaded slider mechanism or slide assembly 30 with an increased pitch and reduced spacing between threads 302 (external threads 302a) and a corresponding threaded knob 10 (such as an inner knob) having internal threads 302b is shown, in accordance with an embodiment of the present invention. The increased pitch 312 provides reduced spacing between threads 302 to improve the device's ability to maintain a particular position, curve or orientation of the articulating portion of the device. In the illustrated embodiment shown, the inefficiency generator 400 comprises a right handed thread 309 with an increased pitch 312 equal to about 9,53mm (3/8") pitch is used.

With reference now to Figs. 26A, 26B, 26C, 26D, 26E, and 26F, an alternate embodiment of a threaded slider mechanism or slide assembly 30 is provided with an reverse or reversed thread 308 and a corresponding threaded knob 10 (such as a threaded inner knob) with a reverse thread 308 is provided, the combination defining an inefficiency generator 400, in accordance with an embodiment of the present invention. The inefficiency generator 400 comprising a reverse thread 308 is usable in a steerable handle 200, as described previously herein above. The reverse thread 308 allows the slide assembly 30 to be positioned within the steerable handle 200 such that it allows the inefficiency generator 400, comprising the threadable engagement 302 comprising a reverse thread 308, to improve the device's ability (in other words the ability of the steerable handle 200) to maintain a particular position, curve, or orientation of the articulating portion of the device such a sheath or catheter 90. The reverse thread 308 ensures that there is maximal engagement between the slide assembly 30 and the knob 10 (such an inner knob), when the sheath is maximally deflected, upon counter clock-wise rotation of the knob 10, placing the slide assembly 30 in the least extended or least outward position of the slide assembly 30 away from the knob 10 (such as the inner knob), which is the case when the sheath is maximally deflected. In the illustrated embodiment, shown in Figs. 26A-26F, the inefficiency generator 400 comprises a left handed thread 310 with a pitch equal to about 12,7mm (1/2") pitch is used.

As such, some embodiments of the present invention, as outlined in the present disclosure, relates to several embodiments of an inefficiency generator 400 (for example comprising one or more components of a steerable handle 200), that is usable in medical devices to assist in maintaining a desired position of an articulating component such as a sheath or catheter 90 by retaining a position or state of the controlling component. For example, some embodiments of an inefficiency generator 400 disclosed herein are operable to increase friction between rotatable (i.e. a knob 10) and translatable (e.g. a slider mechanism or slide assembly 30) components of a steerable handle 200 of a steerable sheath assembly.

As noted previously, the present invention provides an inefficiency generator 400 that functions to add inefficiency in the translation mechanism or system of a steerable catheter control system 200 (a rotation to linear translation system 300) that enables deflection of the sheath or catheter 90 via movement of a slide assembly 30 upon actuation of a knob 10. The inefficiency generator 400 functions to increase the amount of force required to actuate (or in other words resistance to actuate) the actuator (such as knob 10) upon increasing deflection of the sheath or catheter 90. The inefficiency generator 400 provides one or more advantages as outlined herein above, including curve retention and providing feedback regarding the amount of actuation of the actuation and the degree of deflection of the sheath or catheter 90.

Some embodiments of the present invention provide an inefficiency generator 400 within the steerable catheter control handle 200 that enables a user such as a physician when operating such a steerable control mechanism to ascertain how much the sheath is being deflected upon actuation, including when they are in the neutral zone, or how far away they are from it - the neutral zone being the region where neither of the deflection wires is placed under tension. Thus, an inefficiency generator 400 as provided within a steerable control system 200 for a steerable sheath or catheter 90, provides feedback regarding the deflection of the steerable sheath (for example, how much the sheath or catheter 90 has been deflected upon actuation or in other words a relative degree of deflection of the sheath or catheter 90) and may additionally provide a feedback mechanism that provides an indication when the catheter control mechanism or assembly 200 and as such the deflectable distal end of the sheath is in its neutral position, as such providing neutral zone feedback.

More specifically in accordance with an embodiment of the present invention, an inefficiency generator 400 provides (in other words includes) a mechanism that provides tactile feedback for identifying the deflection or position of the sheath or catheter 90, and additionally in some embodiments, tactile feedback for identifying the neutral zone.

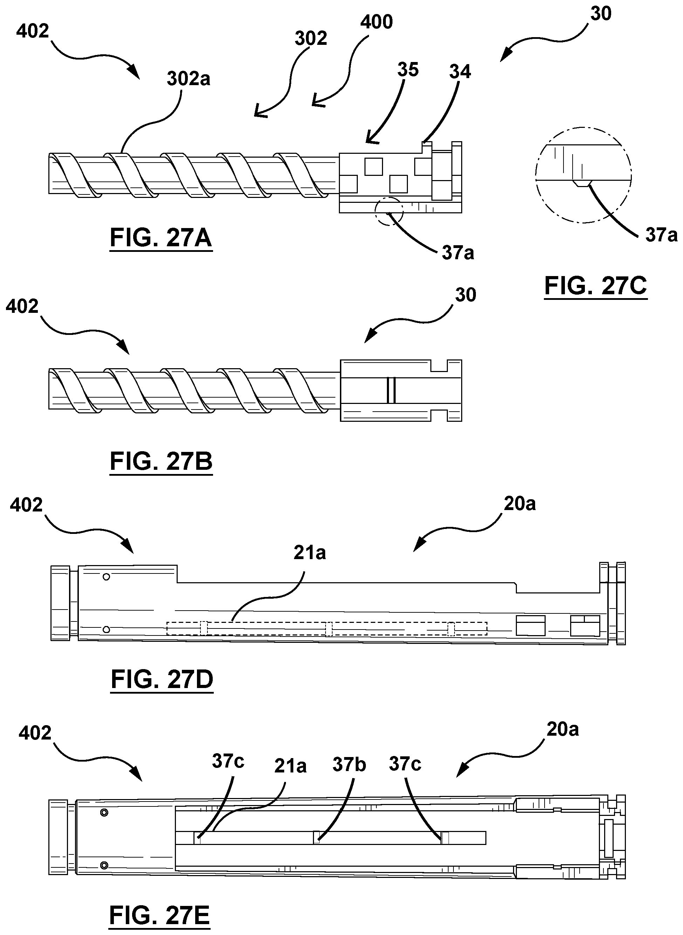

With reference now to Figs. 27A, 27B, 27C and 27D and 27E, in accordance an embodiment of the present invention a steerable catheter control handle 200 as previously, comprises an actuation mechanism for deflecting the sheath or catheter 90 (or in other words a means for deflecting the sheath or catheter or a deflection mechanism). The actuation mechanism or deflection mechanism is provided that comprises a control knob 10 that is operational to deflect the distal end of the sheath or catheter 90 by causing linear movement of a slide assembly 30 within a housing 20, such as inner housing 20a, as shown in Figs. 27D and 27E. In the specific embodiment shown, the handle 200 comprises an inefficiency generator that comprises a feedback mechanism 402 (that in other words includes or incorporates a feedback mechanism 402). The feedback mechanism 402 introduces inefficiency into the actuation mechanism of the handle 200 that enables deflection of the sheath or catheter 90 upon actuation of the knob 10. The feedback mechanism 402 interacts with the actuation mechanism to provide feedback. The actuation mechanism in this example is a rotation to linear translation mechanism 300, comprising a knob 10 that actuates a slide assembly 30, to allow the slide assembly 230 to translate within a track of the inner housing 20a of the handle 200, to actuate one of the two pull wires of the sheath or catheter 90 in order to deflect a distal end of the sheath or catheter 90.

Similar to embodiments outlined previously herein above, the pull wires may be coupled at their proximal ends to slide assembly 30 (for example at a carriage 34 or specifically a wire crimping area of slide 35 of via a crimp,) with one of the wires being passed through a direction reversing element prior to being coupled to the slide 35. The pull wires may also be coupled to the distal end of the sheath at their respective distal ends. Actuation of the knob 10 results in movement of the slide assembly 30, which comprises a bolt or threaded component or shaft 32 coupled to slide 35 that is operable to engage the control knob 10 to enable translation of the slide assembly 30.

In the present example, the inefficiency generator 400 comprises a feedback mechanism 402 (that in other words includes or incorporates a feedback mechanism 402) that interacts indirectly with the rotation to linear system 300. Specifically, the feedback mechanism 402 comprises a tactile feedback mechanism 404 that provides a tactile indicator or feedback. In the specific example shown, the feedback mechanism 402 provides tactile feedback when the slide assembly 30 reaches a specific point along a track of the inner housing to indicate the deflection of the sheath or catheter 90, which in some examples indicates the neutral zone and in some examples indicates the level or degree of deflection or curvature of the sheath of catheter upon actuator of the actuator.

For example, the inefficiency generator 400 comprises a feedback mechanism or an indicator mechanism 402 (or in other words the inefficiency generator 400 includes or incorporates a feedback mechanism 402). The feedback mechanism 402 is defined by a tactile indicator or in other words tactile feedback mechanism 404 that in one example is provided by the addition of material at a specified location on the handle track (formed within the inner housing 20a) as well as on the slide assembly 30 to provide a tactile indicator when the handle knob 10 activates the slide assembly 30 to travel through the specified location along the track. In one such example, the feedback mechanism 402 is positioned which indicates deflection of the sheath or catheter 90. The steerable catheter control handle 200 may be assembled such that the tactile indicator coincides with the position of the slide assembly when sheath is curved (As such the tactile indicator provides physicians with feedback to indicate how much the catheter control handle and thus distal end of the sheath has been deflected. In one such example, the feedback mechanism 402 is positioned towards the opposing sides of the track 21a (for example towards the extremities of the track 21a).

In another example, the feedback mechanism 402 is positioned for identifying the neutral zone. The steerable catheter control handle 200 may be assembled such that the tactile indicator coincides with the neutral position of the distal sheath curvature to indicate when the sheath or catheter 90 is its nominal or neutral position. In one such example, the feedback mechanism 402 is positioned towards the center of the track 21a.

In some such embodiments, the inefficiency generator 400 comprises a feedback mechanism 402 (that in other words includes or incorporates a feedback mechanism 402) that comprises additional material in the form of a projection that is provided on both the underside of the slide 35 of the slide assembly 30 as well as within the track 21a of the inner handle (specifically the inner housing 20a of a handle, such as a handle 100,200, in order to provide tactile feedback. More specifically, a tactile indicator is provided within the steerable catheter control handle that comprises a first indicator portion 37a that extends downwards from a bottom face of the slide 35 (as shown in Figs. 27A-27C) along a location on the slide 35, and one or more second indicator portion 37b, 37c that are formed at one or more locations within the track 21a of the inner housing 20a and extend upwards into the handle interior (as shown in Figs. 27D-27E). The position of the first indicator portion 37a on the slide 35 may be referred to as the activation point on the slide assembly 30. For the sheath or catheter 90 to be deflected in the first or second direction upon actuation of the knob 10, the slide assembly 30 may first cross the neutral zone.

In some such embodiments, the physician may choose to deflect the sheath or catheter 90 in a first direction by actuating the knob 10 clockwise, The physician may also choose to deflect the sheath or catheter 90 in a second direction by rotating the knob counter clockwise, enabling the slide assembly 30 to travel distally along the track 20t in a second direction opposite to the first direction of travel. In order for the sheath to be deflected in the second direction the slide assembly 30 has to first cross the neutral zone. In one example, of the inefficiency generator 400 comprising the feedback mechanism 402 (that in other words includes or incorporates a feedback mechanism 402) as shown, once the slide assembly 30 reaches the neutral zone (for example when switching between clockwise and counter clockwise actuation of the knob 10), the first indicator portion 37a of the slide assembly 30 interacts with the second indicator portion 37b to provide a tactile indication, informing the physician that the steerable sheath is now in its neutral position. In one such example the first and second indicator portions 37a, 37b provide a mechanical indication as well as an audible indication upon interaction with one another. The first and second indicators 37a, 37b are tactile indicators that interact with one another such that sufficient resistance is created to provide a tactile indication but the movement of the slide assembly 30 is not stopped. In the present example, the feedback mechanism 402 provides simulated feedback of catheter deflection. The inefficiency generator 400 comprising the feedback mechanism 402, additionally provides discrete feedback of the state of deflection of the sheath or catheter 90 (specifically for when the sheath or catheter is substantially un-deflected and slide assembly 30 is in the neutral zone, thus indicating that the sheath or catheter 90 is in the neutral position.

In another example of the inefficiency generator 400 comprising the feedback mechanism 402 (that in other words includes or incorporates a feedback mechanism 402) as shown, upon actuation as the sheath or catheter 90 is deflected in a first or a second direction, once the slide assembly 30 travels towards the opposing ends of the inner housing 20a, the first indicator portion 37a of the slide assembly 30 interacts with the second indicator portion 37c to provide a tactile indication, informing the physician that the steerable sheath is now in its neutral position. The first and second indicator portions 37a, 37c that define tactile indicators, interact with one another such that sufficient resistance is created to provide a tactile indication but the movement of the slide assembly 30 is not stopped. Similar to the neutral zone feedback, in the present example, the inefficiency generator 400 provides simulated feedback of catheter deflection. The inefficiency generator 400 comprising the feedback mechanism 402, additionally provides discrete feedback of the state of deflection of the sheath or catheter 90 (specifically for when the sheath or catheter is in a substantially deflected position and the slide assembly 30 is beyond the neutral zone towards the maximum translated position of the slide assembly 30, thus indicating that the sheath or catheter 90 is in a deflected position. In some such examples, the discrete indication provided by the inefficiency generator 400 comprising the feedback mechanism 402, may provide an indication of the curvature of the sheath or catheter 90 upon deflection. In one example, the feedback mechanism 402 may provide an indication upon a 90 degree deflection or curvature of the sheath or catheter 90. In another example, the feedback mechanism may provide an indication upon a 180 degree deflection or curvature of the sheath of catheter 90. In one such example the first and second indicator portions 37a, 37c provide a tactile indication that provides a mechanical indication as well as an audible indication upon interaction with one another.

In one specific example of the inefficiency generator 400 comprising the feedback mechanism 402 (in other words the inefficiency generator 400 includes or incorporates a feedback mechanism 402). The feedback mechanism 402 comprising first and second indicator portions 37a and 37b, 37c respectively, the first and second indicator portions 37a and 37b, 37c may have a height of about 1 mm. In some such instances sufficient clearance may be provided within the handle to allow the slide assembly 30 to bump up and down over the second indicator portion 37b, 37c. In some embodiments, the added material of the first and second indicator portions 37a and 37b, 37c may have a square leading and trailing edge. In some embodiments the trailing and leading edges of the first and second indicator portions 37a and 37b,37c may be angled which may make the movement of the slide assembly 30 along the track 20t smoother at the second indicator portion 37b, 37c and may minimize the risk of binding. This may allow the physician to continue to rotate the knob 10 either clockwise or counter clockwise to deflect the sheath or catheter 90 in the first or second directions as desired. Thus, in accordance with one embodiment of the present invention, the feedback mechanism 402 provides a neutral zone indicator is provided that identifies a neutral zone for a bidirectional steerable control handle. The tactile indicator allows a single neutral zone to be identified independent of the direction that the slide assembly 30 travels. Alternatively or in conjunction with the neutral zone indicator, in accordance with one embodiment of the present invention, the feedback mechanism 402 provides an indication that identifies a degree of deflection for a bidirectional steerable control handle. In some examples, the indicator may provide a relative degree of deflection of the sheath 90. In other examples, the indicator may provide an indication of substantially absolute deflection of the sheath 90 [for example the angle of curvature of the sheath 90].

In some such embodiments, the material forming the first and second indicator portions 37a and 37b, 37c is sufficiently hard to provide a tactile indication, while being sufficiently thin or narrow such that it provides feedback without impeding or stopping the movement of the slide assembly 30 within the handle. As such the tactile indicator provides feedback to the user without impeding the functionality of the system control system. In a specific example of this, each of the first and second indicator portions 37a and 37b, 37c have relatively narrow length along the longitudinal axis of the handle, in reference to the longitudinal length of the slide 35 and the inner housing 20a. In some such embodiments, the closer the added material forming indicator portions 37a and 37b, 37c is to a line/point [i.e. the narrower the length of the added material], the more precise the tactile indicator is as it enables the tactile trigger to be triggered during travel of the slide assembly 30 in both the proximal and the distal directions along the track 20t. In one such example, the tactile indicator first and second portions 37a and 37b, 37c are positioned so as to facilitate repeatability during manufacturing and to ensure that the remaining components of the handle assembly can be assembled and positioned with respect to it.

In some embodiments of the present invention, the first and second indicator portions 37a and 37b, 37c may be permanently fixed to the slide assembly 30 and the inner housing 20a, respectively. In one such example the first and second indicator portions 37a and 37b, 37c may be formed through injection molding or alternatively the first and second indicator portions 37a and 37b, 37c are machined feature. Alternatively, these components may be machined. In one particular example, the slide assembly 30 as well the first indicator portion 37a may be molded together and may comprise DELRIN^{®}, whereas Acrylonitrile butadiene styrene (ABS) may be utilized for the inner housing 20a as well as the second indicator portion 37b, 37c. Alternatively, in some embodiments polycarbonate may be used to form one or more of the components.

Alternatively, in some embodiments of the present invention, the slide assembly 30 may be modified such that the position of the first indicator portion 37a (or alternatively speaking the activation point on the slide assembly 30) may be altered or varied. In some such embodiments, the first indicator portion 37a may be positioned at different locations on the slide 35 and may have varying geometry or dimensions. Similarly, the inner housing 20a may be modified such that the position of the second indicator portion 37b, 37c (or alternatively speaking the activation point on the inner housing 20a) may be altered. In some such embodiments, the second indicator portion 37b, 37c be may be positioned at different locations within the track 21a of the inner housing 20a and may have varying geometry or dimensions. As such in some examples, an inefficiency generator 400 provides feedback mechanism 402 to indicate when the slide assembly 30, and as such, the steerable control system and the distal end of the sheath, are within the neutral zone. In other words, the handle 200 comprises a mechanism for providing feedback when the slide assembly 30 reaches a point along the track 21a where neither of the pull wires are in tension, and the sheath distal end is in its neutral position. The inefficiency generator 400 providing feedback such as feedback of deflection of the sheath or catheter 90 in its substantially un-deflected state provides neutral zone feedback, as such may facilitate the use of the steerable control handle by the physician during a procedure and may additionally reduce procedural complexity and/or time. In one such embodiment, feedback may be provided in the form of a tactile indicator when the slide assembly 30 reaches a specific point along the track 21a of the inner housing 20a which indicates the neutral zone.

As such the inefficiency generator 400 may facilitate the use of the steerable control handle 200 by the physician during a procedure and may additionally reduce procedural complexity and/or time. Furthermore, the inefficiency generator 400 of the present invention provides tactile feedback and as such may not cause the user to look at their hands or the product during the procedure and as such may facilitate use of the steerable sheath by the physician.

Therefore in accordance with an embodiment of the present invention a method and apparatus are disclosed for a steerable catheter control handle 200 that provide an inefficiency generator 400 that functions to provide feedback in the form of an indicator to assist the physician in identifying the degree of deflection of the sheath or catheter 90, which may include for example an indication of the neutral zone.

In one embodiment of the present invention, the steerable control system comprising a handle with an inefficiency generator 400 comprising a feedback mechanism 402 that may include feedback of sheath deflection and/or neutral feedback indicator as discussed herein above, which in some examples may be used for steering an introducer sheath for use in an electrophysiology environment. In further alternatives, the thread pitch on the threaded portion may be increased such that the handle knob rotates less than about 360 degrees in each direction and a visual or tactile indicator may be integrated directly into the handle to provide an indication of the deflection of the sheath or catheter 90, including for example the neutral zone.

In alternative embodiments, the handle may be attached to and used with other steerable catheter products that may be used in other applications and an inefficiency generator 400 may be provided that may function to provide an indication of the neutral zone when deflecting the steerable products. Alternatively, the inefficiency generator 400 may provide a neutral feedback mechanism that may be used in the handle to indicate another distal curve position, for example when deflecting the steerable products.

Further details regarding an exemplary medical device with which embodiments of the present invention may be utilized are provided in U.S. provisional patent application serial number 61/661,664, filed on 19-June-2012, and in PCT application serial number PCT/IB2013/055013, filed in English on 18-June-2013 designating the United States of America.

As described hereinabove, the present inventors have discovered an apparatus and method for a steerable catheter control handle that provides an inefficiency generator 400 that provides a feedback mechanism 402 that provides an indicator for identifying the deflection of the sheath or catheter 90 and may help identify the neutral zone of the steerable control handle. More specifically in accordance with an embodiment of the present invention, a mechanism is provided for identifying the deflection of the sheath or catheter 90 through tactile feedback. Further examples are provided herein below for an inefficiency generator 400 comprising a feedback mechanism 402 for indicating deflection of the sheath or catheter 90, such as a neutral zone feedback mechanism for a steerable control handle.

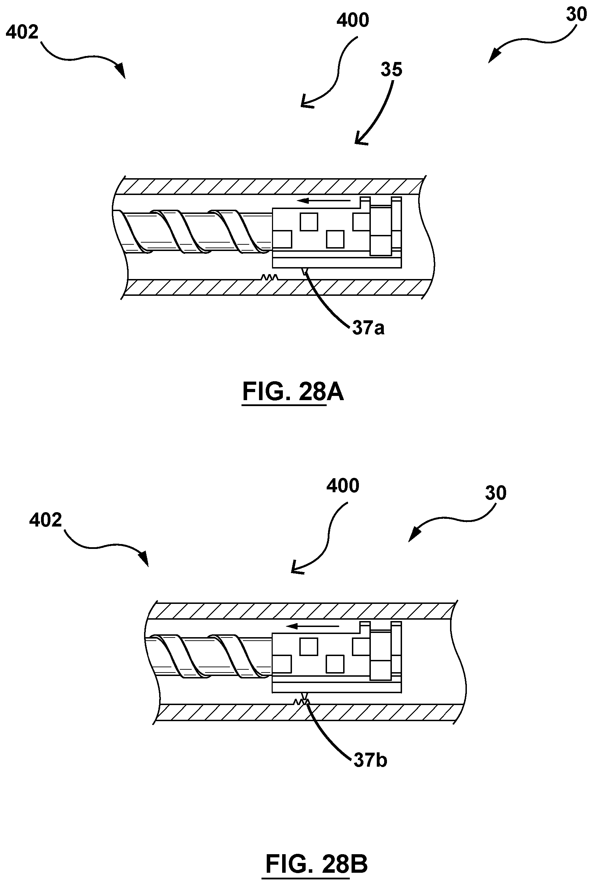

In accordance with an additional embodiment of the present invention, with reference to Figs. 28A-28B, an inefficiency generator 400 is provided comprising a feedback mechanism 402 comprising a tactile indicator. In one such example, the feedback mechanism comprises a handle position indicator. In some embodiments, a steerable catheter control handle 200 is provided s such as a bi-directional steerable handle 200.

As noted previously hereinabove, an actuator is provided on the control handle 200 that comprises a knob 10 on the steerable control handle 200 such as a steerable sheath handle may be actuated for example by rotating or twisting in either direction to pull one of the two pull wires and deflect the sheath or catheter 90 such as a distal tip of the sheath or catheter 90. In the embodiments describe herein, an inefficiency generator 400 is provided that provides a feedback mechanism 402 that provides enhanced feedback when deflecting the distal tip from one direction to the next, and may be advantageous to indicate to the user the degree and/or direction of deflection of the sheath or catheter 90.

The feedback mechanism 402 may additionally indicate a change or switch in the direction of deflection of the sheath or catheter 90. Additionally, as before, in some embodiments, where the steerable catheter control handle 200 provides a "dead zone" where neither of the two pull wires is in tension (for example for a few millimeters) as the knob 10 is turned, the inefficiency generator 400 comprising the feedback mechanism 402 as presently provided, may provide tactile feedback that is indicative of this "dead zone" or in other words tactile feedback is provided from this "dead zone". As such, the present disclosure provides an inefficiency generator 400 that provides a feedback mechanism 402 that provides enhanced feedback of sheath or catheter deflection such as for example the degree of curvature and/or direction of the sheath curvature and/or neutral zone feedback for a steerable control handle.

In one embodiment, an inefficiency generator 400 is provided that comprises a feedback mechanism 402 that provides a tactile indication. The feedback mechanism 402 comprises a first indicator portion 37a, that for example comprises one or more fins, such as a fin in one example that is incorporated into a posterior portion of the slider or slide 35 of the slide assembly 30, as shown in Figs. 28A and 28B. The feedback mechanism 402 additionally comprises a second indicator portion 37b that in one example comprises one or more ridges (that may be similar to a washboard) that are added to a handle housing 20 such as an inner housing 20a of the steerable catheter control handle 200 (or an inner portion of an inner handle thereof). As the knob 10 of the steerable catheter control handle 200 is actuated such as for example by turning it or by twisting it, the slide assembly 30 moves via a threadable engagement 302 between the bolt or shaft 32 of the slide assembly 30 and the knob 10, causing the slide 35 to move within the inner housing 20a creating interaction between the first and second indicator portions 37a, 37b (in other words enabling the two features (the fin and the ridges to meet). At this point, the inefficiency generator 400 comprises the feedback mechanism 402, where the feedback mechanism 402 provides a mechanical indication that would allow a user such as a physician to receive either tactile or sound feedback indicating the first indicator portion 37a such as fin is moving against the second indicator portion 37b such as one or more ridges indicating the amount of deflection or the state of the sheath or catheter with respect to how much it is curved. In one such example as shown, the feedback mechanism 402 as shown indicates that the sheath or catheter 90 (such as a distal tip thereof) is at a neutral state (in other words the sheath or catheter 90 is substantially not deflected). In other examples, similar to embodiments shown in Figs. 27A-27D, the second indicator 37b (such as the ridges) may be positioned elsewhere in the inner housing 20a of the steerable catheter control handle 200, and as such the feedback mechanism 402 indicates the deflection of the sheath or catheter 90 (such as a distal tip thereof) to indicate whether it is in its deflected position. In some such examples, the feedback mechanism 402 may indicate that the sheath or catheter 90 is substantially deflected. In some such example, the feedback mechanism 402 provides discrete feedback. In other examples, the feedback mechanism 402 provides relative feedback of deflection. In some such examples, the feedback mechanism 402 may provide variable feedback for a range of deflection of the sheath or catheter 90.

In additional embodiments of the present invention as noted herein, the position of first and second indicator portions 37a, 37b of the inefficiency generator 400 (such as one or more fins and ridges), as well as the geometrical dimensions of the first and second indicator portions 37a, 37b (such as one or more fins and ridges may be varied).

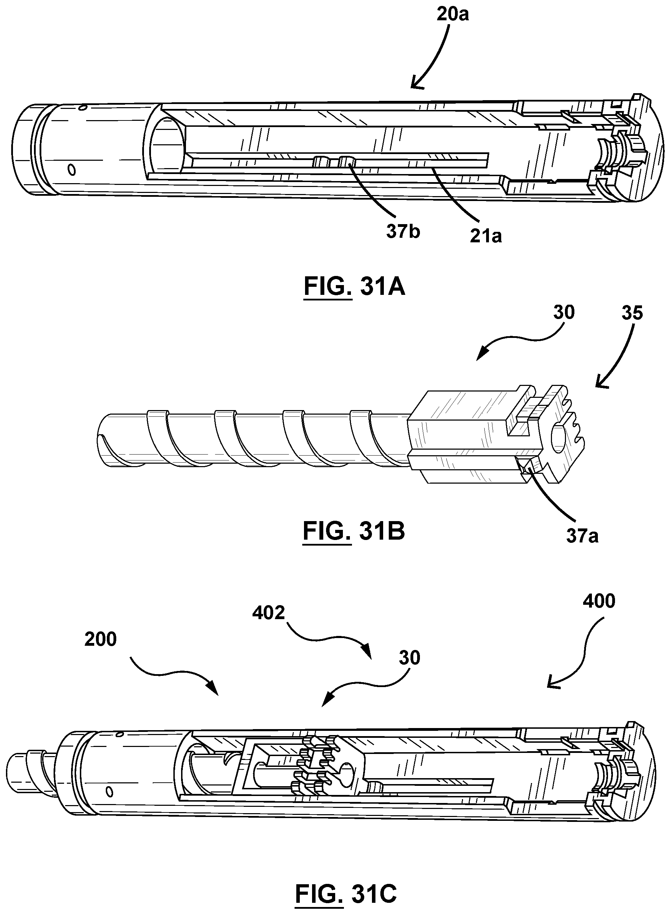

In another such embodiment of the present invention, as shown in Figs. 31A, 31B, 31C, an inefficiency generator 400 is provided that comprises a feedback mechanism 402 (that in other words includes or incorporates a feedback mechanism 402) for providing or adding tactile feedback for indicating sheath deflection.
As before the feedback mechanism 402 comprises a first indicator portion 37a on the bottom of the slide portion 35 of the slide assembly 30 such as fin (as shown in Fig. 31B) that is operable to interact with the second indicator portion 37b such as one or more ridges or bumps on the inner housing 20a (as shown in Fig. 31A, for example along a track 21a of the inner housing 20a). The first indicator portion 37a is operable to interact with the second indicator portion 37b as the slide assembly 30 moves there against as shown in Fig. 31C, indicating the amount of deflection or the state of the sheath or catheter with respect to how much it is curved, or as shown in Figs. 31A- 31C, the position of the neutral zone where the sheath 90 is in its substantially un-deflected state.

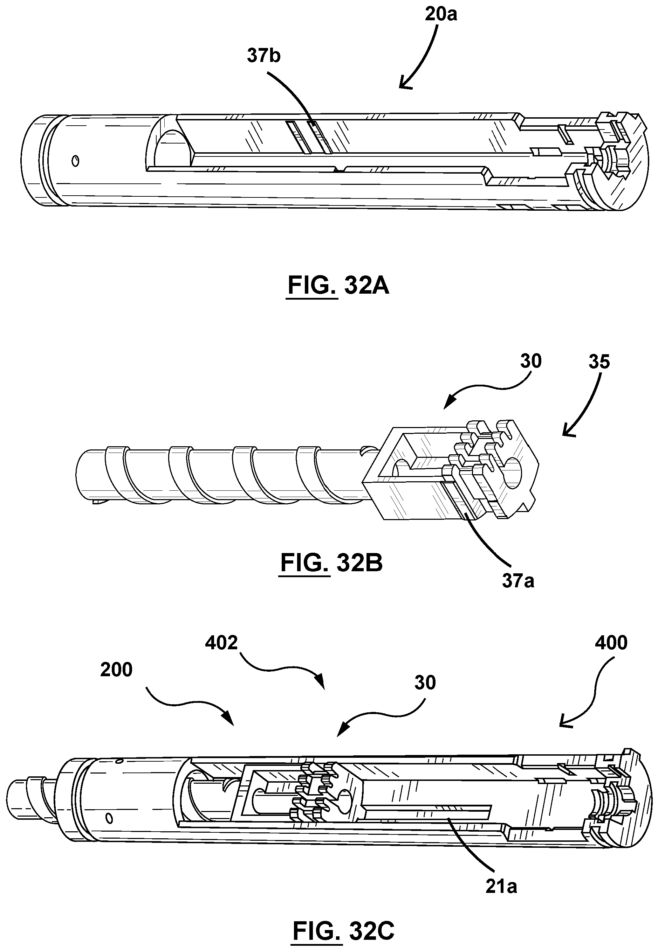

In another such embodiment of the present invention, as shown in Figs. 32A, 32B and 32C, an inefficiency generator 400 is provided that comprises a feedback mechanism 402 (that in other words includes or incorporates a feedback mechanism 402) for providing or adding tactile feedback for indicating sheath deflection. As before the feedback mechanism 402 comprises a first indicator portion 37a on a side of the slide or slide portion 35 of the slide assembly 30 such as a side bump (as shown in Fig. 32B) that is operable to interact with the second indicator portion 37b such as one or more side bumps on the inner housing 20a (as shown in Fig. 32A, for example along an inner wall of the inner housing 20a). The first indicator portion 37a is operable to interact with the second indicator portion 37b as the slide assembly 30 moves there against as shown in Fig. 32C, indicating the amount of deflection or the state of the sheath or catheter with respect to how much it is curved, or as shown in Figs. 32A-32C, the position of the neutral zone where the sheath 90 is in its substantially un-deflected state.

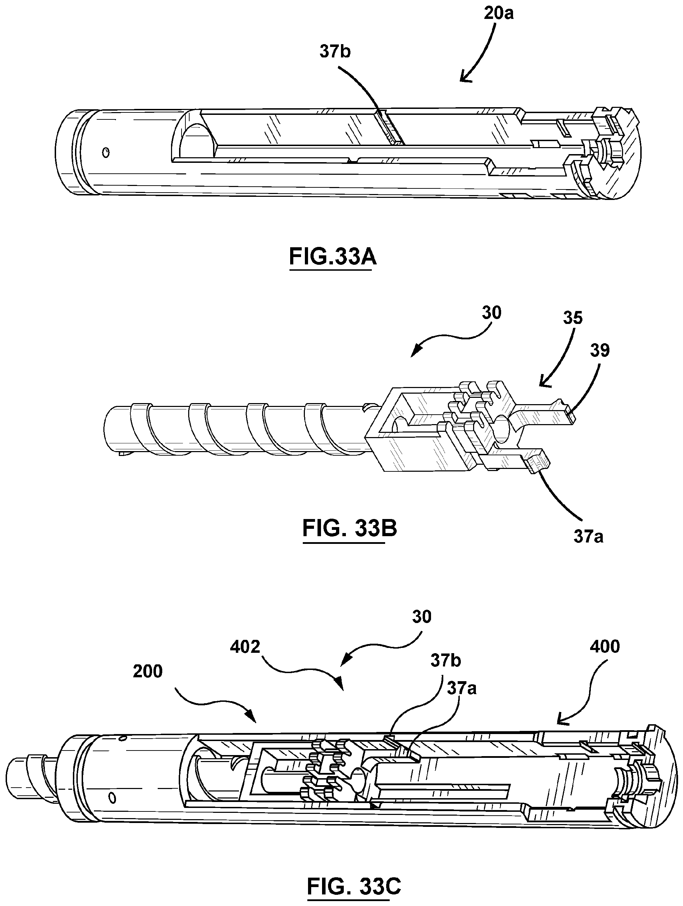

In another such embodiment of the present invention, as shown in Figs. 33A, 33B, 33C, an inefficiency generator 400 is provided that comprises a feedback mechanism 402 (that in other words includes or incorporates a feedback mechanism 402) for providing or adding tactile feedback for indicating sheath deflection. As before the feedback mechanism 402 comprises a first indicator portion 37a on a side of the slide portion 35 of the slide assembly 30 such as a side bump along a cantilever portion 39 of the slide assembly (as shown in Fig. 33B) that is operable to interact with the second indicator portion 37b such as one or more grooves on the inner housing 20a (as shown in Fig. 33A, for example along an inner wall of the inner housing 20a). The first indicator portion 37a is operable to interact with the second indicator portion 37b as the slide assembly 30 moves there against as shown in Fig. 33C, for example by being received within the one or more grooves of the second indicator portion 37b, indicating the amount of deflection or the state of the sheath or catheter 90 with respect to how much it is curved, or as shown in Figs. 33A-33C, the position of the neutral zone where the sheath 90 is in its substantially un-deflected state.

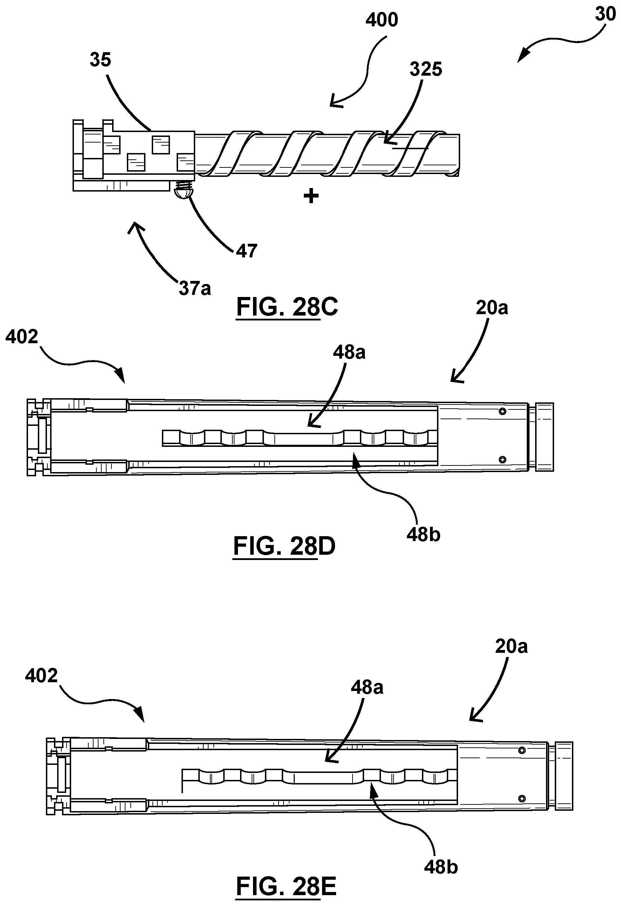

In accordance with an additional embodiment of the present invention, with reference to Figs. 28C-28E, an inefficiency generator 400 is provided comprising a feedback mechanism 402 comprising a tactile indicator. In some such example, the feedback mechanism 402 comprises tactile knob feedback. In some embodiments, a steerable catheter control handle 200 is provided such as a bi-directional steerable handle 200. In one such example, a tactile indicator may be usable with other forms of visualizing the position or deflection of the bi-directional steerable sheath for example during use thereof. For example, a visual indication is provided to the physicians on distal tip curve reaction to handle knob rotation through the use of medical imaging (i.e. fluoroscopy).

In an embodiment of the present invention, tactile feedback is provided through a feedback mechanism 402 defining an efficiency generator 400 as described herein below with reference to Figs. 28C, 28D and 28E. In some embodiments, the feedback mechanism 402 may provide variable tactile feedback over the range of motion of knob rotation. In other words the feedback mechanism 402 may provide variable feedback based on the amount of rotation of the knob 10. In some such examples, as the knob 10 is rotated the amount of feedback given to user changes giving the user a different tactile indication or feel as the knob 10 is rotated. This may allow the user to ascertain whether the user is in the neutral position or if the sheath or catheter has been deflection and may additionally provide the user with an indication of the amount or degree of deflection of the steerable sheath. In some embodiments, the tactile feel of knob rotation may have some variance over the range of motion (in both curve directions) with the current embodiment. In some embodiments, the tactile feel may also be influenced by (for example to enhance or to reduce the tactile feel) by incorporating additional components and the properties thereof, for example, such as a friction enhancing component such as an O-ring and/or lubricant such as a damping grease.

As such, some embodiments of the present invention provide an inefficiency generator 400 that provides (or in other words includes or incorporates) a feedback mechanism 402 for providing or adding tactile feedback that may be tied directly or indirectly to knob rotation (which in turn relates to expected distal tip curve reaction for the physician), as well as tactile feedback to indicate neutral zone which in some embodiments may correlate with a substantially straight sheath tubing - in other words substantially no distal curve activation.

With reference again to Figs 28C, 28D and 28E, another embodiment of present invention incorporates an inefficiency generator 400 comprising a feedback mechanism 402 (that in other words includes or incorporates a feedback mechanism 402) that provides tactile feedback. In one such example, the feedback mechanism 402 comprises a first indicator portion 37a that comprises a spring biased protrusion or protruding surface 47, or a spring protruding surface 47, and a second indicator portion 37b comprising one or more depression or grooves 48a, as shown in Figs, 28D and 28E. As the knob 10 is actuated to move the slide assembly 30, a tactile indication is created as the spring biased protruding surface 47 travels over a fixed surface, for example as defined by a bottom surface of the inner housing 20a, that comprises the depressions or grooves 48a, as shown in Fig. 28E. In some such examples, the depressions or grooves 48a may be formed within the bottom surface of the inner housing 20a, where raised projections or ridges 48b are formed or defined by a bottom surface of the inner housing 20a of the handle 200. In another example, as shown in Fig. 28D, the raised projections or ridges 48b are formed along the bottom surface of the inner housing 20a, as shown in Fig. 28D where the depressions or grooves 48a are formed by or defined by the bottom surface of the inner housing 20a, as shown in Fig. 28D. In some such embodiments, with reference to Figs. 28D and 28E, the length of the depression or grooves 48a may be varied to define different zones of actuation and corresponding deflection of the sheath or catheter 90 upon actuation of the knob 10. In one such example, a longer depression or groove 48c is provided along the portion of the inner housing 20a that corresponds to the position of the slide assembly 30 when the sheath or catheter 90 is in a substantially un-deflected position. In some such embodiments, as the knob 10 is actuated, the neutral zone can be felt as the slide assembly 30 travels within the inner housing 20a, along the fixed surface area or region of the longer depression or groove 48c.

In some such examples, the spring biased protruding surface 47 provides spring biased characteristics that increase the resistance to actuation as the knob 10 is actuated and as such it may get harder to actuate the knob 10 as the spring biased protruding surface 47 moves across a raised projection or ridge 48b until it finds the next depression or groove 48a or 48c. In other words, the inefficiency generator 400 provides a feedback mechanism 402 that comprises the combination of the spring biased protruding surface 47 and a series of depressions or grooves 48a, 48c that are spaced apart by raised projections or ridges 48b which provide one or more indications based on the degree of resistance or in other words how hard it is for the physician to rotate to the next groove, the spring constant of the spring biased component, the size and/or frequency of the depression or grooves 48a, 48c, the size and/or frequency of the raised projections or ridges 48b, and surface features on the components that interact with one another to produce an indication (such as the spring biased protruding surface 47 of the slide assembly 30 and the grooves 48a, 48c and ridges 48b) in order to produce an audible indication. In other words, in some such embodiments, rotating the knob 10 will result in the protruding surface 47 of the slide assembly 30 to move to the next depression or groove 48a, 48c to create an audible "click". The frequency of the grooves 48a of feedback mechanism 402 as defined by the present embodiment may function to impact the handle lubricity (as it may make it harder to turn the knob 10) as well as frequency of feedback received by the physician (based on the number and spacing of grooves 48a, 48c and ridges 48b).

A steerable sheath embodiment is provided in PCT application serial number PCT/IB2013/055013. In some such embodiments an inefficiency generator 400 is provided comprising feedback mechanism 402, that comprises the spring biased protruding surface 47 at the bottom of a portion of the slide assembly 30 such as the slide 35, and one or more grooves 48a, 48c as applied to the base or bottom of the inner housing 20a (or in other words may also be referred to as the inner handle rail) of the handle 200.

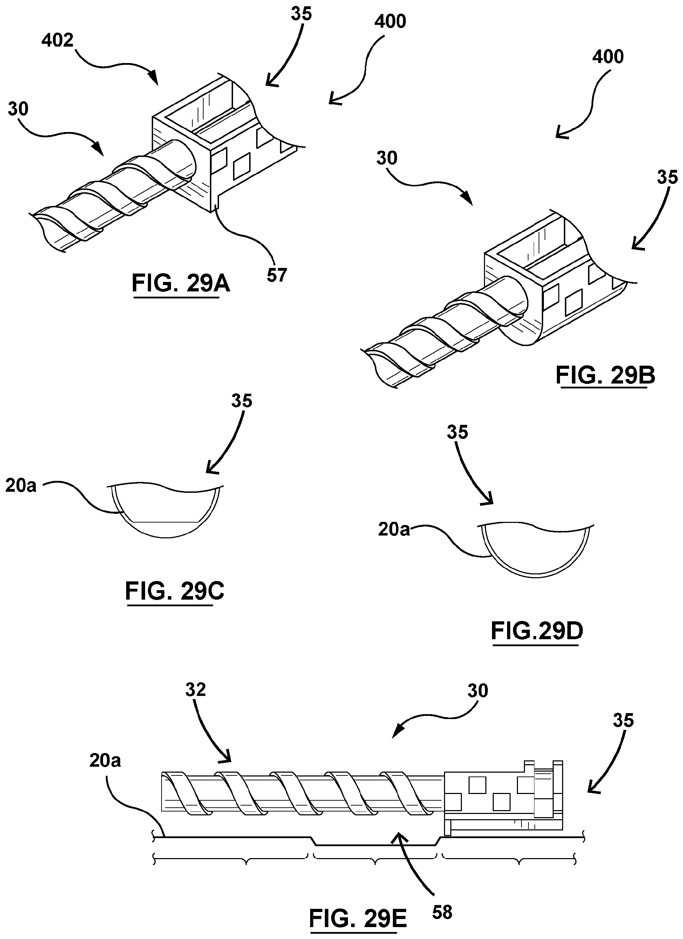

Referring now to Figs. 29A, 29B, 29C and 29D and 29E, similar to embodiments described herein above, the present inventors have discovered an apparatus and method for a steerable catheter control handle 200 that provides an inefficiency generator 400 that provides (or in other words includes or incorporates) a feedback mechanism 402 that provides an indicator for identifying the deflection of the sheath or catheter 90 and may additionally help identify the neutral zone of the steerable control handle 200. More specifically, in accordance with an embodiment of the present invention, a mechanism is provided for identifying the deflection of the sheath or catheter 90 through tactile feedback.

This may be useful when the steerable sheath 200 is inside the patient, as it may be difficult to know the orientation of the sheath curve. The inefficiency generator 400 comprising the feedback mechanism 402 provides tactile feedback to provide an indication of the sheath curvature and/or neutral zone. In some such embodiments, the feedback mechanism 402 provides tactile difference or a differential feel or resistance when the sheath or catheter 90 is curved versus when the sheath or catheter 90 in the neutral zone. In some embodiments, the handle 200 may provide an actuation mechanism that is optimized and as such may not require the user to turn the handle knob 10 a significant amount to exit the neutral zone and a corresponding feedback mechanism 402 is provided to give the user an indication when the slide assembly 30 and thus the sheath 90 exits the neutral zone.

In the present embodiment as shown in Figs. 29A-29E, the inefficiency generator 400 comprises (or in other words includes or incorporates) the feedback mechanism 402 that provides variable friction, such that the neutral zone is differentiated from the curved zone by the difference in friction when turning the handle knob 10.

In the present embodiment, with reference now to Fig. 29A, as feedback mechanism 402 comprises a feature that is added to the slider 35 to protrude out and create a frictional engagement or interface with the inner handle. In one such example, the feature comprises a protrusion 57 that provides frictional engagement between the slide assembly 30 and the inner housing 20a. In some such embodiments, as shown in Figs. 29A and 29C, creates a constant friction feel or frictional engagement (such as a high friction interface) to indicate the outer regions of the neutral zone and additionally to indicate that the sheath or catheter 90 is in the deflectable or curved region of the sheath or catheter 90. The constant frictional engagement creates a constant high friction on either side of the neutral zone region in order to mitigate any risk of confusion as to which side of the neutral zone region the user is on (in other words whether the user has entered the neutral zone or whether the sheath or catheter is in the deflectable region). As such, the feedback mechanism 402 of the present embodiment provides the combination of a protrusion 57 and the inner housing 20a that provides a wider or longer region of feedback (beyond the neutral zone) indicating that the sheath or catheter 90 is being deflected upon actuation of the actuator. In some such examples, as shown in Fig. 29C and 29E, where the neutral zone exists, a cavity 58 is created or provided in the bottom of the inner handle (specifically the inner housing 20a). As such, when the slide assembly 30 enters the cavity 58, the friction that existed between the protrusion 57 and the inner housing 20a is relieved, creating a region of low friction that substantially corresponds to the sheath or catheter 90 when it is in its substantially un-deflected position. In some such embodiments, the feedback mechanism 402 provides a sufficiently long region of low friction to provide a sufficiently long neutral zone region to allow the user to feel a change in friction, while the region of low friction is sufficiently short so that a comfortable rotation may be sufficient to exit the neutral zone. In other examples, the feedback mechanism 402 comprising a combination of the protrusion 57 and inner housing 20a may provide a single point of frictional engagement that provides discrete feedback similar to embodiments discussed hereinabove.

In other examples, as shown in Figs. 29B and 29D, the slide assembly 30, defines a slide 35 that has a uniform lower surface that interacts with a bottom surface of the inner housing 20a that defines a uniform surface along the bottom surface thereof. The inefficiency generator 400 in some such embodiments may comprise (or in other words include or incorporate) a feedback mechanism 402 as provided in an alternate embodiment as shown and described in the present disclosure.

As such, some embodiments of the present invention provide a bidirectional sheath control handle 200 that comprises an inefficiency generator 400 that comprises a feedback mechanism 402 (or in other words includes or incorporates a feedback mechanism 402) comprising a neutral zone identifier that minimizes confusion for the user without misrepresenting the neutral point within the handle 200. The feedback mechanism 402 as provided does not impede the functionality of the control handle 200. In some such embodiments of the present invention, even though the slide assembly 30 travels in both direction, in the feedback mechanism 402 of the present embodiment only one neutral identified, independent of the direction that the slide assembly 30 is traveling. In some such embodiments, the feedback mechanism may help reduce any contradictory information regarding the position of the neutral zone. As such, some such embodiments may help reduce confusion as to which side of the neutral zone region the user is on.

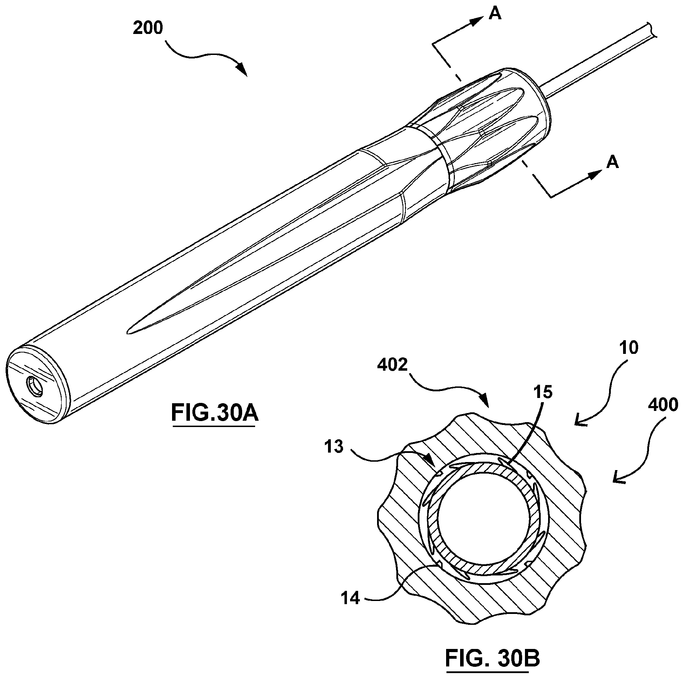

Referring now to Figs. 30A, 30B, similar to embodiments described herein above, the present inventors have discovered an apparatus and method for a steerable catheter control handle 200 that provides an inefficiency generator 400. The inefficiency generator 400 comprises a feedback mechanism 402 (or in other words includes or incorporates a feedback mechanism 402) that provides an indicator for identifying the deflection of the sheath or catheter 90 and may additionally help identify the neutral zone of the steerable control handle 200. More specifically, in accordance with an embodiment of the present invention, a mechanism is provided for identifying the deflection of a steerable sheath or catheter 90 through tactile and auditory feedback for direction of curve.

In some such embodiments, the inefficiency generator 400 provides a feedback mechanism 402 that may make it easier for physicians to determine the direction of curving of the distal end of the sheath or catheter 90, when it is inside the patient, based on the direction, the handle knob 10 is actuated in (such as by turning it). In some such instances, the feedback mechanism 402 may provide information on sheath curvature, for example in instances where it may be difficult to see the shape of the sheath curve from the fluoroscopy images, especially if the sheath is artificially curved due to anatomy (for example, regardless of the amount of actuation of the actuator for example even if a pull wire is fully engaged). Additionally, the feedback mechanism 402 may help the physician avoid damaging the sheath or catheter 90, for example, by minimizing the risk the physician thinks they are straightening out the sheath, but are curving it. The feedback mechanism 402 as provided may help avoid placing unnecessary stress on the pull wires. Furthermore, the feedback mechanism 402 may help provide an indication to allow the physician to ascertain whether the sheath 90 is being straightened out while trying to retract the sheath 90, in order to help avoid the procedural complication of pulling a curved sheath through vasculature or dislodging other devices.

More specifically, with reference now to Figs. 30A and 30B, an embodiment of the present invention provides a steerable sheath handle 200 that comprises an inefficiency generator 400 that comprises a feedback mechanism 402 that provides a tactile response and an audible clicking when the sheath or catheter 90 is being curved and has a smooth response when the sheath or catheter 90 is being straightened. This may help provide feedback to the physician without looking at the sheath or having to rely solely on fluoroscopy imaging.

With reference specifically to Fig. 30B, in some embodiments, the inefficiency generator 400 comprises (in other words includes or incorporates) the feedback mechanism 402 provides a steerable sheath handle component responsible for tightening the pull wires (such as an actuator such as a knob 10). In a specific example, the knob 10 has an undulated inner surface 13. In some such embodiments, the undulated surface 13 is ridged and comprises a series of ridges 14, and is operable to interact with a complementary inner ring 15. The feedback mechanism 402 as defined the undulated inner surface 13 and the complementary inner ring 15 (that it is functional to interact with) provides a feedback mechanism 402 that provides a smooth feel when the knob 10 is actuated or turned in one direction to actuate the sheath. The feedback mechanism 402 thus defined additionally, provides a tactile and/or audible feedback such as a click when the knob 10 is actuated or turned in the opposing direction, as the inner ring 15 engages with the undulated inner surface 13 of the outer handle component.

In some such examples, the feedback mechanism 402 as defined by the interaction between the inner ring 15 and the undulated inner surface 13 of the inner handle component may increase the torque required by the user to curve the sheath.

In some such examples, the feedback mechanism 402 as defined by the interaction between the inner ring 15 and the undulated inner surface 13 of the inner handle component may not significantly increase the torque required by the user to curve the sheath.

Some embodiments of the feedback mechanisms 402 as described herein may be usable with one or more of the steerable handles 200, 210, 220, 230 described herein.

In one broad aspect, embodiments of the present invention comprise, inter alia, a steerable catheter control handle for deflecting a catheter. The control handle comprises an actuator, one or more pull wires, and a means for deflecting the pull wires by translating the actuation of the actuator into movement of the pull wires. A distal end of each of the one or more pull wires is coupled to the catheter and a proximal end of each of the pull wires is coupled to the means for deflecting the pull wires. The control handle further comprises an inefficiency generator for interacting with the means for deflecting the pull wires, wherein the inefficiency generator substantially increases a force required to actuate the actuator to deflect the catheter.

In a further broad aspect, embodiments of the present invention comprise, inter alia, a control handle for a steerable catheter comprises a rotation to linear translation system comprising a rotatable knob, a slide assembly, the rotatable knob being coupled to the slide assembly via a threadable engagement, and a catheter comprising at least one control wire. A distal end of the control wire is coupled the catheter, and a proximal end of the at least one control or pull wire is coupled to the slide assembly. The control handle further comprises a means to introduce inefficiency into the rotation to linear translation system, wherein the means to introduce inefficiency is inherent in the rotation to linear translation system, wherein the rotation of the knob causes a linear movement of the slide assembly via rotation of the knob, to enable deflection of the catheter via actuation of the at least one control wire to impart desired curvature to the catheter, and Wherein the means to introduce inefficiency results in increased force required to curve the sheath while providing an optimized handle design that provides ease of handling for the physician and ease of use.

In a further broad aspect, embodiments of the present invention comprise, inter alia, a steerable catheter control handle for a catheter, comprising, a rotatable knob, and a slide assembly. The rotatable knob is coupled to the slide assembly via a threadable engagement, the threadable engagement comprising an angled thread. The catheter comprises at least one control wire, where a distal end of the at least one control wire is coupled the catheter, and a proximal end of the at least one control wire is coupled to the slide assembly, wherein the rotation of the knob causes a linear movement of the slide assembly via rotation of the knob, to enable deflection of the catheter via actuation of the at least one control wire to impart desired curvature to the catheter, and wherein the angled thread provides enhanced tactile feedback to the user regarding the deflection of the catheter at the distal end, and enables curve retention allowing the deflection of the catheter to be maintained at the desired curvature.

The embodiment(s) of the invention described above are intended to be exemplary only. The scope of the invention is therefore intended to be limited solely by the scope of the appended claims.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the broad scope of the appended claims. In addition, citation or identification of any reference in this application shall not be construed as an admission that such reference is available as prior art to the present invention.

## Claims

1. A steerable catheter control handle (200) for deflecting a catheter (90), the control handle (200) comprising:
an actuator (10) comprising a rotatable knob;
one or more pull wires (40, 42), wherein at least one of the one or more pull wires (40, 42) is coupled to a distal end of the catheter (90) to enable deflection of the distal end of the catheter (90);
a means for deflecting the catheter (90) upon actuation of the actuator (10), the means for deflecting the catheter (90) comprising a slide assembly (30) being coupled to the catheter (90) via the one or more pull wires (40, 42) and being coupled to the rotatable knob by a threadable engagement (302) configured to translate rotational movement of the knob into linear movement of the slide assembly (30); **characterised in that**, the handle comprises
an inefficiency generator (400) configured to interact with the means for deflecting the catheter and which comprises one or more angled threads (304) of the threadable engagement (302), wherein the one or more angled threads (304) are tapered angled to reduce, when under tension from the pull wires (40, 42), the mechanical efficiency in translating knob rotation into linear actuation of the slide assembly (30) and the mechanical efficiency in translating linear movement or actuation of the slide assembly (30) into rotation of the rotatable knob;
wherein the inefficiency generator (400) provides increasing resistance to actuation, requiring an increasing force to actuate the actuator (10) upon increasing deflection of the catheter (90); and
wherein the inefficiency generator (400) enables curve retention allowing the deflection of the catheter (90) to be substantially maintained at a desired curvature after it has been deflected.

2. The steerable catheter control handle (200) of claim 1, wherein the slide assembly (30) is moveable within an inner housing of the steerable catheter control handle (200).

3. The steerable catheter control handle (200) of claim 1, wherein the inefficiency generator (400) is configured to provide feedback regarding a relative degree of deflection of the catheter (90) including a neutral zone when the actuator is easier to turn and which corresponds to a neutral position of the catheter (90) in which pull-wire tension is the lowest.

4. The steerable catheter control handle (200) of claim 1, wherein the angled threads are configured to require a greater force to actuate the actuator (10) than a force exerted on the actuator (10) by the catheter (90) from the deflection of the catheter (90).

5. The steerable catheter control handle (200) of claim 1, wherein the rotation to linear translation system (300) comprises a single step rotation or a two-step rotation to linear translation.

6. The steerable catheter control handle (200) of claim 1 or claim 5, wherein the threadable engagement (302) is defined by external angled threads (304a) on a shaft (32) of the slide assembly (30), and corresponding internal angled threads (304b) on the knob (10) that are engageable therewith.

7. The steerable catheter control handle (200) of claim 6, wherein the angled threads have an angle of: greater than about zero degrees and less than about 180 degrees, greater than about 160 degrees and less than about 180 degrees, or an angle of between about 160 degrees and 163 degrees.

## Patentansprüche

1. Steuerbarer Katheterbediengriff (200) zum Biegen eines Katheters (90), wobei der Bediengriff (200) umfasst:
ein Betätigungselement (10), das einen Drehknopf umfasst;
einen oder mehrere Zugdrähte (40, 42), wobei mindestens einer von dem einen oder den mehreren Zugdrähten (40, 42) an ein distales Ende des Katheters (90) gekoppelt ist, um eine Biegung des distalen Endes des Katheters (90) zu ermöglichen;
ein Mittel zum Biegen des Katheters (90) bei einer Betätigung des Betätigungselements (10), wobei das Mittel zum Biegen des Katheters (90) eine Gleitbaugruppe (30) umfasst, die über den einen oder die mehreren Zugdrähte (40, 42) an den Katheter (90) gekoppelt ist und über einen Gewindeeingriff (302), der dazu konfiguriert ist, eine Drehbewegung des Knopfes in eine lineare Bewegung der Gleitbaugruppe (30) umzuwandeln, an den Drehknopf gekoppelt ist;
**dadurch gekennzeichnet, dass** der Griff umfasst:
einen Ineffizienzgenerator (400), der dazu konfiguriert ist, mit dem Mittel zum Biegen des Katheters zu interagieren, und der ein oder mehrere abgewinkelte Gewindegänge (304) des Gewindeeingriffs (302) umfasst, wobei das eine oder die mehreren abgewinkelten Gewindegänge (304) verjüngt abgewinkelt sind, um, wenn unter Spannung durch die Zugdrähte (40, 42), die mechanische Effizienz beim Umwandeln einer Knopfdrehung in eine lineare Betätigung der Gleitbaugruppe (30) und die mechanische Effizienz beim Umwandeln einer linearen Bewegung oder Betätigung der Gleitbaugruppe (30) in eine Drehung des Drehknopfes zu reduzieren;
wobei der Ineffizienzgenerator (400) ein Erhöhen des Widerstands gegen eine Betätigung bereitstellt, was ein Erhöhen von Kraft zum Betätigen des Betätigungselements (10) beim Erhöhen der Biegung des Katheters (90) erfordert; und
wobei der Ineffizienzgenerator (400) eine Kurvenretention ermöglicht, die ermöglicht, dass die Biegung des Katheters (90) im Wesentlichen bei einer gewünschten Krümmung gehalten wird, nachdem er gebogen wurde.

2. Steuerbarer Katheterbediengriff (200) nach Anspruch 1, wobei die Gleitbaugruppe (30) innerhalb eines inneren Gehäuses des steuerbaren Katheterbediengriffs (200) beweglich ist.

3. Steuerbarer Katheterbediengriff (200) nach Anspruch 1, wobei der Ineffizienzgenerator (400) dazu konfiguriert ist, eine Rückmeldung bezüglich eines relativen Grades der Biegung des Katheters (90) bereitzustellen, einschließlich eines neutralen Bereichs, wenn sich das Betätigungselement leichter drehen lässt und der einer neutralen Position des Katheters (90) entspricht, in der die Zugdrahtspannung am geringsten ist.

4. Steuerbarer Katheterbediengriff (200) nach Anspruch 1, wobei die abgewinkelten Gewindegänge dazu konfiguriert sind, eine größere Kraft zum Betätigen des Betätigungselements (10) als eine Kraft, die durch den Katheter (90) aufgrund der Biegung des Katheters (90) auf das Betätigungselement (10) ausgeübt wird, zu erfordern.

5. Steuerbarer Katheterbediengriff (200) nach Anspruch 1, wobei das System (300) für die Umwandlung einer Drehung in eine lineare Bewegung eine Umwandlung einer Drehung in einzelnen Schritten oder eine Drehung in zwei Schritten in eine lineare Bewegung umfasst.

6. Steuerbarer Katheterbediengriff (200) nach Anspruch 1 oder Anspruch 5, wobei der Gewindeeingriff (302) durch äußere abgewinkelte Gewindegänge (304a) an einer Welle (32) der Gleitbaugruppe (30) und entsprechende innere abgewinkelte Gewindegänge (304b) an dem Knopf (10), die damit in Eingriff bringbar sind, definiert ist.

7. Steuerbarer Katheterbediengriff (200) nach Anspruch 6, wobei die abgewinkelten Gewindegänge einen folgenden Winkel aufweisen: größer als etwa null Grad und kleiner als etwa 180 Grad, größer als etwa 160 Grad und kleiner als etwa 180 Grad oder einen Winkel zwischen etwa 160 Grad und 163 Grad.

## Revendications

1. Poignée de commande (200) de cathéter orientable destinée à dévier un cathéter (90), la poignée de commande (200) comprenant :
un actionneur (10) comprenant un bouton rotatif ;
un ou plusieurs fils de traction (40, 42), dans laquelle au moins l'un des un ou plusieurs fils de traction (40, 42) est couplé à une extrémité distale du cathéter (90) afin de permettre la déviation de l'extrémité distale du cathéter (90) ; et
un moyen pour dévier le cathéter (90) lorsque l'actionneur (10) est actionné, le moyen pour dévier le cathéter (90) comprenant un ensemble coulissant (30) qui est couplé au cathéter (90) par l'intermédiaire des un ou plusieurs fils de traction (40, 42) et qui est couplé au bouton rotatif par un engagement fileté (302) configuré pour transformer un mouvement de rotation du bouton en un mouvement linéaire de l'ensemble coulissant (30) ;
**caractérisée en ce que** la poignée comprend :
un générateur de perte d'efficacité (400) configuré pour interagir avec le moyen pour dévier le cathéter et comprenant un ou plusieurs filets inclinés (304) de l'engagement fileté (302), dans laquelle les un ou plusieurs filets inclinés (304) sont effilés et inclinés de manière à réduire, lorsqu'ils sont soumis à une tension exercée par les fils de traction (40, 42), l'efficacité mécanique de la transformation de la rotation du bouton en un actionnement linéaire de l'ensemble coulissant (30) ainsi que l'efficacité mécanique de la transformation du mouvement ou de l'actionnement linéaire de l'ensemble coulissant (30) en une rotation du bouton rotatif ;
dans laquelle le générateur de perte d'efficacité (400) oppose une résistance croissante à l'actionnement de sorte qu'une force croissante est requise pour actionner l'actionneur (10) lorsque la déviation du cathéter (90) augmente ; et
dans laquelle le générateur de perte d'efficacité (400) permet le maintien de la courbure, ce qui permet de maintenir essentiellement la déviation du cathéter (90) selon une courbure souhaitée après que celui-ci a été dévié.

2. Poignée de commande (200) de cathéter orientable selon la revendication 1, dans laquelle l'ensemble coulissant (30) est mobile à l'intérieur d'un logement interne de la poignée de commande (200) de cathéter orientable.

3. Poignée de commande (200) de cathéter orientable selon la revendication 1, dans laquelle le générateur de perte d'efficacité (400) est configuré pour fournir un retour d'information concernant le degré relatif de déviation du cathéter (90), comprenant une zone neutre dans laquelle l'actionneur est plus facile à tourner et qui correspond à une position neutre du cathéter (90) dans laquelle la tension des fils de traction est la plus faible.

4. Poignée de commande (200) de cathéter orientable selon la revendication 1, dans laquelle les filets inclinés sont configurés de manière à avoir besoin, pour actionner l'actionneur (10), d'une force supérieure à une force exercée sur l'actionneur (10) par le cathéter (90) du fait de la déviation du cathéter (90).

5. Poignée de commande (200) de cathéter orientable selon la revendication 1, dans laquelle le système de transformation d'un mouvement de rotation en un mouvement linéaire (300) comprend une transformation du mouvement de rotation en un mouvement linéaire en une seule étape ou en deux étapes.

6. Poignée de commande (200) de cathéter orientable selon la revendication 1 ou la revendication 5, dans laquelle l'engagement fileté (302) est défini par des filets inclinés externes (304a) sur un arbre (32) de l'ensemble coulissant (30) et par des filets inclinés internes correspondants (304b) sur le bouton (10), lesdits filets inclinés externes et internes pouvant être mutuellement engagés.

7. Poignée de commande (200) de cathéter orientable selon la revendication 6, dans laquelle les filets inclinés présentent un angle supérieur à environ zéro degré et inférieur à environ 180 degrés, supérieur à environ 160 degrés et inférieur à environ 180 degrés, ou un angle compris entre environ 160 degrés et 163 degrés.
